# EUROPEAN PATENT APPLICATION

(11) **EP 2 113 954 A1**
(43) Date of publication of application: **04.11.2009**
(21) Application number: 08711507.7
(22) Date of filing: 19.02.2008
(51) Int. Cl.: H01L 51/50, C07D 235/18, C09K 11/06

(54) **ORGANIC ELECTROLUMINESCENT DEVICE**

(30) Priority: 19.02.2007 JP 2007038654
(71) Applicant: Idemitsu Kosan Co., Ltd., Chiyoda-ku Tokyo 100-8321 (JP)
(72) Inventor: TAKASHIMA, Yoriyuki, Sodegaura-shi Chiba 299-0293 (JP); FUNAHASHI, Masakazu, Sodegaura-shi Chiba 299-0293 (JP); ARAKANE, Takashi, Sodegaura-shi Chiba 299-0293 (JP); MIZUKI, Yumiko, Sodegaura-shi Chiba 299-0293 (JP); YAMAMOTO, Hiroshi, Sodegaura-shi Chiba 299-0293 (JP); HOSOKAWA, Chishio, Sodegaura-shi Chiba 299-0293 (JP); IKEDA, Kiyoshi, Sodegaura-shi Chiba 299-0293 (JP)
(74) Representative: Gille Hrabal Struck Neidlein Prop Roos
(86) International application number: PCT/JP2008/052683
(87) International publication number: WO 2008/102740

(57) **Abstract**

An organic electroluminescent device including, a cathode, an anode, and at least an emitting layer and an electron transporting layer between the cathode and the anode, the emitting layer including a host compound and a fluoranthene compound, the electron transporting layer including a compound represented by (A)u-(B)v wherein A is a group containing an aromatic hydrocarbon group having 3 or more carbon rings, B is a heterocyclic group which may be substituted, and u and v are each an integer of 1 to 6.

## Description

### TECHNICAL FIELD

The invention relates to an organic electroluminescence (EL) device in which a fluoranthene compound and a specific electron-transporting compound are used in combination.

### BACKGROUND ART

An organic electroluminescence device is a self-emission device utilizing the principle that a fluorescent compound emits light by the recombination energy of holes injected from an anode and electrons injected from a cathode when an electric field is impressed.

Since C. W. Tang et al. of Eastman Kodak Co. reported a low-voltage driven organic EL device in the form of a stacked type device (Non-Patent Document 1), studies on organic EL devices wherein organic materials are used as the constituent materials has actively been conducted.

Tang et al. uses a stack structure in which tris(8-quinolinol)aluminum is used in an emitting layer and a triphenyldiamine derivative is used in a hole-transporting layer. The advantages of the stack structure are to increase injection efficiency of holes to the emitting layer, to increase generation efficiency of excitons generated by recombination by blocking electrons injected in the cathode, to confine the generated excitons in the emitting layer, and so on. Like this example, as the structure of the organic EL device, a two-layered type of a hole-transporting (injecting) layer and an electron-transporting emitting layer, and a three-layered type of a hole-transporting (injecting) layer, an emitting layer and an electron-transporting (injecting) layer are widely known. In such stack structure devices, their device structures and fabrication methods have been contrived to increase recombination efficiency of injected holes and electrons.

As emitting materials to be used in an organic EL device, a chelate complex such as tris(8-quinolinol)aluminum complex, a coumaline complex, a tetraphenylbutadiene derivative, a bisstrylarylene derivative, an oxadiazole derivative or the like are known. It is reported that they can emit light in a visible range from blue to red, and the realization of a color display device is expected (Patent Documents 1 to 3, for example). However, their luminous efficiency and lifetime are not at a practical level or insufficient. A full color display requires three prime colors, blue, green and red, particularly a highly efficient blue device.

Recently, a blue type luminescent device in which a fluoranthene compound is added in an emitting layer was developed. However, the color purity of the luminescent devices disclosed in Patent Documents 4 to 7 is blue green or is not described. Furthermore, the applied voltage is high of 12V and the luminous efficiency is low of about 4 to 4.5 cd/A. Patent Document 8 describes the color purity but it is not a pure blue region. The luminous efficiency is 4 cd/A or less. The luminous devices of Patent Documents 9 and 10 are excellent in blue purity but do not have a practical lifetime.

Patent Document 11 discloses a device in which a benzodiazole compound is used in an electron transporting layer. However the efficiency thereof is 4.0 cd/A and the lifetime is short.

[Patent Document 1] JP-A-H8-239655
[Patent Document 2] JP-A-H7-138561
[Patent Document 3] JP-A-H3-200889
[Patent Document 4] JP-B-3853042
[Patent Document 5] JP-A-H10-189247
[Patent Document 6] JP-A-H11-012205
[Patent Document 7] JP-A-2001-250688
[Patent Document 8] JP-A-2000-007594
[Patent Document 9] JP-A-2003-026616
[Patent Document 10] JP-A-2005-068087
[Patent Document 11] WO2004/080975
[Not-Patent Document 1] C.W.Tang, S.A.Vanslyke, Applied Physics Letters, 51, 913 (1987)

The object of the invention is to provide an organic EL device which has a high efficiency, long lifetime and excellent color purity for practical use.

### DISCLOSURE OF THE INVENTION

In order to attain the above object, through an intensive study, the inventors found that the use of a fluoranthene compound in an emitting layer and a specified compound in an electron transporting layer allows high efficiency and long lifetime of an organic EL device. The invention has been made on the finding.

According to the invention, the following organic EL device can be provided.
1. An organic electroluminescent device comprising,
   a cathode,
   an anode, and
   at least an emitting layer and an electron transporting layer between the cathode and the anode, the emitting layer comprising a host compound and a fluoranthene compound,
   the electron transporting layer comprising a compound represented by the following formula (1),

   (A)u-(B)v (1)

   wherein A is a group containing an aromatic hydrocarbon group having 3 or more carbon rings, B is a heterocyclic group which may be substituted, and u and v are each an integer of 1 to 6.
2. The organic electroluminescent device according to 1, further comprising a hole injecting layer between the anode and the emitting layer.
3. The organic electroluminescent device according to 1 or 2, further comprising a hole transporting layer between the anode and the emitting layer.
4. The organic electroluminescent device according to any one of 1 to 3, wherein the fluoranthene compound is represented by the following formula (2),

   FLₙ-G¹ (2)

   wherein n is an integer of 2 to 4,
   FL is a monovalent group having a fluoranthene structure, plural FLs may be the same or different, and
   G¹ is a substituted or unsubstituted aromatic group having 6 to 40 carbon atoms, a substituted or unsubstituted monoamino aromatic group having 6 to 40 carbon atoms, a substituted or unsubstituted diamino aromatic group having 6 to 60 carbon atoms, a substituted or unsubstituted triamino aromatic group having 6 to 60 carbon atoms, a substituted or unsubstituted heterocyclic group having 3 to 40 carbon atoms, a substituted or unsubstituted ethenylene group, or a single bond.
5. The organic electroluminescent device according to 4, wherein FL of the formula (2) is a monovalent fluoranthene compound residue represented by any one of the following formulas (3) to (6), 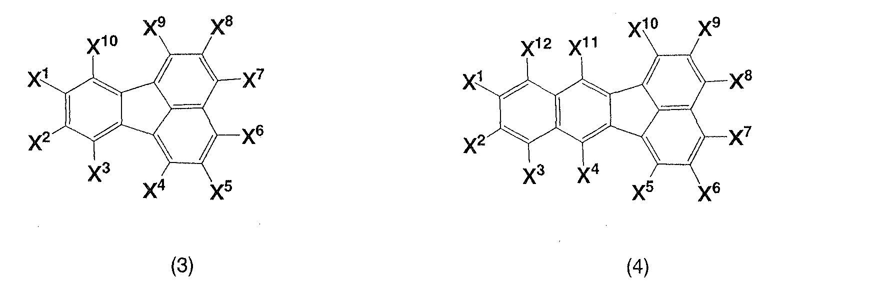 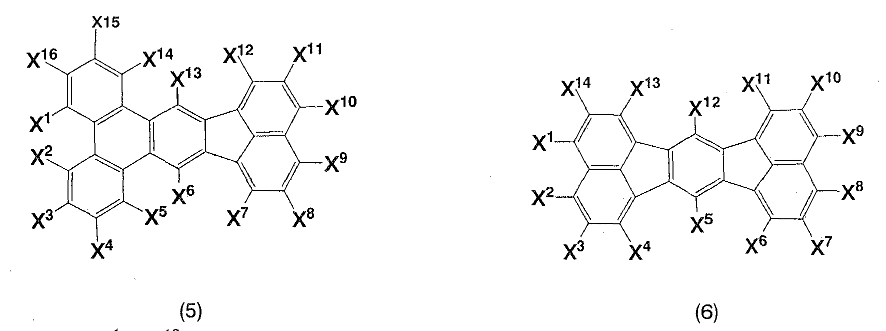 wherein X¹ to X¹⁶ are independently a hydrogen atom, a halogen atom, a nitro group, a cyano group, a substituted or unsubstituted straight, branched, or cyclic alkyl group having 1 to 30 carbon atoms, a substituted or unsubstituted straight , branched, or cyclic alkoxy group having 1 to 30 carbon atoms, a substituted or unsubstituted straight, branched, or cyclic alkylthio group having 1 to 30 carbon atoms, a substituted or unsubstituted straight, branched, or cyclic alkenyl group having 2 to 30 carbon atoms, a substituted or unsubstituted straight, branched, or cyclic alkenyloxy group having 2 to 30 carbon atoms, a substituted or unsubstituted straight, branched, or cyclic alkenylthio group having 2 to 30 carbon atoms, a substituted or unsubstituted aralkyl group having 7 to 30 carbon atoms, a substituted or unsubstituted aralkyloxy group having 7 to 30 carbon atoms, a substituted or unsubstituted aralkylthio group having 7 to 30 carbon atoms, a substituted or unsubstituted aryl group having 6 to 20 carbon atoms which is not shown by the formulas (3) to (6), a substituted or unsubstituted aryloxy group having 6 to 20 carbon atoms, a substituted or unsubstituted arylthio group having 6 to 20 carbon atoms, a substituted or unsubstituted heterocyclic group having 6 to 20 carbon atoms, a substituted or unsubstituted amino group having 2 to 30 carbon atoms, nitro group, cyano group, silyl group, hydroxy group, -COOR^{1e} group wherein R^{1e} is a hydrogen atom, a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms or a substituted or unsubstituted aryl group having 6 to 30 carbon atoms, -COR^{2e} group wherein R^{2e} is a hydrogen atom, a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms, a substituted or unsubstituted aryl group having 6 to 30 carbon atoms or an amino group, or -OCOR^{3e} group wherein R^{3e} is a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms or a substituted or unsubstituted aryl group having 6 to 30 carbon atoms; and adjacent groups and substituents of each group of X¹ to X¹⁶ may be bonded to each other to form a substituted or unsubstituted carbon ring.
6. The organic electroluminescent device according to 5, wherein the monovalent fluoranthene compound residue is a monovalent residue of a compound represented by the formula (3) or (4).
7. The organic electroluminescent device according to 4, wherein G¹ in the formula (2) is any one of groups represented by the following formulas. 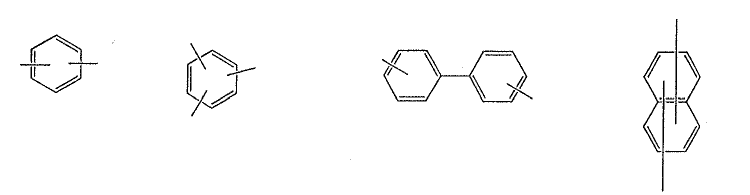 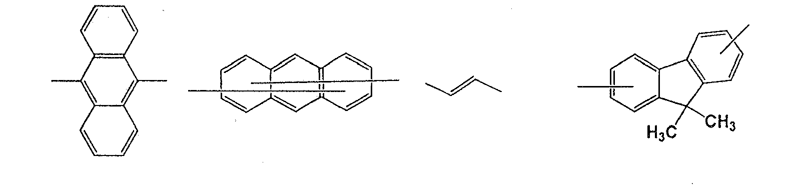 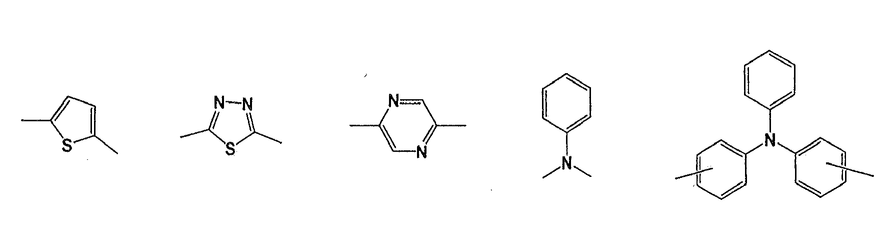 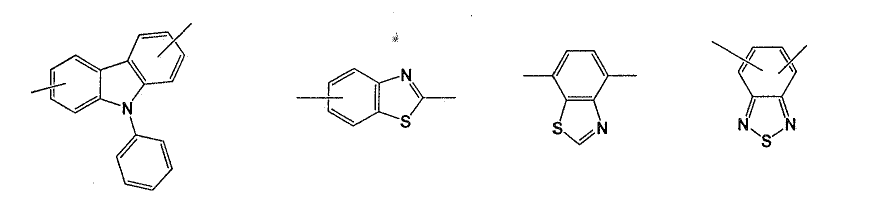
8. The organic electroluminescent device according to any one of 1 to 3, wherein the fluoranthene compound is a compound represented by any one of the following formulas (3) to (6): 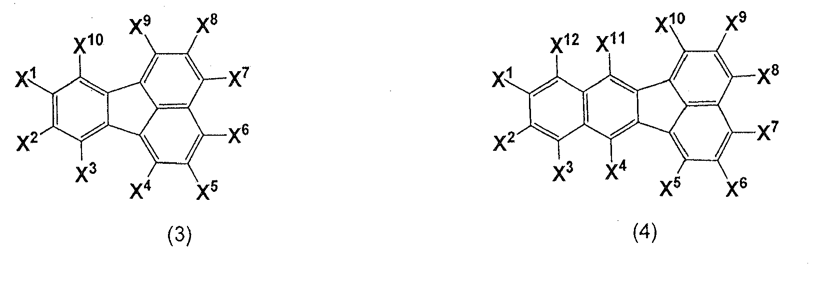 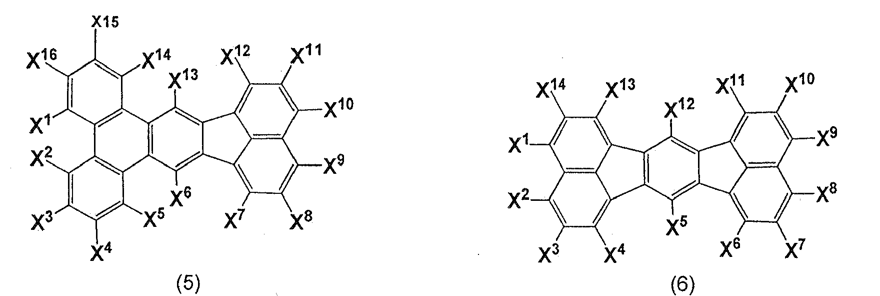 wherein X¹ to X¹⁶ are independently a hydrogen atom, a halogen atom, a nitro group, a cyano group, a substituted or unsubstituted straight, branched, or cyclic alkyl group having 1 to 30 carbon atoms, a substituted or unsubstituted straight , branched, or cyclic alkoxy group having 1 to 30 carbon atoms, a substituted or unsubstituted straight, branched, or cyclic alkylthio group having 1 to 30 carbon atoms, a substituted or unsubstituted straight, branched, or cyclic alkenyl group having 2 to 30 carbon atoms, a substituted or unsubstituted straight, branched, or cyclic alkenyloxy group having 2 to 30 carbon atoms, a substituted or unsubstituted straight, branched, or cyclic alkenylthio group having 2 to 30 carbon atoms, a substituted or unsubstituted aralkyl group having 7 to 30 carbon atoms, a substituted or unsubstituted aralkyloxy group having 7 to 30 carbon atoms, a substituted or unsubstituted aralkylthio group having 7 to 30 carbon atoms, a substituted or unsubstituted aryl group having 6 to 40 carbon atoms which is not shown by the formulas (3) to (6), a substituted or unsubstituted aryloxy group having 6 to 40 carbon atoms, a substituted or unsubstituted arylthio group having 6 to 40 carbon atoms, a substituted or unsubstituted heterocyclic group having 6 to 40 carbon atoms, a substituted or unsubstituted amino group having 2 to 30 carbon atoms, nitro group, cyano group, silyl group, hydroxy group, -COOR^{1e} group wherein R^{1e} is a hydrogen atom, a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms or a substituted or unsubstituted aryl group having 6 to 30 carbon atoms, -COR^{2e} group wherein R^{2e} is a hydrogen atom, a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms, a substituted or unsubstituted aryl group having 6 to 30 carbon atoms or an amino group, or -OCOR^{3e} group wherein R^{3e} is a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms or a substituted or unsubstituted aryl group having 6 to 30 carbon atoms; and adjacent groups and substituents of each group of X¹ to X¹⁶ may be bonded to each other to form a substituted or unsubstituted carbon ring.
9. The organic electroluminescent device according to 8, wherein the fluoranthene compound is a compound represented by the formula (3) or (4).
10. The organic electroluminescent device according to 8, wherein at least one of X¹ to X¹⁶ in the formulas (3) to (6) is an electron attracting group or a group having an electron attracting group selected from the group consisting of a nitro group, a cyano group, a halogen atom, a haloalkyl group, -COOR^{1e} group wherein R^{1e} is a hydrogen atom, a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms or a substituted or unsubstituted aryl group having 6 to 30 carbon atoms, and -COR^{2e} group
   wherein R^{2e} is a hydrogen atom, a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms or a substituted or unsubstituted aryl group having 6 to 30 carbon atoms or an amino group.
11. The organic electroluminescent device according to 10, wherein the fluoranthene compound is a compound represented by the formula (3) or (4).
12. The organic electroluminescent device according to 8, wherein at least one of X¹ to X¹⁶ of the formulas (3) to (6) is a substituted aryl group having one or more electron attracting groups.
13. The organic electroluminescent device according to 8, wherein at least one of X¹ to X¹⁶ of the formulas (3) to (6) is a substituted aryl group, and a substituent of the aryl group is a substituted or unsubstituted aryl group having an electron attracting group.
14. The organic electroluminescent device according to any one of 10 to 13, wherein the electron attracting group is a group selected from the group consisting of a nitro group, a cyano group, a fluorine group and an haloalkyl group.
15. The organic electroluminescent device according to any one of 10 to 14, wherein X⁴ and X¹¹ of the compound (4) is a phenyl group.
16. The organic electroluminescent device according to any one of 10 to 15, wherein X⁷ of the compound (4) is a hydrogen atom, and X⁸ of the compound (4) is an aryl group having an electron attracting group.
17. The organic electroluminescent device according to any one of 10 to 15, wherein X⁷ of the compound (4) is a hydrogen atom, and X⁸ of the compound (4) is a substituted aryl group having an aryl group having an electron attracting group.
18. The organic electroluminescent device according to any one of 10 to 14, wherein X³ and X¹⁰ of the compound (3) is a phenyl group.
19. The organic electroluminescent device according to any one of 10 to 14 and 18, wherein X⁶ of the compound (3) is a hydrogen atom and X⁷ of the compound (3) is an aryl group having an electron attracting group.
20. The organic electroluminescent device according to any one of 10 to 14 and 18, wherein X⁶ of the compound (3) is a hydrogen atom and X⁷ of the compound (3) is a substituted aryl group having an aryl group having an electron attracting group.
21. The organic electroluminescent device according to any one of 1 to 20, wherein the compound represented by the formula (1) is a compound having in its molecule one or more skeletons selected from anthracene, phenanthrene, naphthacene, pyrene, chrysene, benzanthracene, pentacene, dibenzanthracene, benzopyrene, fluorene, benzofluorene, fluoranthene, benzofluoranthene, naphthofluoranthene, dibenzofluorene, dibenzopyrene and dibenzofluoranthene.
22. The organic electroluminescent device according to any one of 1 to 21, wherein the compound represented by the formula (1) is a nitrogen-containing heterocyclic compound.
23. The organic electroluminescent device according to 22, wherein the nitrogen-containing heterocyclic compound is a compound having in its molecule one or more skeletons selected from pyridine, pyrimidine, pyrazine, pyridazine, triazine, quinoline, quinoxaline, acridine, imidazopyridine, imidazopyrimidine and phenenthroline.
24. The organic electroluminescent device according to 22, wherein the nitrogen-containing heterocyclic compound is a benzimidazole derivative represented by the following formula (7) or (8): 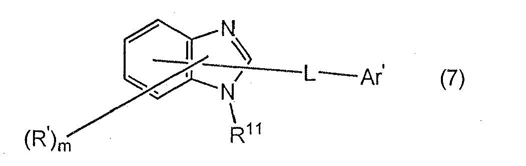 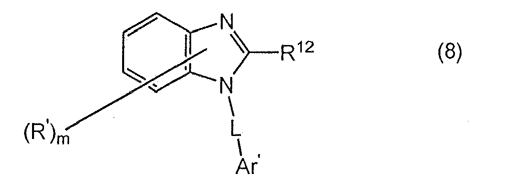 wherein R' is a hydrogen atom, a substituted or unsubstituted aryl group having 6 to 60 carbon atoms, a substituted or unsubstituted pyridyl group, a substituted or unsubstituted quinolyl group, a substituted or unsubstituted alkyl group having 1 to 20 carbon atoms, or a substituted or unsubstituted alkoxy group having 1 to 20 carbon atoms;
   m is an integer of 0 to 4;
   R¹¹ is a substituted or unsubstituted aryl group having 6 to 60 carbon atoms, a substituted or unsubstituted pyridyl group, a substituted or unsubstituted quinolyl group, a substituted or unsubstituted alkyl group having 1 to 20 carbon atoms, or an alkoxy group having 1 to 20 carbon atoms;
   R¹² is a hydrogen atom, a substituted or unsubstituted aryl group having 6 to 60 carbon atoms, a substituted or unsubstituted pyridyl group, a substituted or unsubstituted quinolyl group, a substituted or unsubstituted alkyl group having 1 to 20 carbon atoms, or a substituted or unsubstituted alkoxy group having 1 to 20 carbon atoms;
   L is a substituted or unsubstituted arylene group having 6 to 60 carbon atoms, a substituted or unsubstituted pyridinylene group, a substituted or unsubstituted quinolinylene group, or a substituted or unsubstituted fluorenylene group; and
   Ar' is a substituted or unsubstituted aryl group having 6 to 60 carbon atoms, a substituted or unsubstituted pyridinyl group, or a substituted or unsubstituted quinolinyl group.
25. The organic electroluminescent device according to any one of 1 to 24, wherein the host compound is an anthracene derivative.
26. The organic electroluminescent device according to 25, wherein the anthracene derivative is a compound represented by the following formula (9): 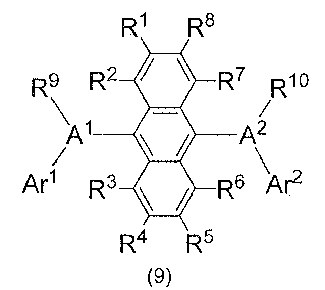 wherein A¹ and A² are independently a hydrogen atom or a substituted or unsubstituted aromatic group having 6 to 50 carbon atoms that form a ring (ring carbon atoms);
   Ar¹ and Ar² are independently a hydrogen atom or a substituted or unsubstituted aromatic group having 6 to 50 ring carbon atoms;
   R¹ to R¹⁰ are independently a hydrogen atom, a substituted or unsubstituted aromatic group having 6 to 50 ring carbon atoms, a substituted or unsubstituted aromatic hetrocyclic group having 5 to 50 atoms that form a ring (ring atoms), a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group or a hydroxyl group; and
   Ar¹ and R⁹ and/or Ar² and R¹⁰ may be bonded to each other to form a saturated or unsaturated cyclic structure.
27. The organic electroluminescent device according to 26, wherein the anthracene derivative is a compound in which the groups are not symmetrically bonded to 9 and 10 positions of the central anthracene with respect to the X-Y axis in the following formula. 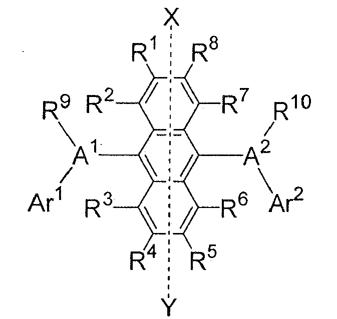
28. The organic electroluminescent device according to any one of 1 to 27, wherein the emitting layer contains the fluoranthene compound in an amount of 0.01 to 20 wt%.
29. The organic electroluminescent device according to any one of 1 to 28, wherein a chalcogenide layer, a metal halide layer or a metal oxide layer is provided on a surface of at least one of the cathode and the anode.
30. A solution for forming an emitting layer of an organic electroluminescent device comprising a fluoranthene compound represented by any one of the formulas (2) to (6) and a solvent.

According to the invention, there is provided an organic EL device which has a high efficiency, long lifetime and excellent color purity for practical use.
According to the invention, by selecting suitable compounds for an emitting layer and an electron transporting layer, a high efficient organic EL device can be obtained. The structure of the invention suppresses the generation of excitons in an electron transporting layer whereby an organic EL device with a high color purity can be obtained in which slight light emission from the electron transporting layer is suppressed to a lower level. For the same reasons, the lifetime of the device can be made longer.

### BRIEF DESCRIPTION OF DRAWINGS

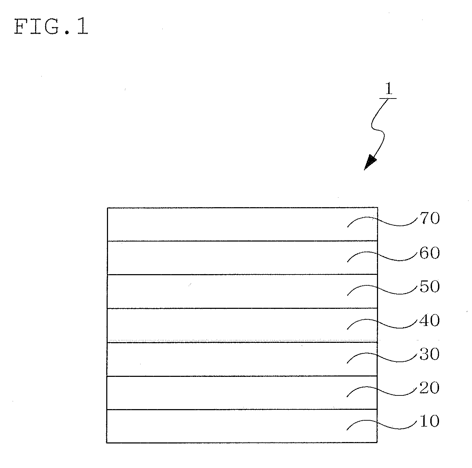

Fig.1 is a view showing an embodiment of the organic EL device according to the invention.

### BEST MODE FOR CARRYING OUT THE INVENTION

The organic EL device according to the invention comprises a cathode, an anode, and at least an emitting layer and an electron transporting layer therebetween. The emitting layer comprises a host compound and a fluoranthene compound, and the electron transporting layer comprises a compound represented by the following formula (1),

(A)u-(B)v (1)

wherein A is a group containing an aromatic hydrocarbon group having 3 or more carbon rings, B is a heterocyclic group which may be substituted, and u and v are each an integer of 1 to 6.
Preferably, the organic EL device further comprises a hole injecting layer between the anode and the emitting layer. Preferably, the device further comprises a hole transporting layer therebetween.

Fig.1 shows one example of the organic EL device according to the invention. In this drawing, the organic EL device 1 has a multilayered structure of an anode 20, hole injecting layer 30, hole transporting layer 40, emitting layer 50, electron transporting layer 60 and cathode 70 in this order on a substrate 10. The emitting layer 50 contains a host material and a dopant material, and it contains a fluoranthene compound as the dopant material. The electron transporting layer 60 contains a compound represented by the formula (1).

The organic EL device of the invention can realize practical efficiency and lifetime by an emitting layer containing a fluoranthene compound and a host material and an electron transporting layer containing a benzodiazole compound. The organic EL device of the invention preferably emits blue light.

The fluoranthene compound is preferably a compound represented by the following formula (2),

FLₙ-G¹ (2)

wherein n is an integer of 2 to 4,
FL is a monovalent group having a fluoranthene structure, plural FLs may be the same or different, and
G¹ is a substituted or unsubstituted aromatic group having 6 to 40 carbon atoms, a substituted or unsubstituted monoamino aromatic group having 6 to 40 carbon atoms, a substituted or unsubstituted diamino aromatic group having 6 to 60 carbon atoms, a substituted or unsubstituted triamino aromatic group having 6 to 60 carbon atoms, a substituted or unsubstituted heterocyclic group having 3 to 40 carbon atoms, a substituted or unsubstituted ethenylene group, or a single bond.

In the formula (2), n is preferably 2 or 3. When n is 2 or more, G¹ is a group having a valence of n. When n is 2, G¹ can be a substituted or unsubstituted ethenylene group or single bond.

FL of the formula (2) is preferably a monovalent fluoranthene compound residue represented by any one of the following formulas (3) to (6), 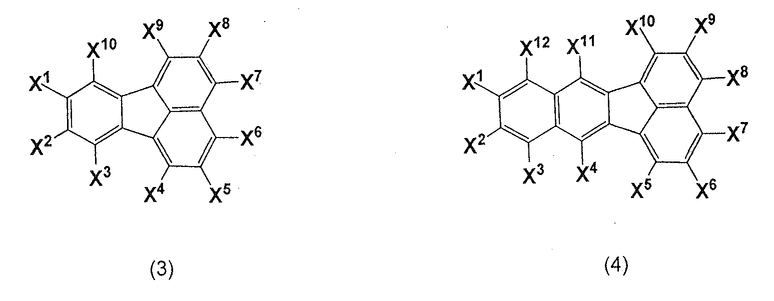 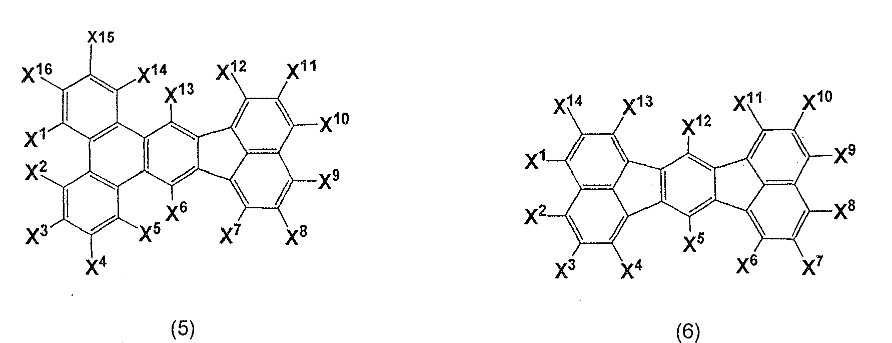 wherein X¹ to X¹⁶ are independently a hydrogen atom, a halogen atom, a nitro group, a cyano group, a substituted or unsubstituted straight, branched, or cyclic alkyl group having 1 to 30 carbon atoms, a substituted or unsubstituted straight , branched, or cyclic alkoxy group having 1 to 30 carbon atoms, a substituted or unsubstituted straight, branched, or cyclic alkylthio group having 1 to 30 carbon atoms, a substituted or unsubstituted straight, branched, or cyclic alkenyl group having 2 to 30 carbon atoms, a substituted or unsubstituted straight, branched, or cyclic alkenyloxy group having 2 to 30 carbon atoms, a substituted or unsubstituted straight, branched, or cyclic alkenylthio group having 2 to 30 carbon atoms, a substituted or unsubstituted aralkyl group having 7 to 30 carbon atoms, a substituted or unsubstituted aralkyloxy group having 7 to 30 carbon atoms, a substituted or unsubstituted aralkylthio group having 7 to 30 carbon atoms, a substituted or unsubstituted aryl group having 6 to 40(preferably 6 to 20) carbon atoms which is not shown by the formulas (3) to (6), a substituted or unsubstituted aryloxy group having 6 to 40(preferably 6 to 20) carbon atoms, a substituted or unsubstituted arylthio group having 6 to 40(preferably 6 to 20) carbon atoms, a substituted or unsubstituted heterocyclic group having 6 to 40(preferably 6 to 20) carbon atoms, a substituted or unsubstituted amino group having 2 to 30 carbon atoms, nitro group, cyano group, silyl group, hydroxy group, -COOR^{1e} group wherein R^{1e} is a hydrogen atom, a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms or a substituted or unsubstituted aryl group having 6 to 30 carbon atoms, -COR^{2e} group wherein R^{2e} is a hydrogen atom, a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms, a substituted or unsubstituted aryl group having 6 to 30 carbon atoms or an amino group, or -OCOR^{3e} group wherein R^{3e} is a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms or a substituted or unsubstituted aryl group having 6 to 30 carbon atoms; and adjacent groups and substituents of each group of X¹ to X¹⁶ may be bonded to each other to form a substituted or unsubstituted carbon ring.

The monovalent fluoranthene compound residue is preferably a monovalent residue of a compound represented by the formula (3) or (4).
In the formulas (3) to (6), X¹ to X¹⁶ are preferably a hydrogen atom, a substituted or unsubstituted aryl group having 6 to 40 carbon atoms (preferably a phenyl group, diphenyl group, naphthyl group, fluorenyl group, pyrenyl group, anthracenyl group, phenylnaphthyl group, naphthylphenyl group, anthranil group, phenanthyl group, pyrenyl group, chrysenyl group, terphenyl group, naphthylphenyl group, naphthylnaphthyl group, fluorenyl group and 9,9-dimetylfluorenyl group), a straight, branched or cyclic alkyl group having 1 to 30 carbon atoms (preferably a methyl group, ethyl group, propyl group, isopropyl group, butyl group, sec-butyl group, tert-butyl group, cyclopentyl, cyclohexyl group and cycloheptyl group) and a substituted or unsubstituted heterocyclic group having 6 to 40 carbon atoms.

The substituents of X¹ to X¹⁶ include a straight, branched or cyclic alkyl group having 1 to 30 carbon atoms (preferably a methyl group, ethyl group, propyl group, isopropyl group, butyl group, sec-butyl group, tert-butyl group, cyclopentyl, cyclohexyl group and cycloheptyl group), a substituted or unsubstituted aryl group having 6 to 40 (preferably 6 to 20) carbon atoms (preferably a phenyl group, naphthyl group, anthranil group, phenanthyl group, pyrenyl group, chrysenyl group, fluorenyl group and 9,9-dimetylfluorenyl group) and an electron attracting group (preferably a nitro group, cyano group, fluorine atom and haloalkyl group).

X¹ to X¹⁶ are preferably -Ar¹-Ar² or -Ar³-Ar⁴-Ar⁵.
Ar¹, Ar³ and Ar⁴ include a substituted or unsubstituted arylene group having 6 to 40 (preferably 6 to 20) carbon atoms (preferably a divalent residue of benzene, naphthalene, anthracene, phenanthrene, pyrene, chrysene, fluorene and 9,9-dimetylfluorene).
Ar² and Ar⁵ include a substituted or unsubstituted aryl group having 6 to 40 (preferably 6 to 20) carbon atoms (preferably a phenyl group, naphthyl group, anthranil group, phenanthyl group, pyrenyl group, chrysenyl group, biphenyl group, terphenyl group, naphthylphenyl group, phenylnaphthyl group, naphthylnaphthyl group, fluorenyl group and 9,9-dimetylfluorenyl group).

The substituents of Ar¹ to Ar⁵ include a straight, branched or cyclic alkyl group having 1 to 30 carbon atoms (preferably a methyl group, ethyl group, propyl group, isopropyl group, butyl group, sec-butyl group, tert-butyl group, cyclopentyl, cyclohexyl group and cycloheptyl group), a substituted or unsubstituted aryl group having 6 to 40 (preferably 6 to 20) carbon atoms (preferably a phenyl group, naphthyl group, anthranil group, phenanthyl group, pyrenyl group, chrysenyl group, fluorenyl group and 9,9-dimetylfluorenyl group) and an electron attracting group (preferably a nitro group, cyano group, fluorine atom and haloalkyl group).

In G¹ of the formula (2), examples of the skeleton of the aromatic group include benzene, diphenyl, naphthalene, anthracene and fluorene.

Examples of the skeleton of the heterocyclic group include thiophene, pyrazine, thiazole, thiadiazole, benzothiadiazole, benzothiazole, and carbazole.

The substituent of G¹ includes a substituted or unsubstituted aryl group having 6 to 50 ring carbon atoms, substituted or unsubstituted aromatic heterocyclic group having 5 to 50 ring atoms, substituted or unsubstituted alkyl group having 1 to 50 carbon atoms, substituted or unsubstituted alkoxy group having 1 to 50 carbon atoms, substituted or unsubstituted aralkyl group having 1 to 50 carbon atoms, substituted or unsubstituted aryloxy group having 5 to 50 ring atoms, substituted or unsubstituted arylthio group having 5 to 50 ring atoms, substituted or unsubstituted carboxyl group having 1 to 50 carbon atoms, halogen group, cyano group, nitro group and hydroxy group. Preferred substituents are an aryl group having 6 to 50 ring carbon atoms such as phenyl and substituted or unsubstituted alkyl group having 1 to 50 carbon atoms.

G¹ in the formula (2) is preferably any one of groups represented by the following formulas.

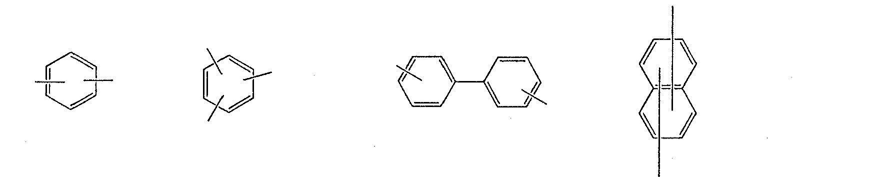

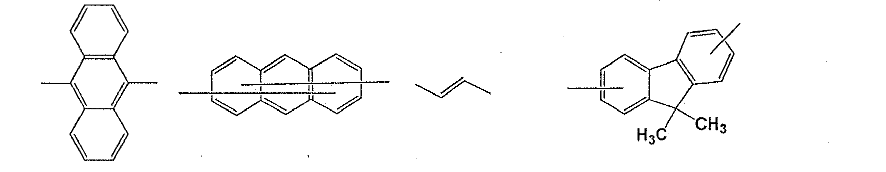

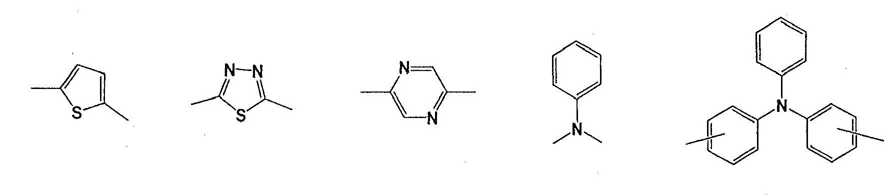

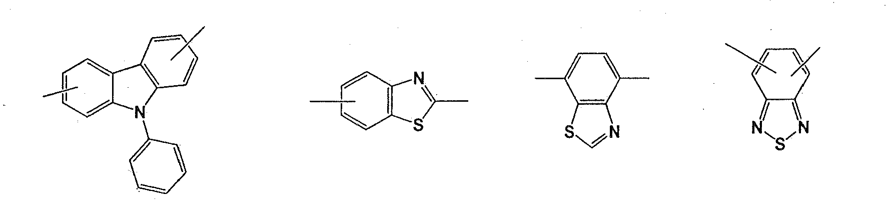

A Preferred fluoranthene compound is a compound represented by any one of the following formulas (3) to (6): 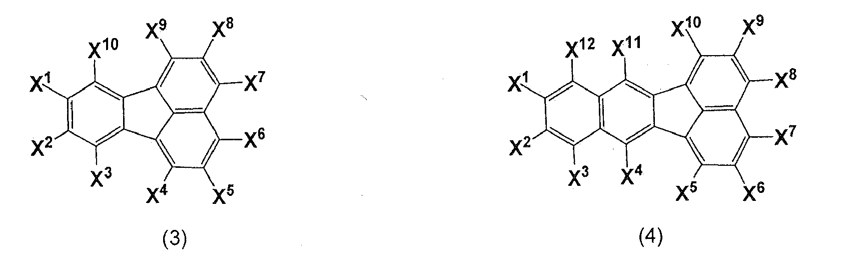 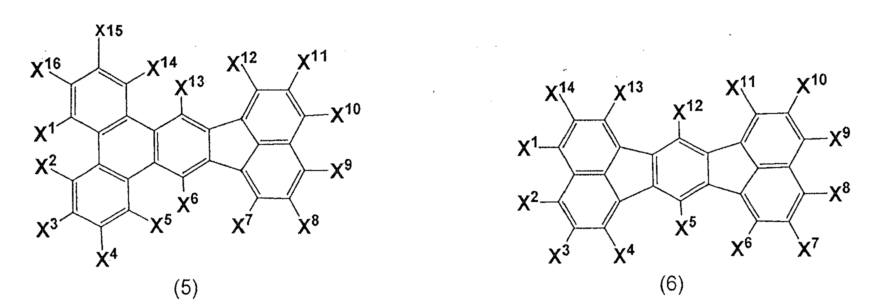 wherein X¹ to X¹⁶ are independently a hydrogen atom, a halogen atom, a nitro group, a cyano group, a substituted or unsubstituted straight, branched, or cyclic alkyl group having 1 to 30 carbon atoms, a substituted or unsubstituted straight , branched, or cyclic alkoxy group having 1 to 30 carbon atoms, a substituted or unsubstituted straight, branched, or cyclic alkylthio group having 1 to 30 carbon atoms, a substituted or unsubstituted straight, branched, or cyclic alkenyl group having 2 to 30 carbon atoms, a substituted or unsubstituted straight, branched, or cyclic alkenyloxy group having 2 to 30 carbon atoms, a substituted or unsubstituted straight, branched, or cyclic alkenylthio group having 2 to 30 carbon atoms, a substituted or unsubstituted aralkyl group having 7 to 30 carbon atoms, a substituted or unsubstituted aralkyloxy group having 7 to 30 carbon atoms, a substituted or unsubstituted aralkylthio group having 7 to 30 carbon atoms, a substituted or unsubstituted aryl group having 6 to 40 (preferably 6 to 20) carbon atoms which is not shown by the formulas (3) to (6), a substituted or unsubstituted aryloxy group having 6 to 40 (preferably 6 to 20) carbon atoms, a substituted or unsubstituted arylthio group having 6 to 40 (preferably 6 to 20) carbon atoms, a substituted or unsubstituted heterocyclic group having 6 to 40 (preferably 6 to 20) carbon atoms, a substituted or unsubstituted amino group having 2 to 30 carbon atoms, nitro group, cyano group, silyl group, hydroxy group, -COOR^{1e} group wherein R^{1e} is a hydrogen atom, a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms or a substituted or unsubstituted aryl group having 6 to 30 carbon atoms, -COR^{2e} group wherein R^{2e} is a hydrogen atom, a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms, a substituted or unsubstituted aryl group having 6 to 30 carbon atoms or an amino group, or -OCOR^{3e} group wherein R^{3e} is a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms or a substituted or unsubstituted aryl group having 6 to 30 carbon atoms; and adjacent groups and substituents of each group of X¹ to X¹⁶ may be bonded to each other to form a substituted or unsubstituted carbon ring.

In particular, a compound represented by the formula (3) or (4) is preferred.

In the formulas (3) to (6), X¹ to X¹⁶ are preferably a hydrogen atom, a substituted or unsubstituted aryl group having 6 to 40 carbon atoms (preferably a phenyl group, diphenyl group, naphthyl group, fluorenyl group, pyrenyl group, anthracenyl group, phenylnaphthyl group, naphthylphenyl group, anthranil group, phenanthyl group, pyrenyl group, chrysenyl group, terphenyl group, naphthylphenyl group, naphthylnaphthyl group, fluorenyl group and 9,9-dimetylfluorenyl group), a straight, branched or cyclic alkyl group having 1 to 30 carbon atoms (preferably a methyl group, ethyl group, propyl group, isopropyl group, butyl group, sec-butyl group, tert-butyl group, cyclopentyl, cyclohexyl group and cycloheptyl group) and a substituted or unsubstituted heterocyclic group having 6 to 40 carbon atoms.

The substituents of X¹ to X¹⁶ include a straight, branched or cyclic alkyl group having 1 to 30 carbon atoms (preferably a methyl group, ethyl group, propyl group, isopropyl group, butyl group, sec-butyl group, tert-butyl group, cyclopentyl, cyclohexyl group and cycloheptyl group), a substituted or unsubstituted aryl group having 6 to 40 (preferably 6 to 20) carbon atoms (preferably a phenyl group, naphthyl group, anthranil group, phenanthyl group, pyrenyl group, chrysenyl group, fluorenyl group and 9,9-dimetylfluorenyl group) and an electron attracting group (preferably a nitro group, cyano group, fluorine atom and haloalkyl group).

X¹ to X¹⁶ are preferably -Ar¹-Ar² or -Ar³-Ar⁴-Ar⁵.
Ar¹, Ar³ and Ar⁴ include a substituted or unsubstituted arylene group having 6 to 40 (preferably 6 to 20) carbon atoms (preferably a divalent residue of benzene, naphthalene, anthracene, phenanthrene, pyrene, chrysene, fluorene and 9,9-dimetylfluorene).
Ar² and Ar⁵ include a substituted or unsubstituted aryl group having 6 to 40 (preferably 6 to 20) carbon atoms (preferably a phenyl group, naphthyl group, anthranil group, phenanthyl group, pyrenyl group, chrysenyl group, biphenyl group, terphenyl group, naphthylphenyl group, phenylnaphthyl group, naphthylnaphthyl group, fluorenyl group and 9,9-dimetylfluorenyl group).

The substituents of Ar¹ to Ar⁵ include a straight, branched or cyclic alkyl group having 1 to 30 carbon atoms (preferably a methyl group, ethyl group, propyl group, isopropyl group, butyl group, sec-butyl group, tert-butyl group, cyclopentyl, cyclohexyl group and cycloheptyl group), a substituted or unsubstituted aryl group having 6 to 40 (preferably 6 to 20) carbon atoms (preferably a phenyl group, naphthyl group, anthranil group, phenanthyl group, pyrenyl group, chrysenyl group, fluorenyl group and 9,9-dimetylfluorenyl group) and an electron attracting group (preferably a nitro group, cyano group, fluorine atom and haloalkyl group).

In the invention, it is preferred that at least one of X¹ to X¹⁶ in the formulas (3) to (6) be an electron attracting group or a group having an electron attracting group.
The electron attracting group is preferably selected from the group consisting of a nitro group, a cyano group, a halogen atom, a haloalkyl group, -COOR^{1e} group wherein R^{1e} is a hydrogen atom such as a fluorine group, chloro group, bromo group and iodine group, a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms or a substituted or unsubstituted aryl group having 6 to 30 carbon atoms, and -COR^{2e} group wherein R^{2e} is a hydrogen atom, a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms or a substituted or unsubstituted aryl group having 6 to 30 carbon atoms or an amino group.

Of the electron attracting groups, a substituted aryl group having one or more electron attracting groups is preferred. Specifically, phenyl and naphtyl groups having the above electron attracting group are exemplified.
It is preferred that at least one of X¹ to X¹⁶ of the formulas (3) to (6) be a substituted aryl group, and a substituent of the aryl group be a substituted or unsubstituted aryl group having an electron attracting group.
The electron attracting group is preferably a group selected from the group consisting of a nitro group, a cyano group, a fluorine group and an haloalkyl group.
Preferable haloalkyl groups include a trichloromethyl group, difluoromethyl group, fluoromethyl group, trifluoro group, 1,1,1-trifluoroethyl group, perfluoroethyl group, perfluoropropyl group, and 1,1,1,2,2-pentafluoropropyl group.

In the formulas (3) to (6), the compound represented by the formula (3) or (4) is preferable.
It is preferred that X³ and X¹⁰ of the compound (3) be a phenyl group.
It is preferred that X⁶ of the compound (3) be a hydrogen atom and X⁷ of the compound (3) be an aryl group having an electron attracting group.
It is preferred that X⁶ of the compound (3) be a hydrogen atom and X⁷ of the compound (3) be a substituted aryl group having an aryl group having an electron attracting group.

In particular, X⁴ and X¹¹ of the compound (4) are preferably a phenyl group.
It is preferred that X⁷ of the compound (4) be a hydrogen atom, and X⁸ of the compound (4) be an aryl group having an electron attracting group.
It is preferred that X⁷ of the compound (4) be a hydrogen atom, and X⁸ of the compound (4) be a substituted aryl group having an aryl group having an electron attracting group.

As examples of the fluoranthene compound, the following compounds are given.

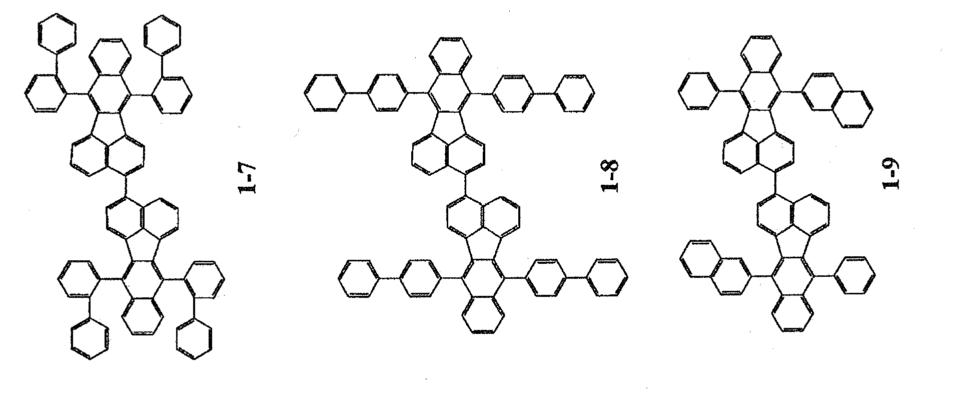

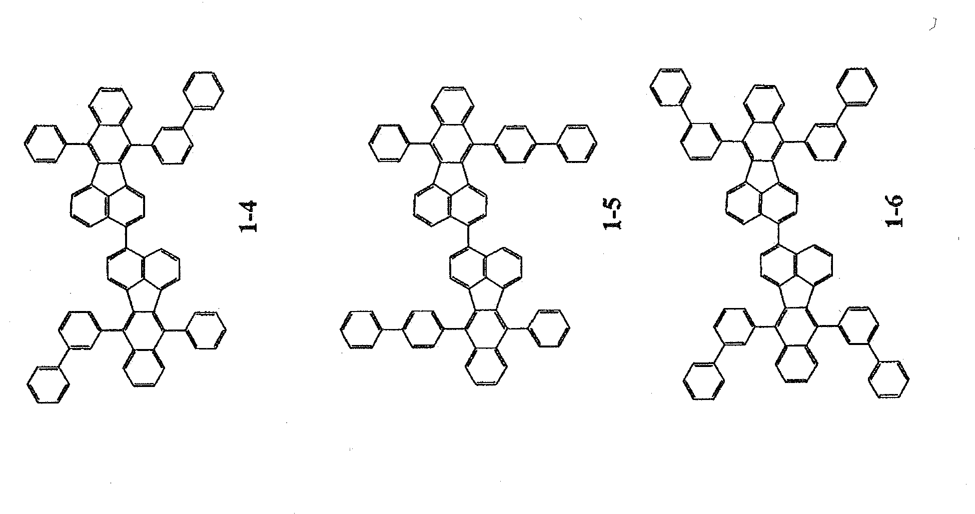

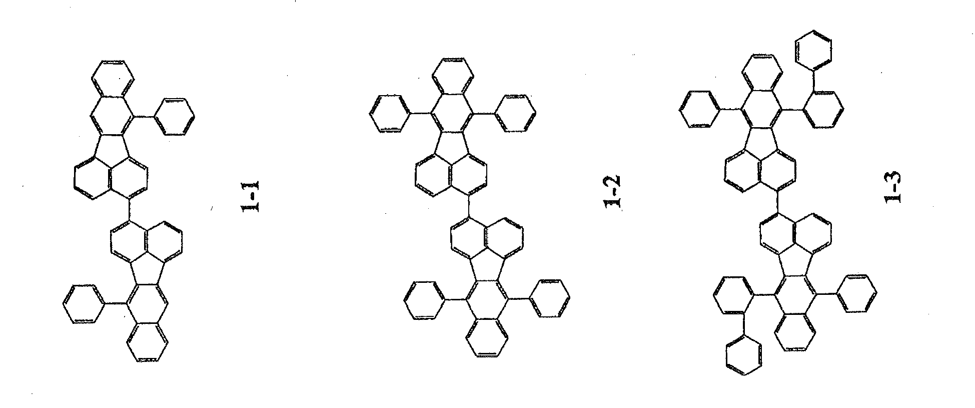

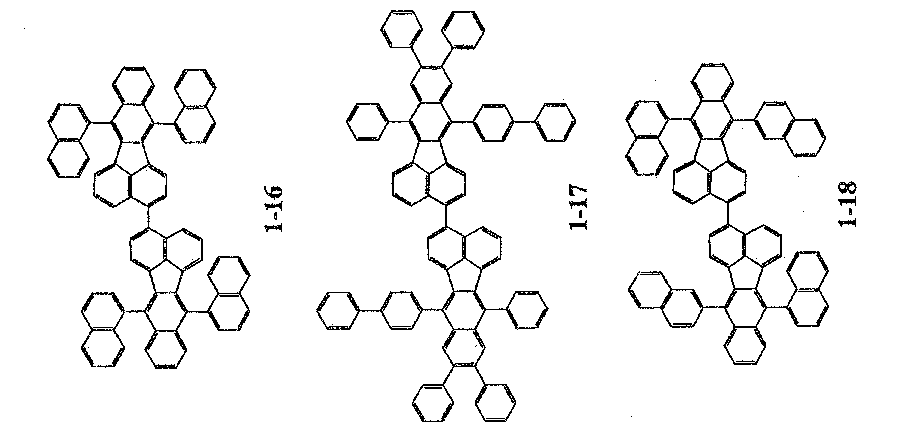

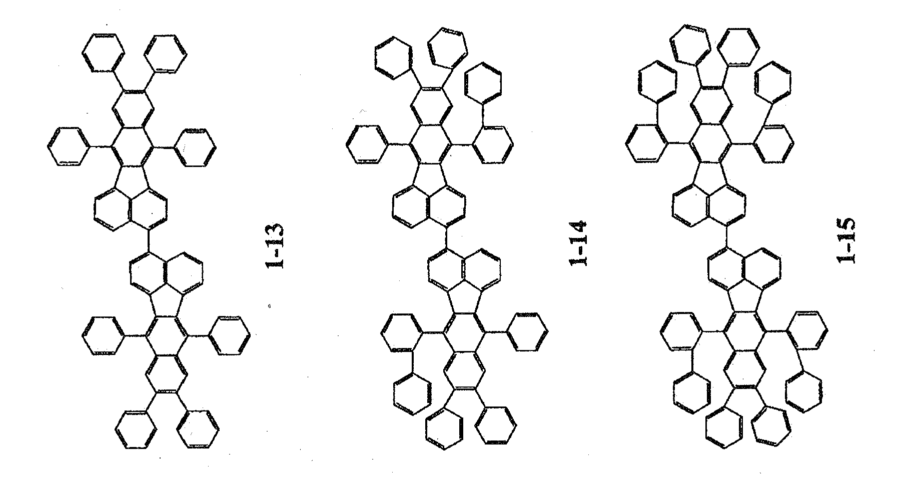

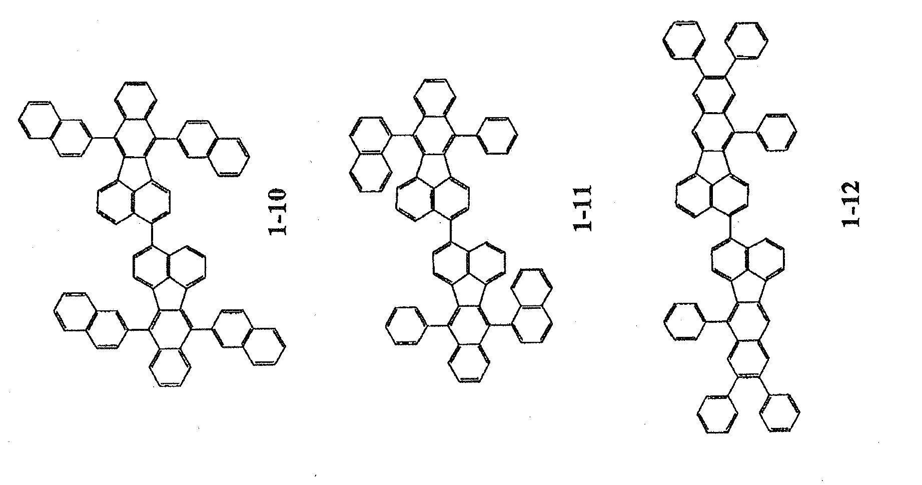

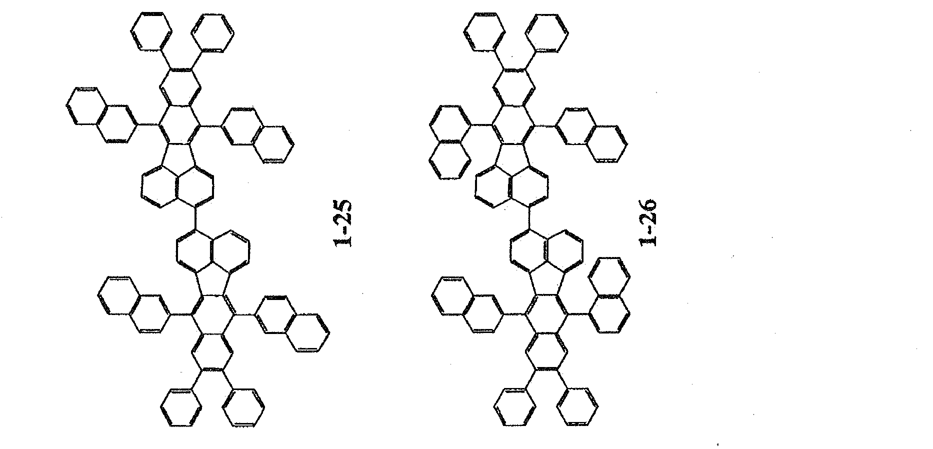

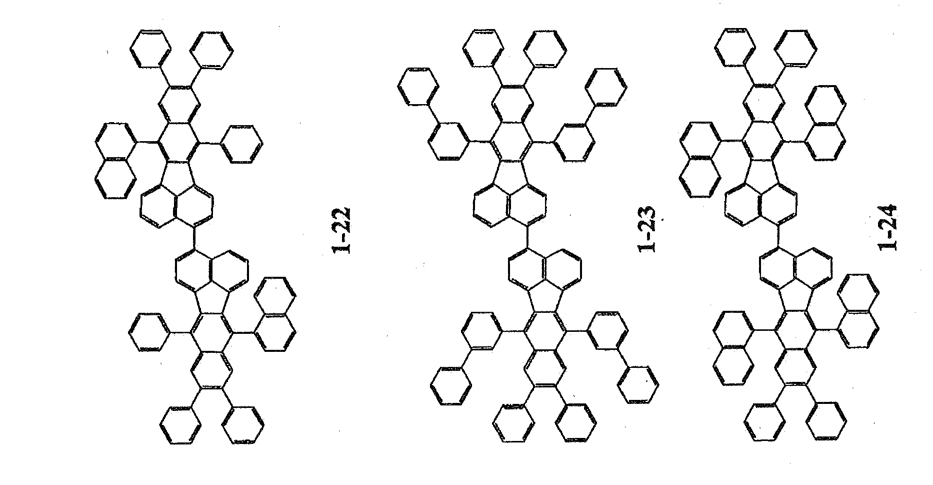

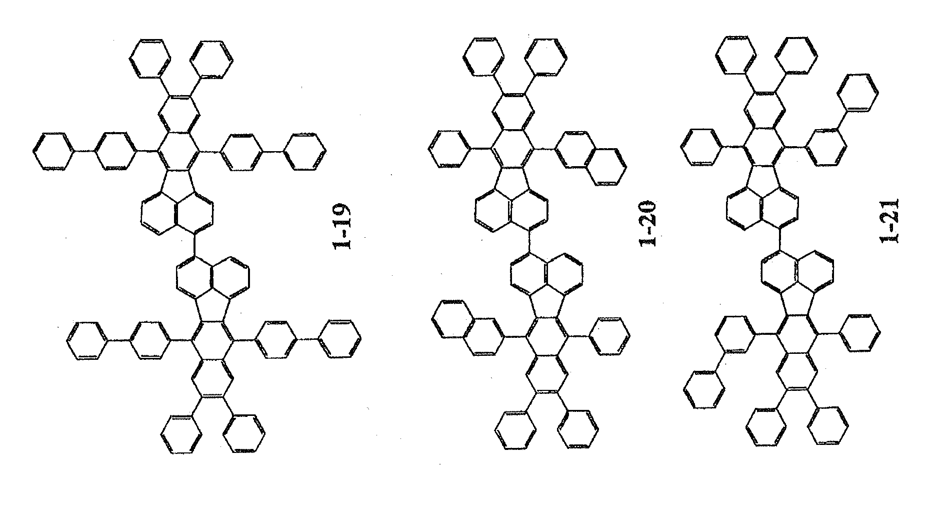

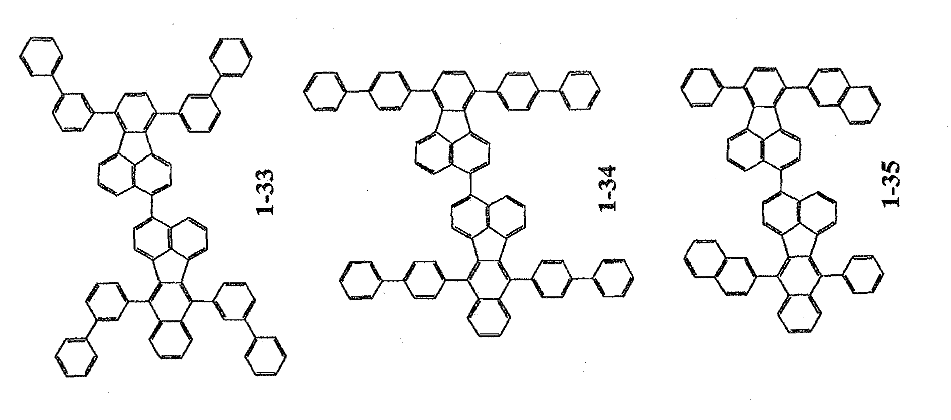

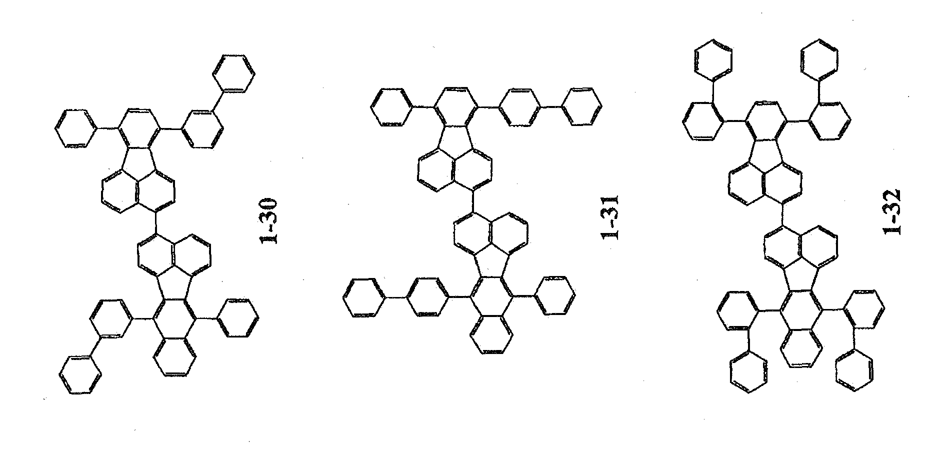

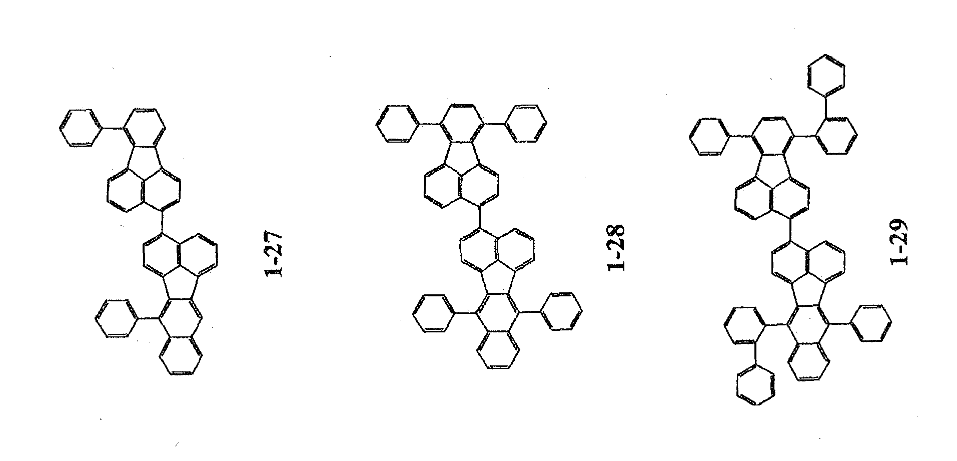

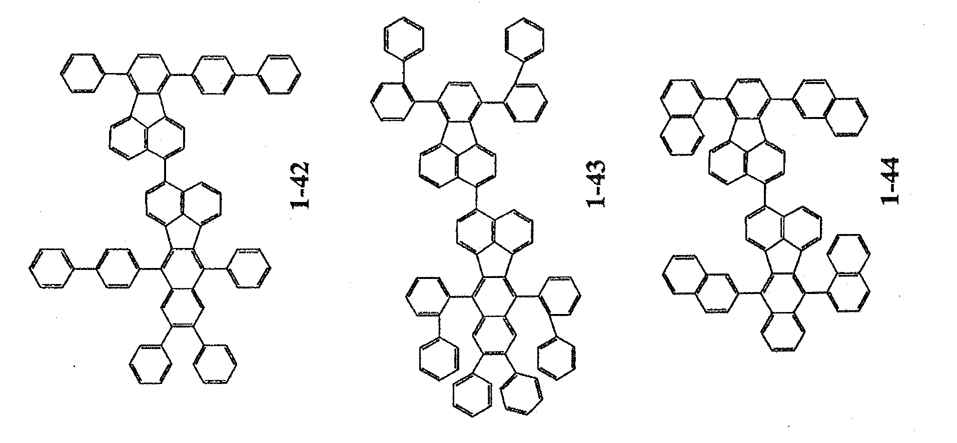

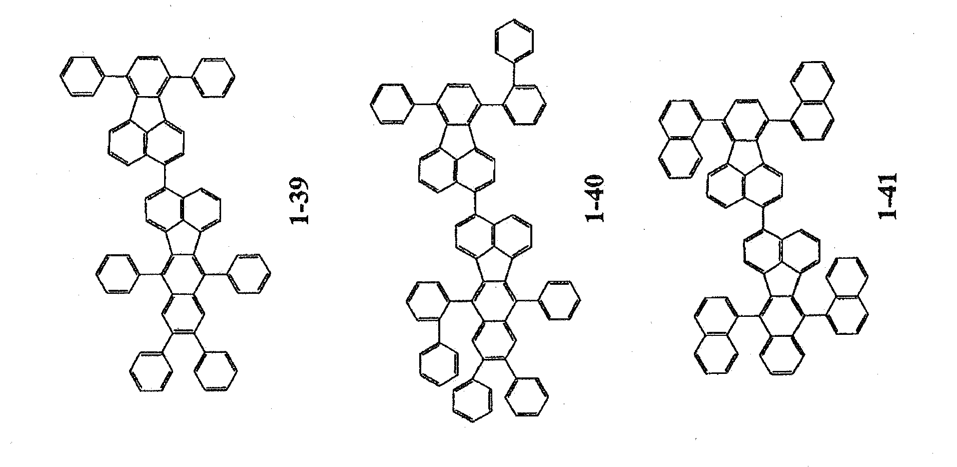

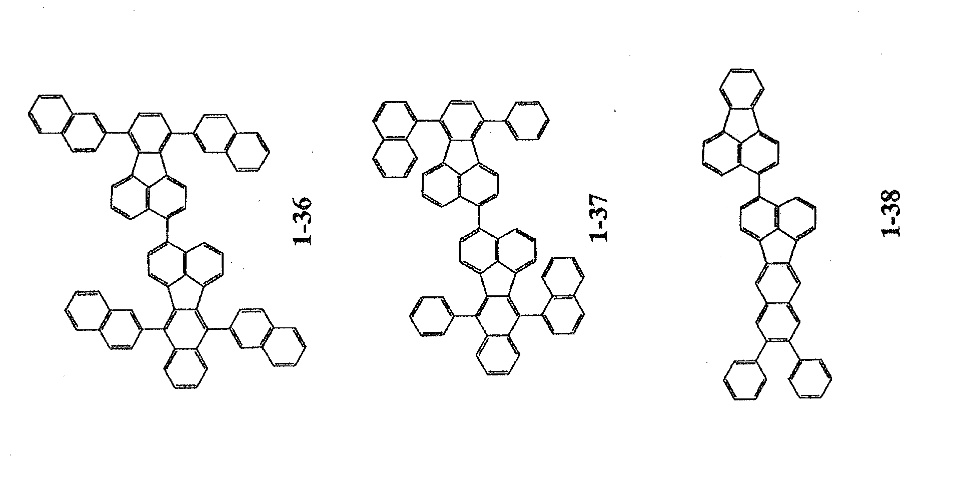

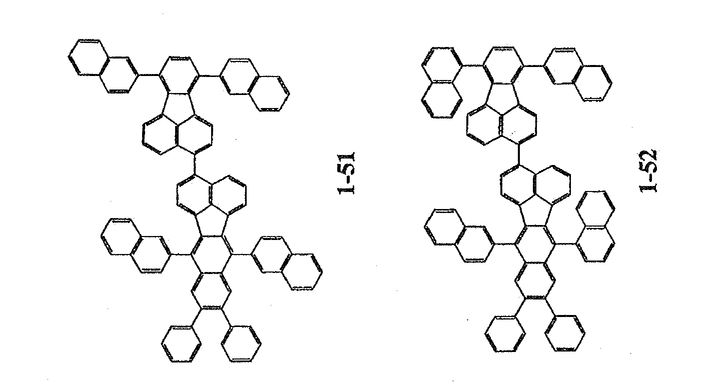

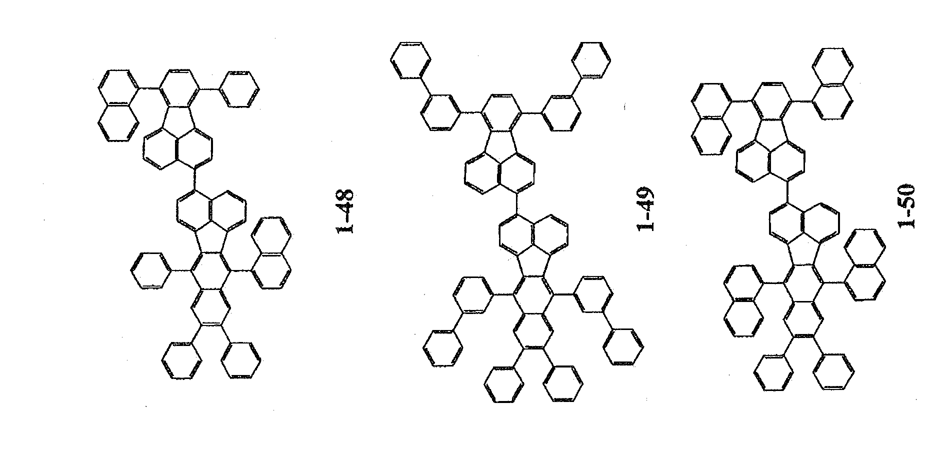

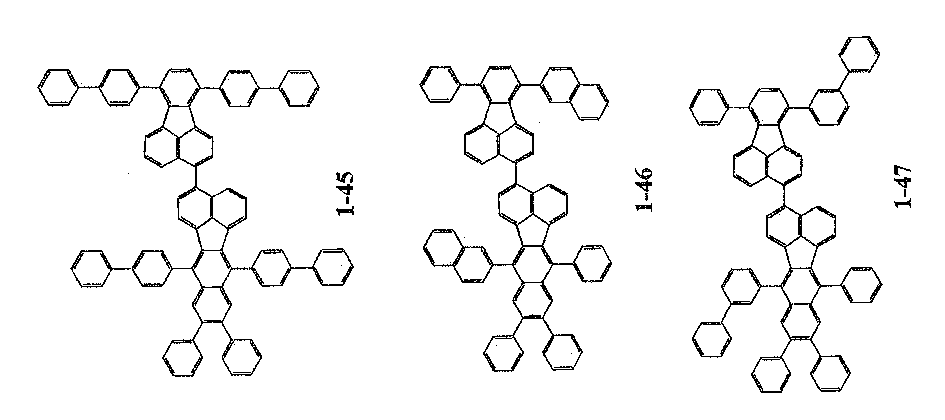

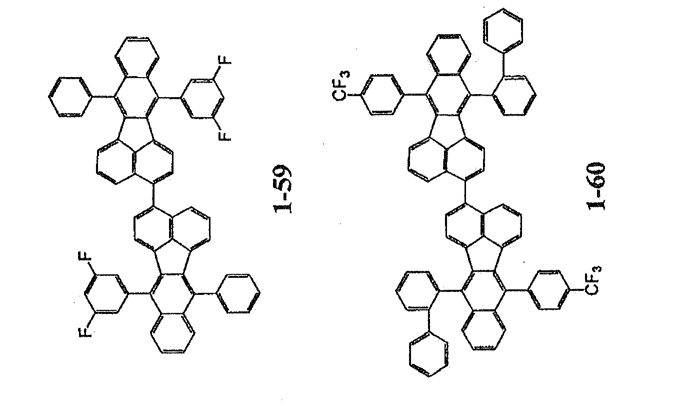

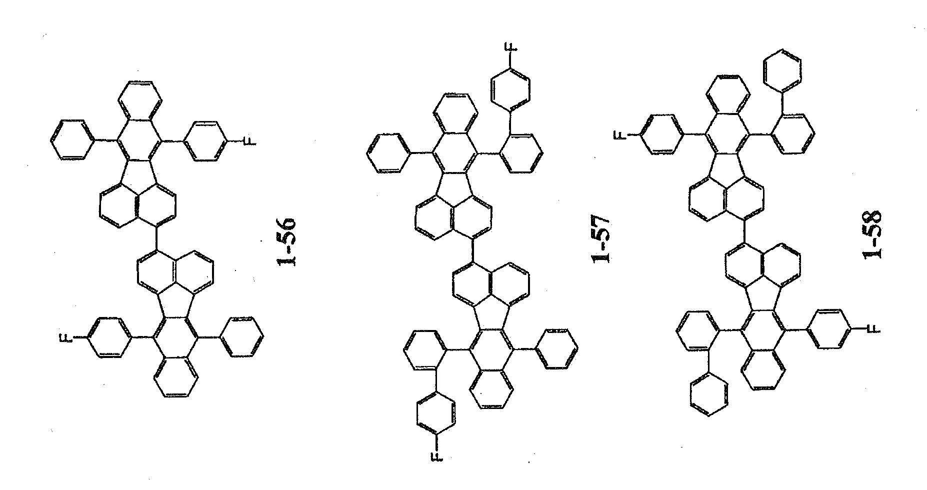

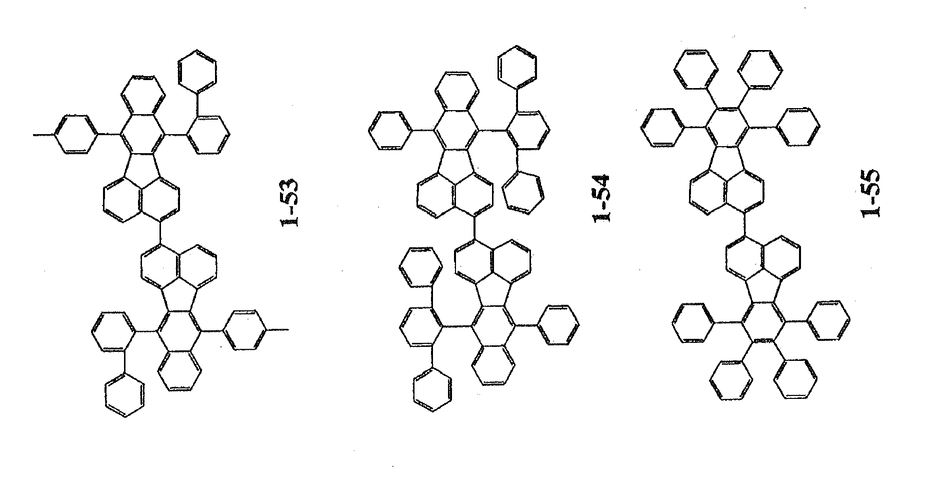

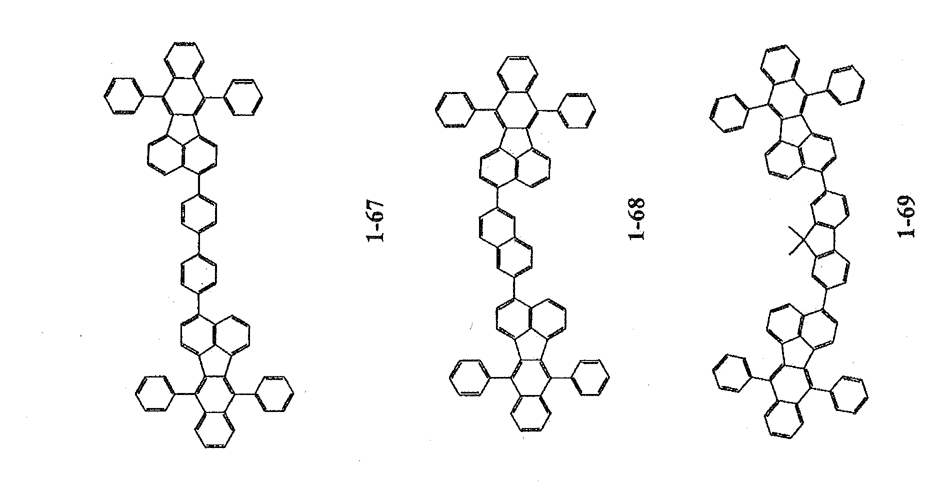

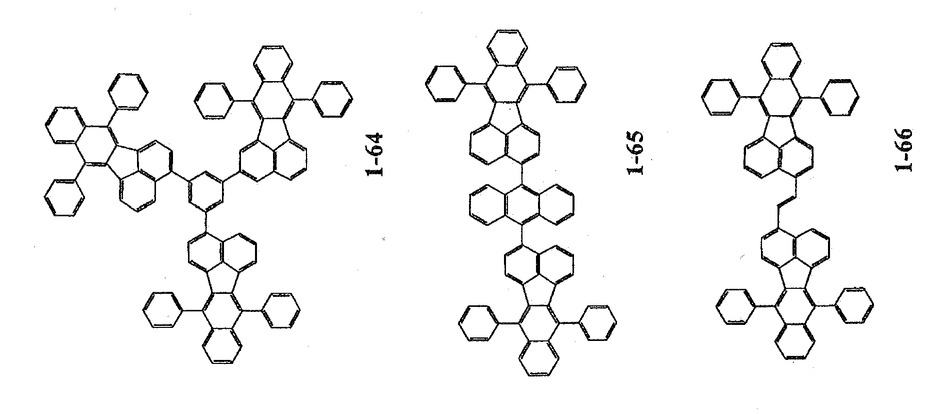

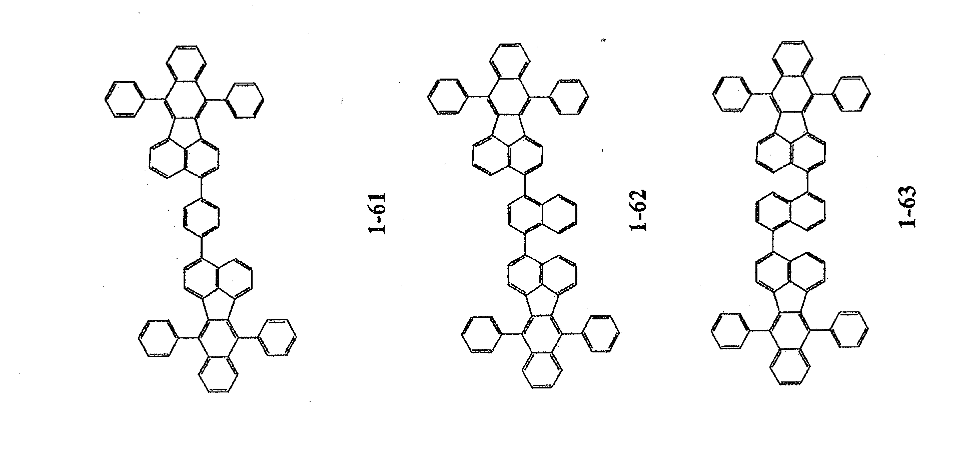

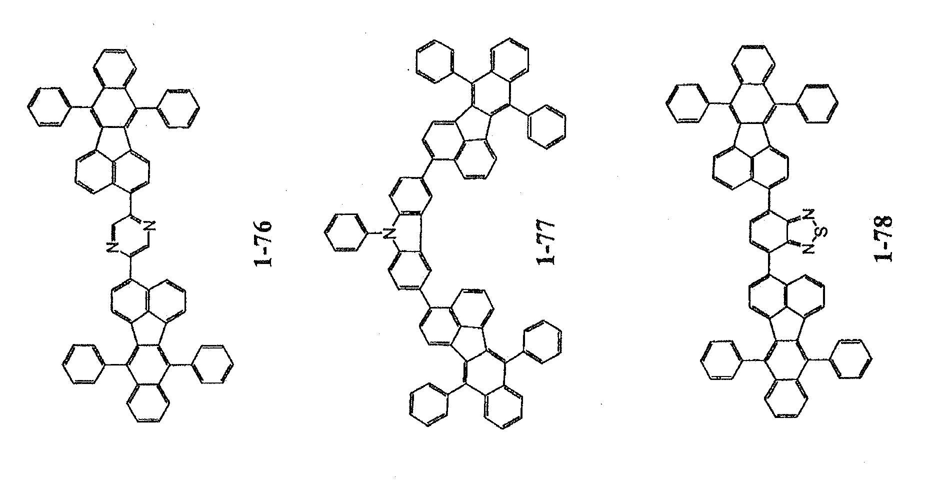

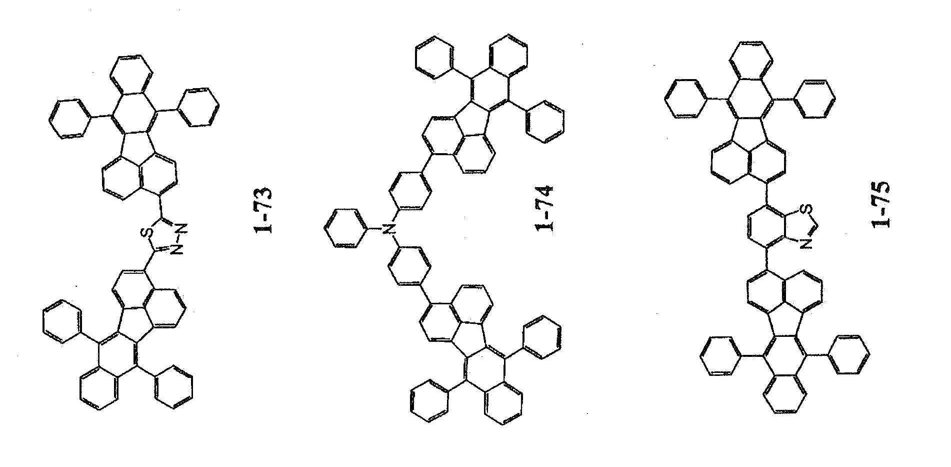

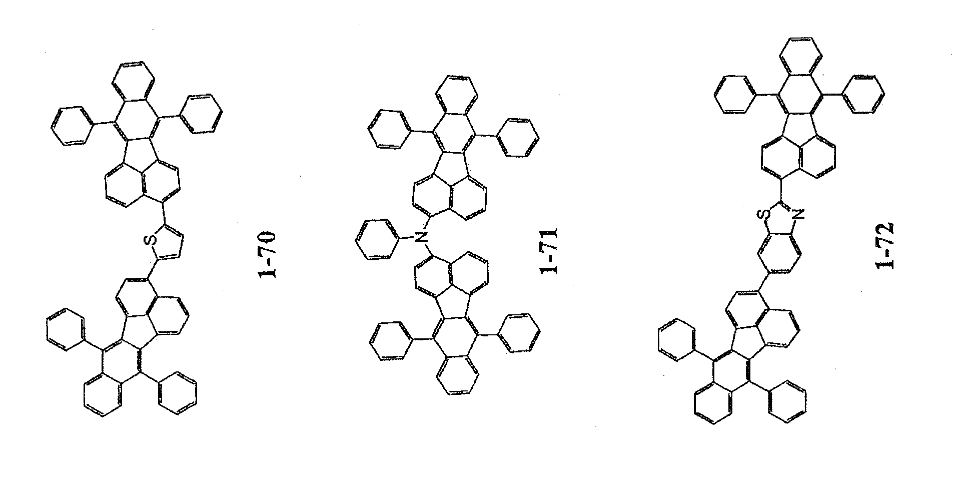

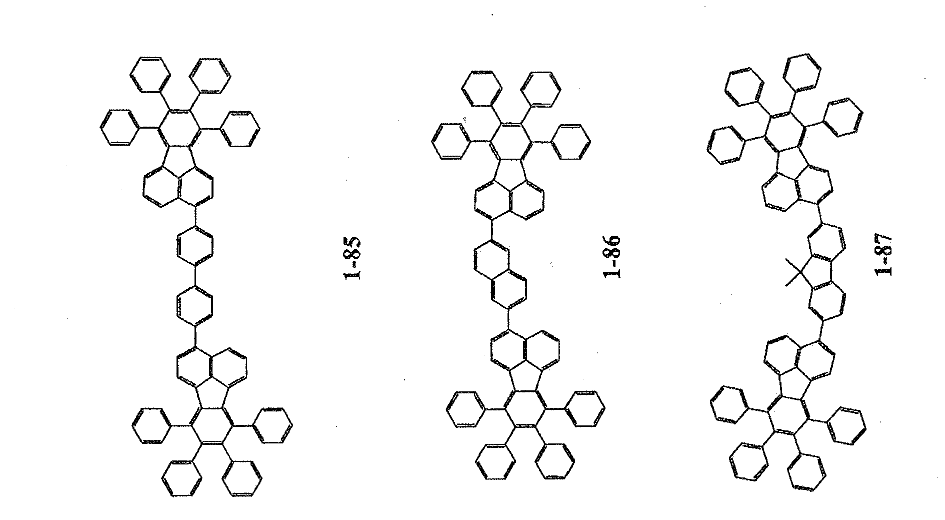

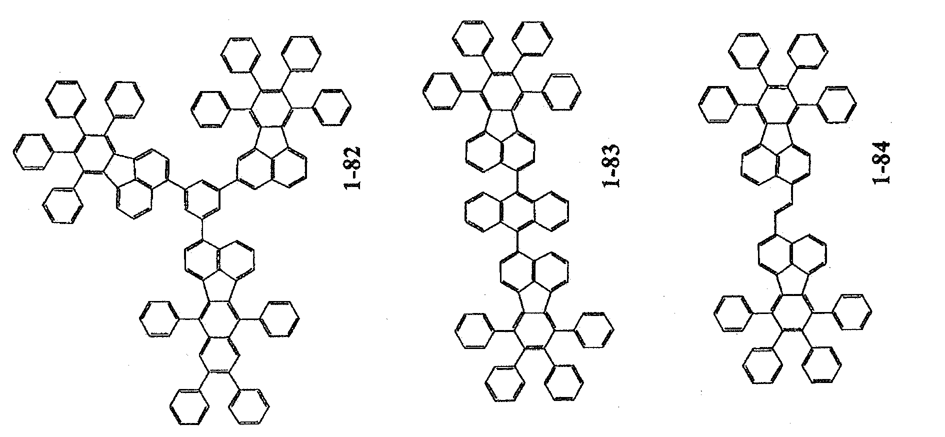

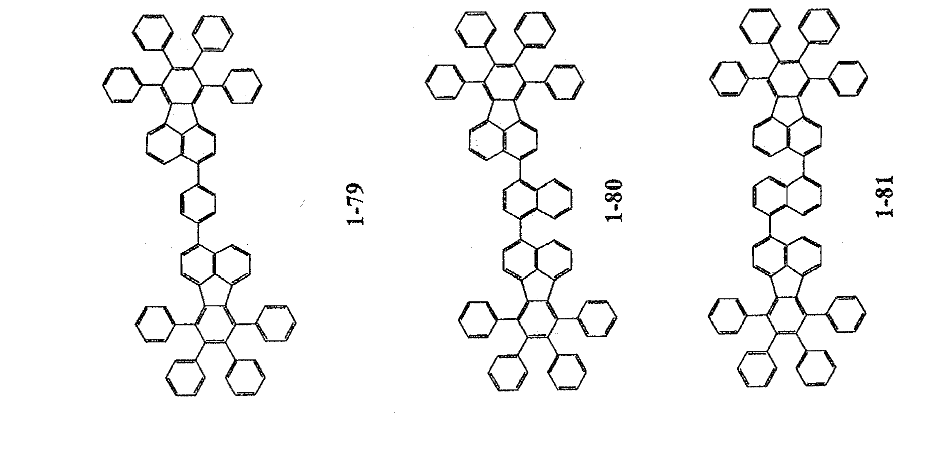

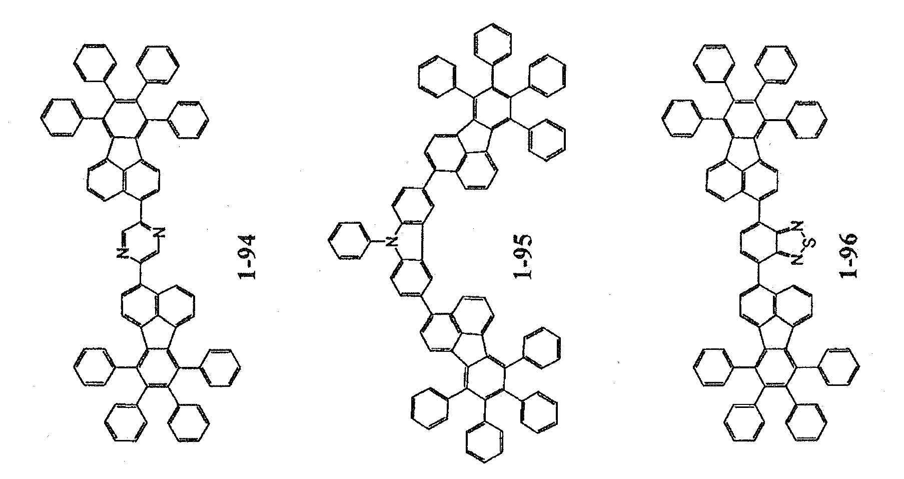

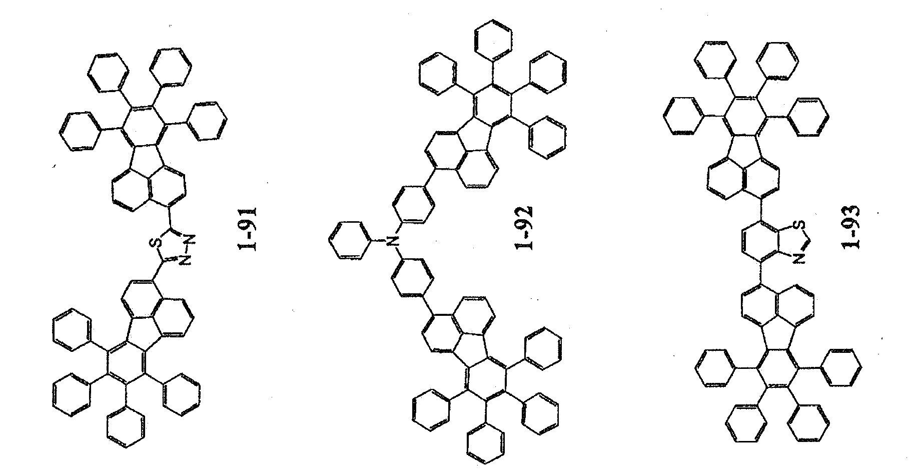

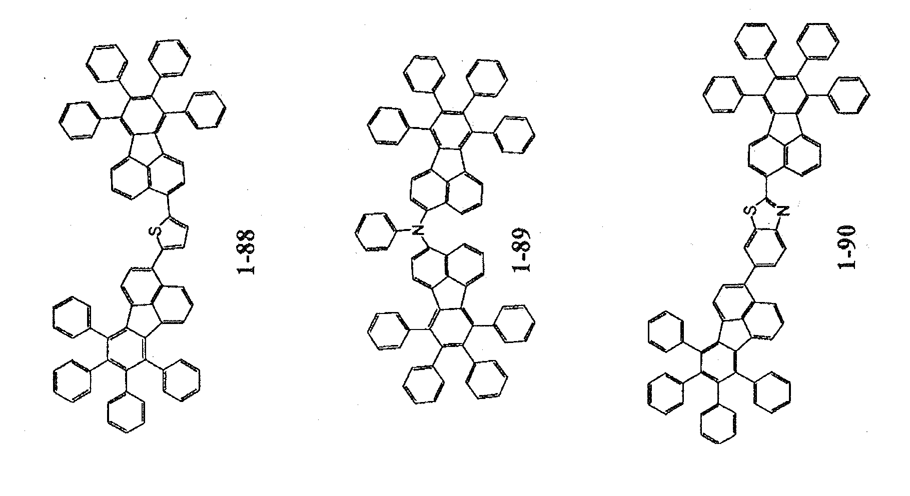

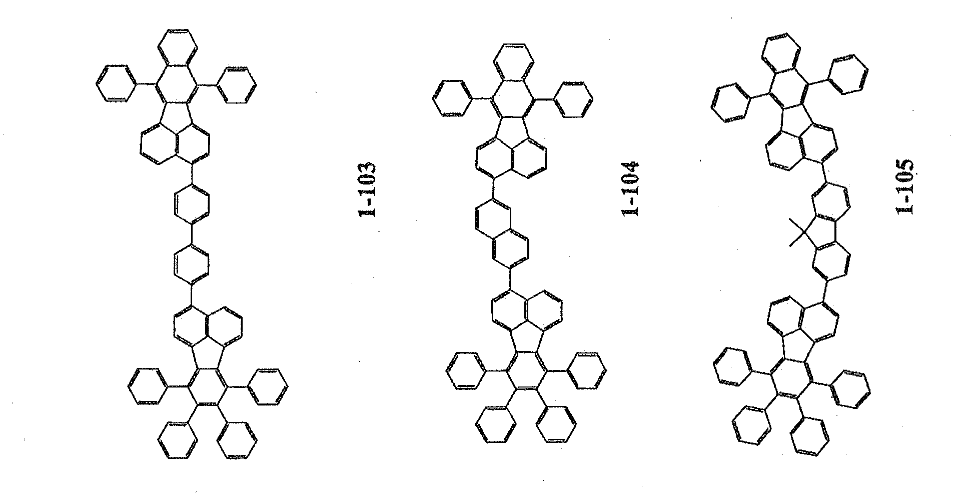

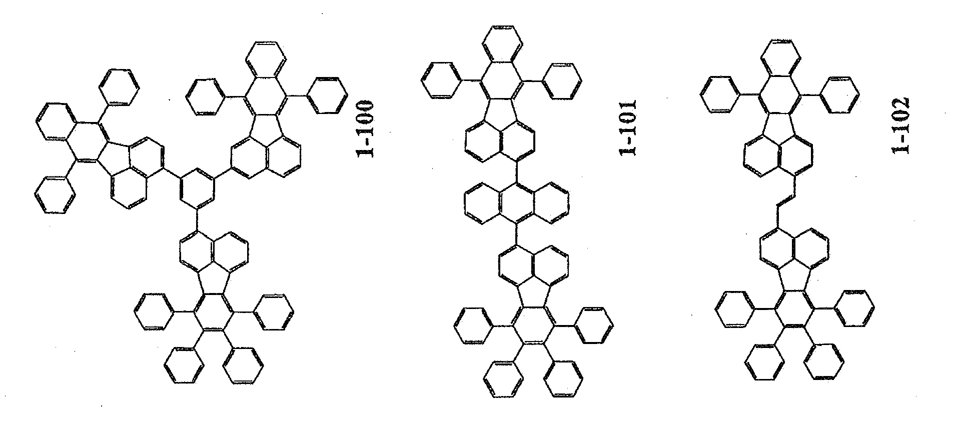

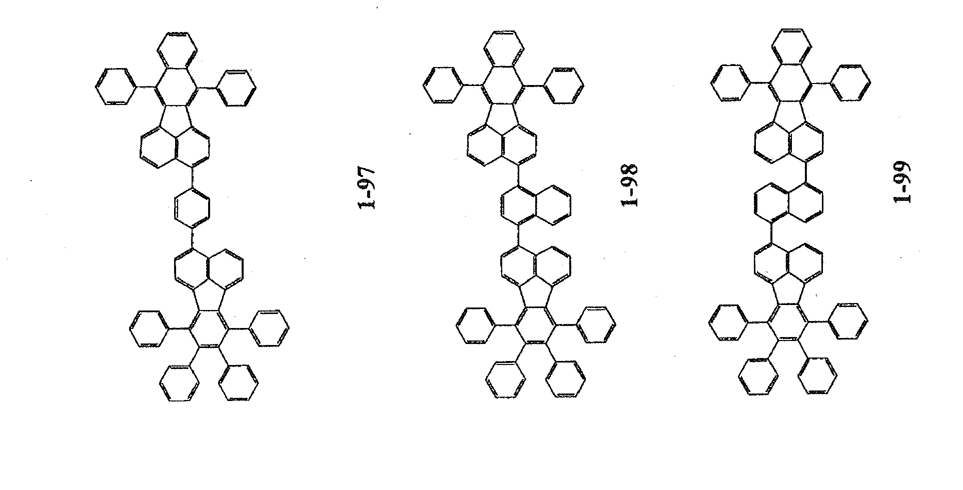

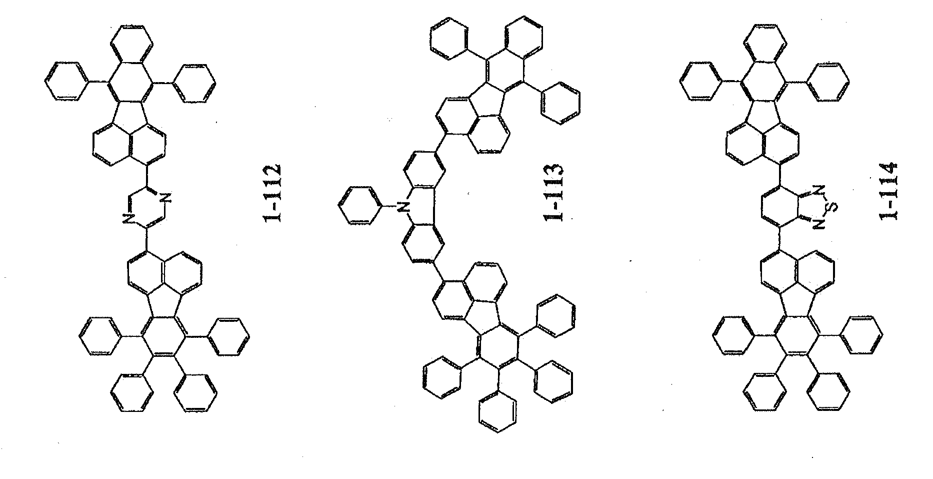

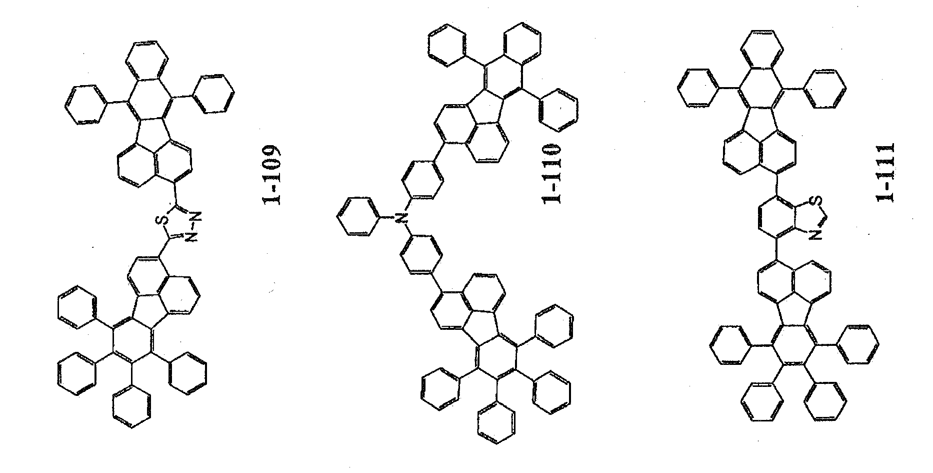

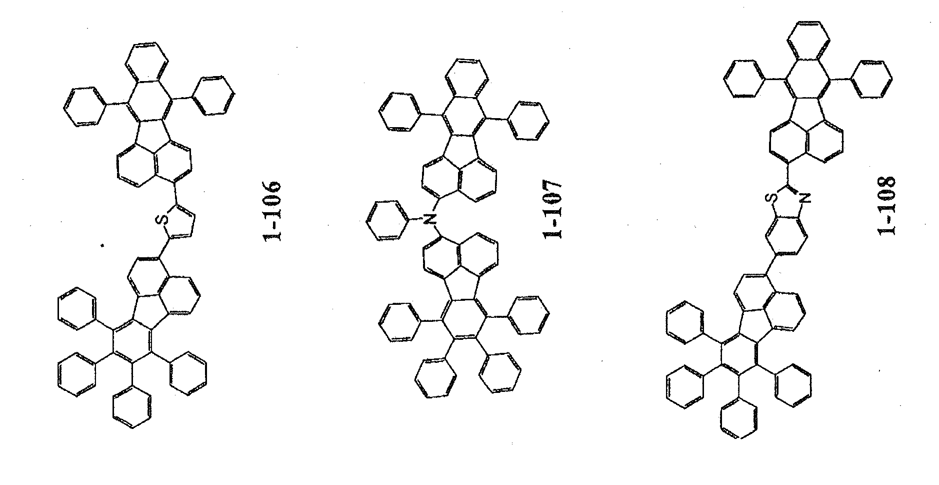

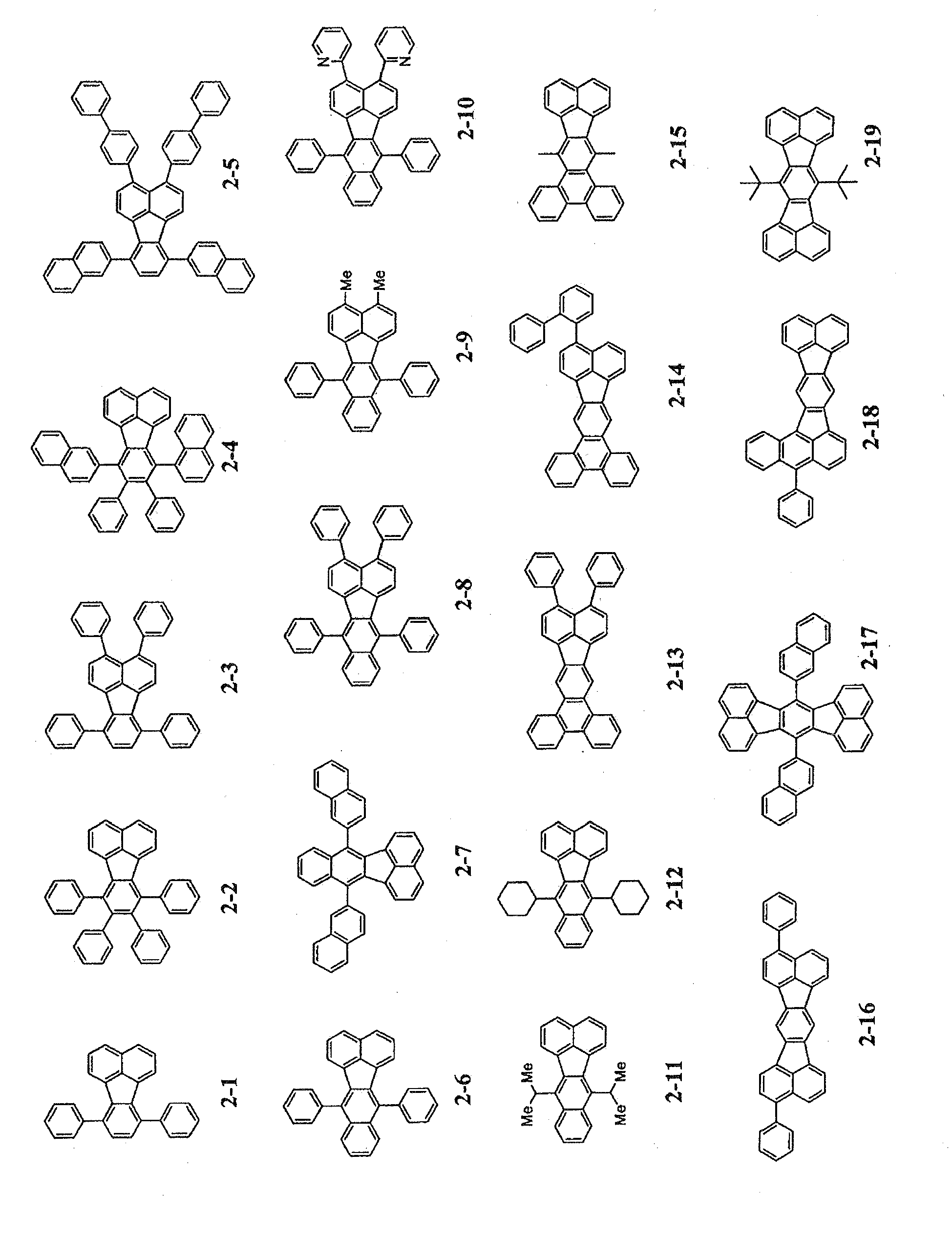

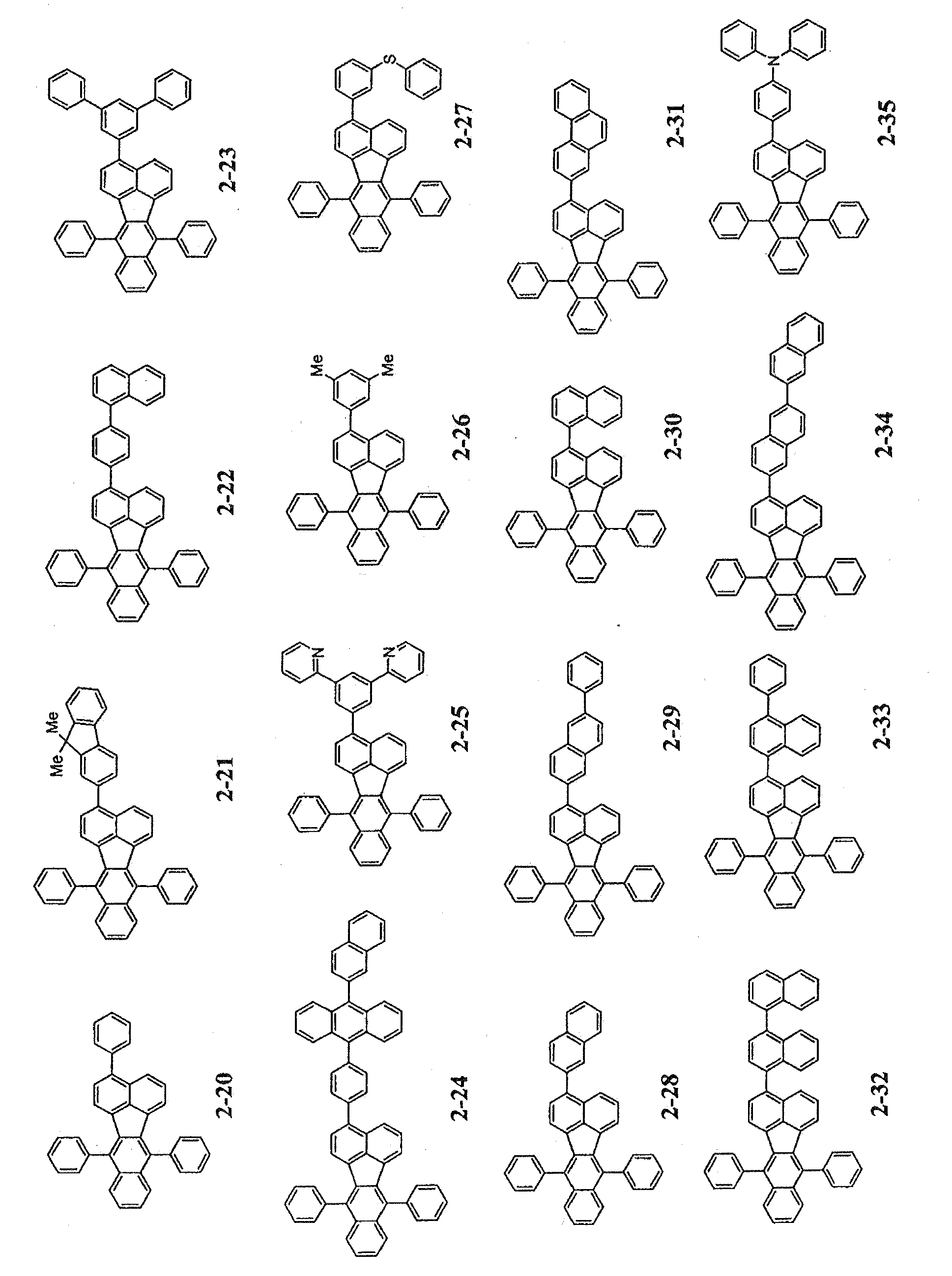

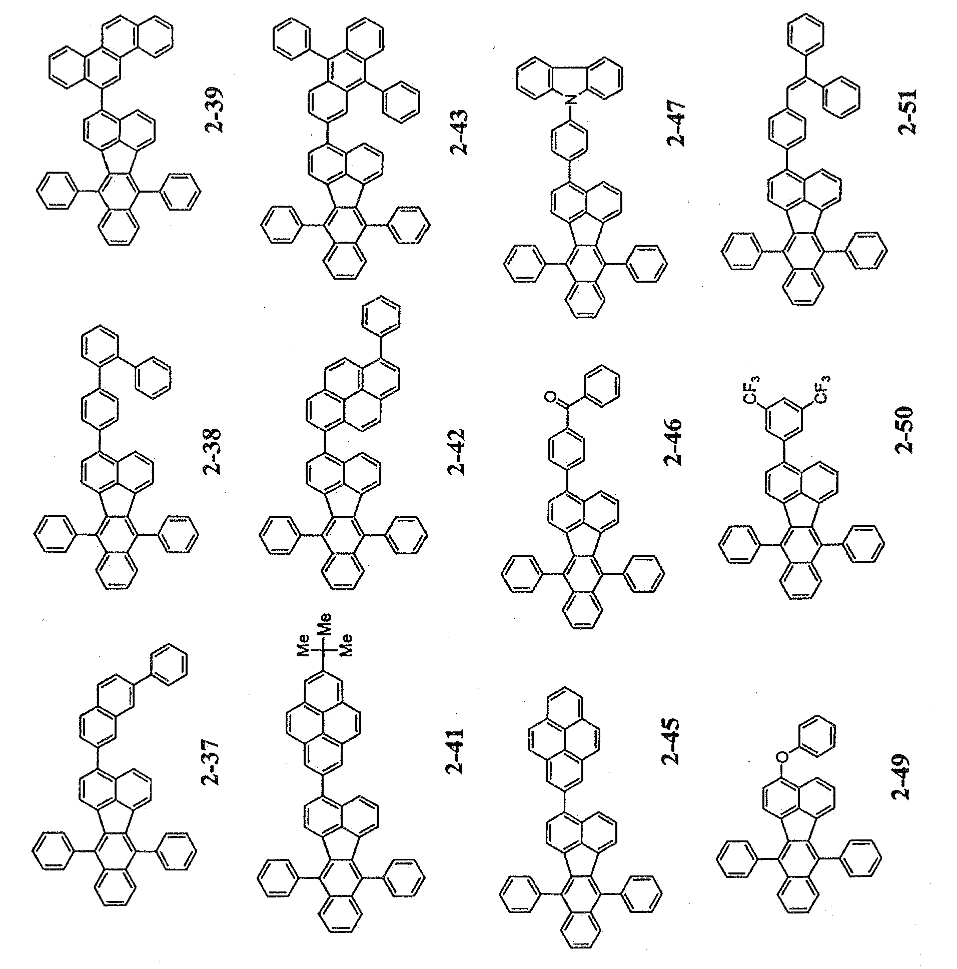

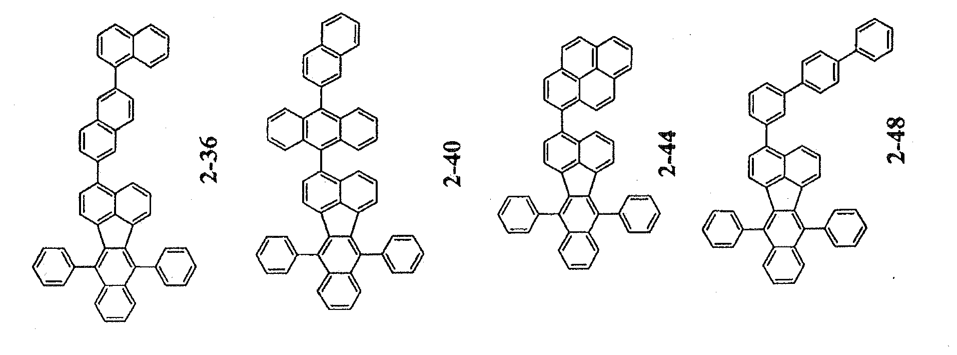

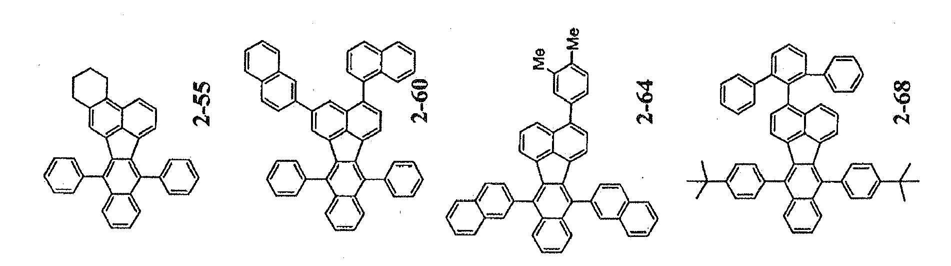

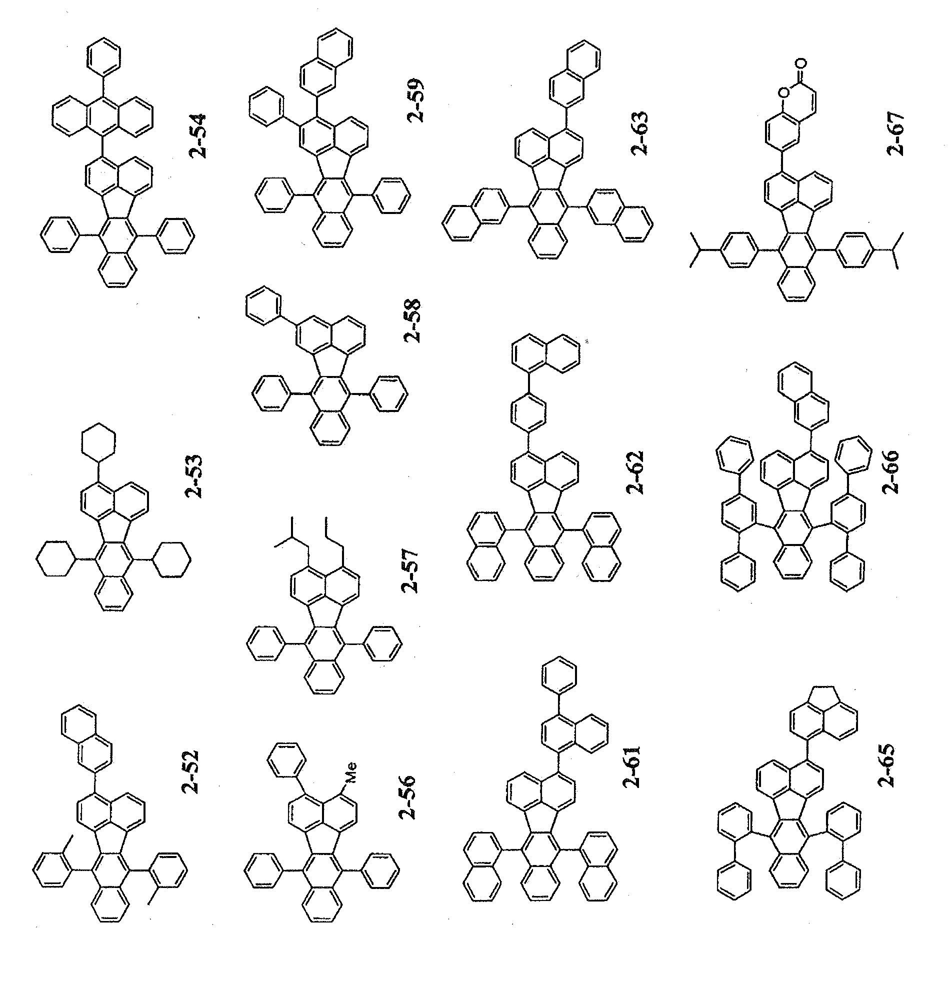

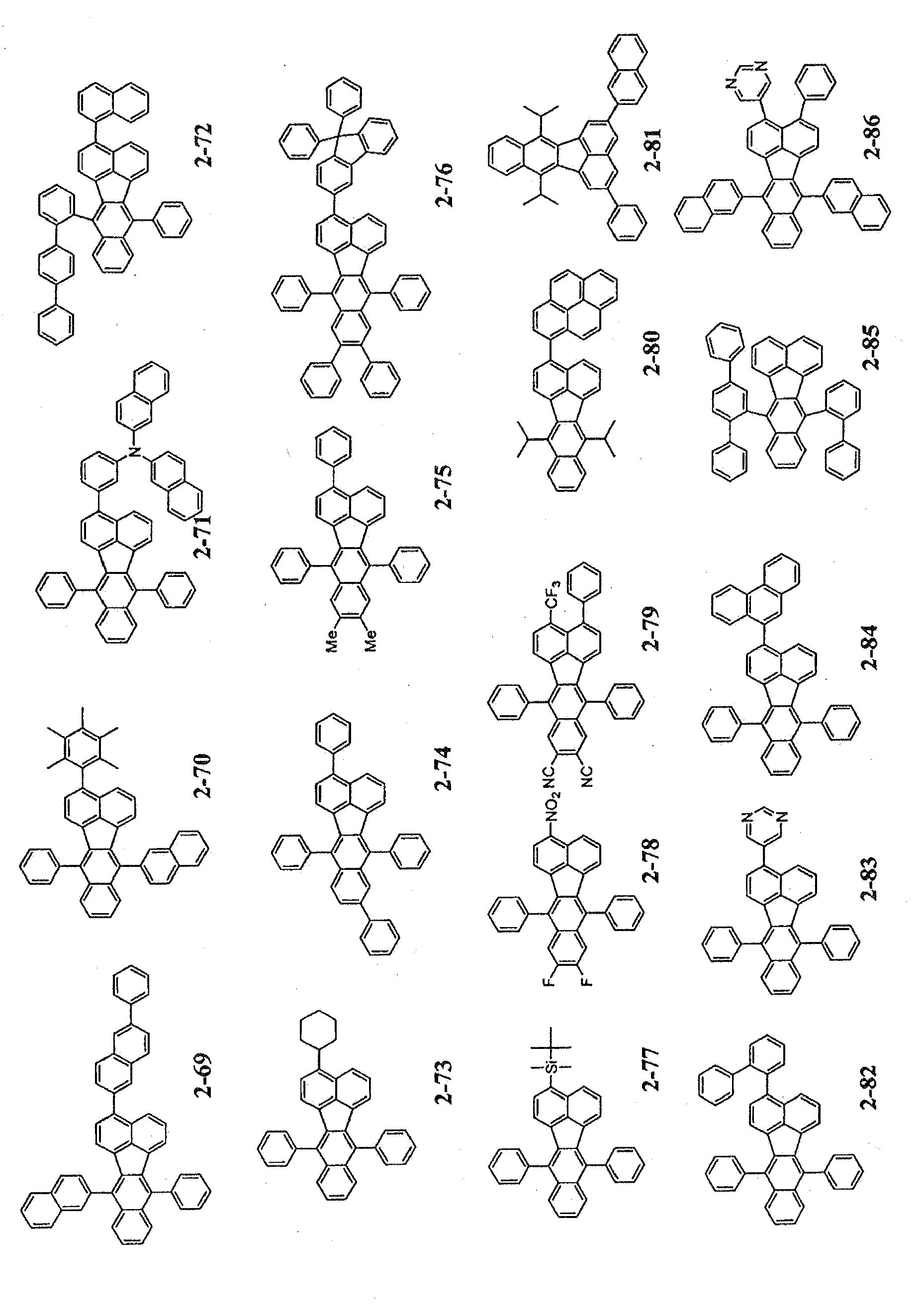

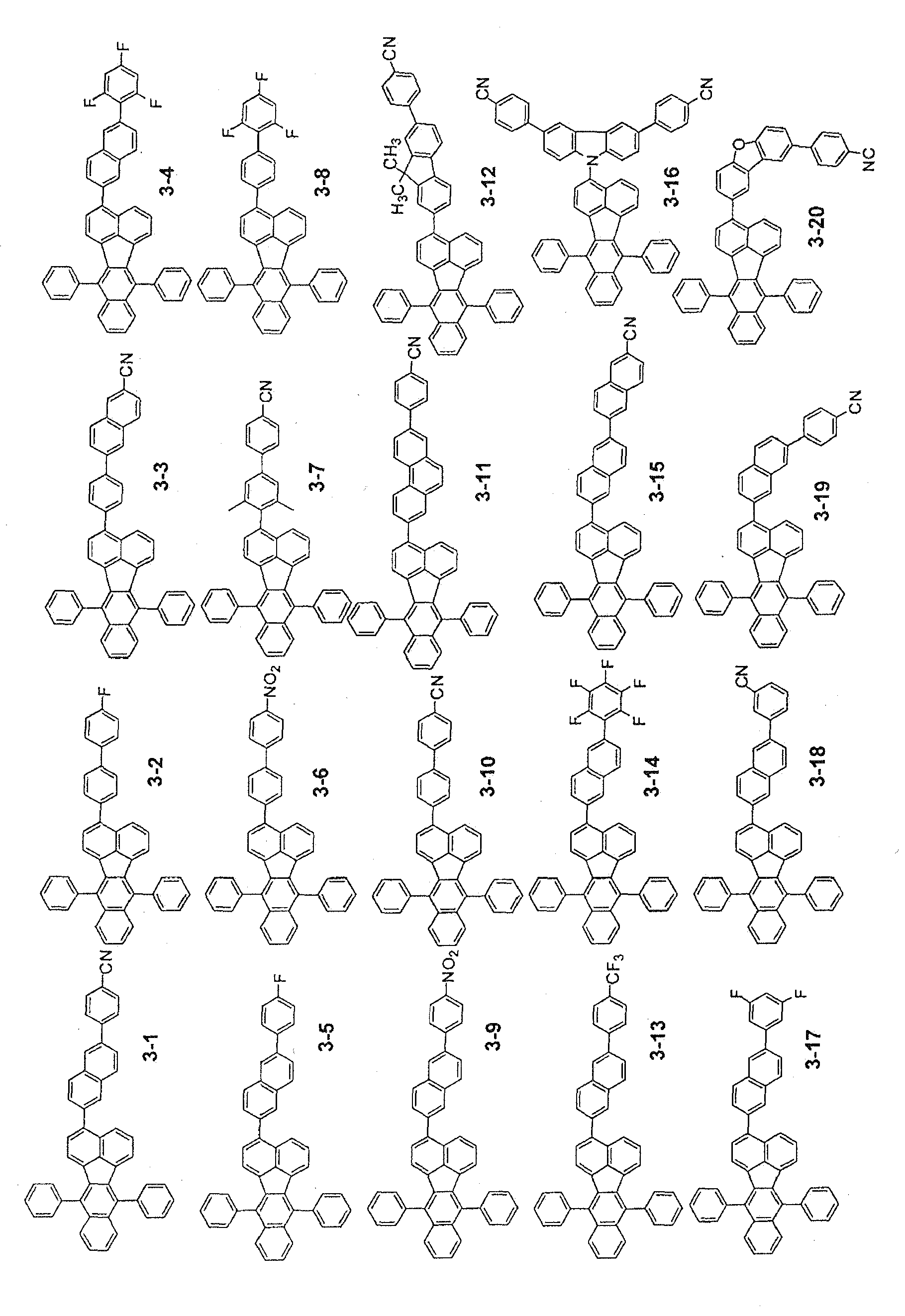

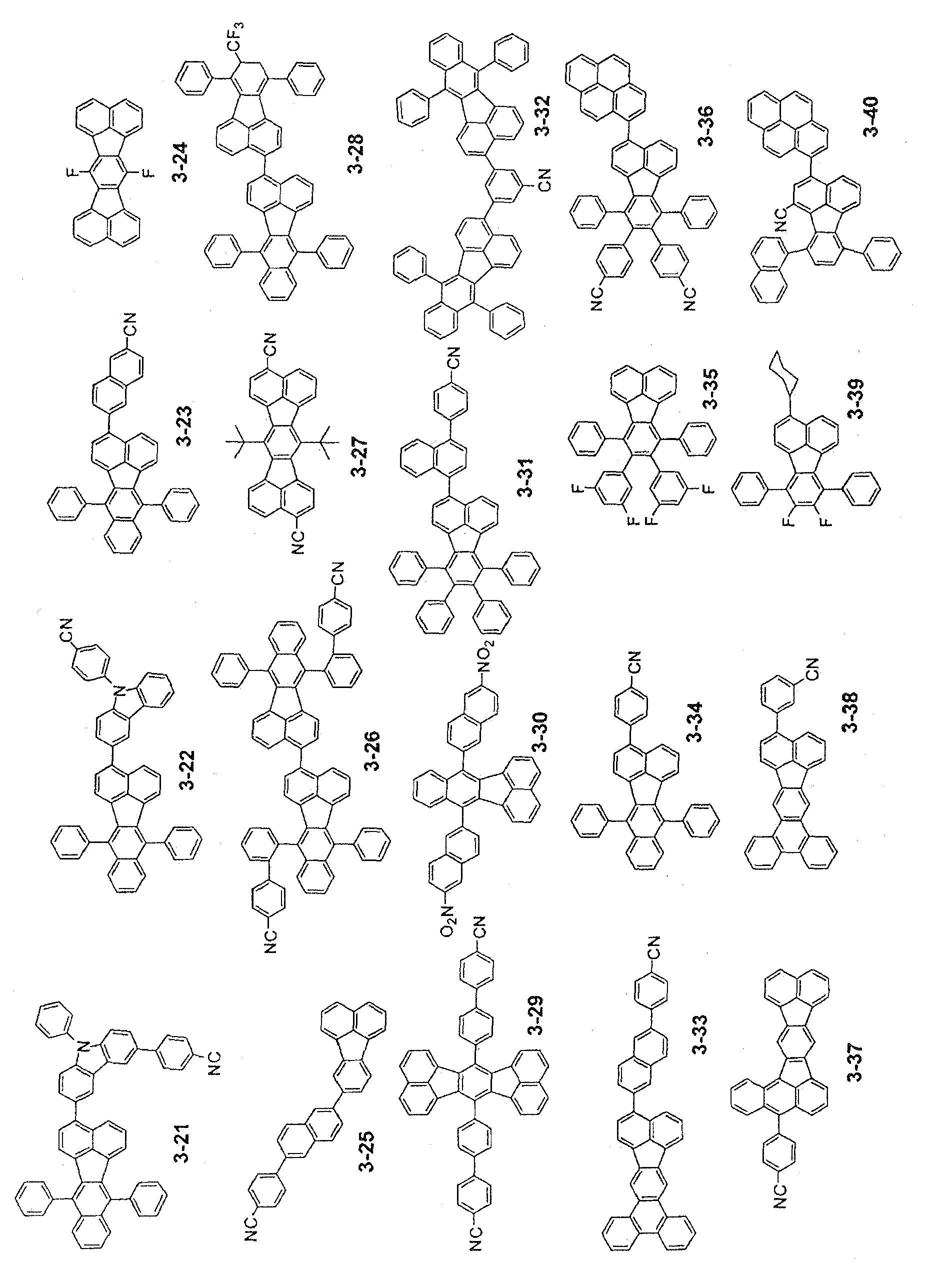

The emitting layer preferably contains the fluoranthene compound in an amount of 0.01 to 20 wt%.

The host compound in the emitting layer is preferably an anthracene derivative.

Specific examples of the anthracene derivative include a compound represented by the following formula (9): 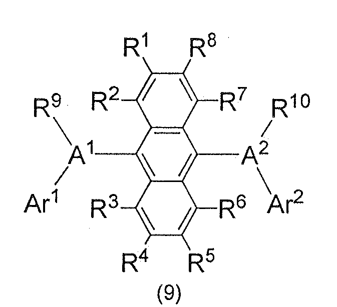 wherein A¹ and A² are independently a hydrogen atom or a substituted or unsubstituted aromatic group having 6 to 50 ring carbon atoms;
Ar¹ and Ar² are independently a hydrogen atom or a substituted or unsubstituted aromatic group having 6 to 50 ring carbon atoms;
R¹ to R¹⁰ are independently a hydrogen atom, a substituted or unsubstituted aromatic group having 6 to 50 ring carbon atoms, a substituted or unsubstituted aromatic hetrocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group or a hydroxyl group; and
Ar¹ and R⁹ and/or Ar² and R¹⁰ may be bonded to each other to form a saturated or unsaturated cyclic structure.

A¹ and A² are preferably a substituted or unsubstituted aromatic group having 6 to 50 carbon atoms, more preferably a phenyl group, α-naphthyl group, β-naphthy! group, anthranil group, phenanthryl group, pyrenyl group, biphenyl group, or terphenyl group.
Ar¹ and Ar² are preferably a hydrogen atom or substituted or unsubstituted aromatic group having 6 to 50 carbon atoms, more preferably a hydrogen atom, phenyl group, α-naphthyl group, β-naphthyl group, anthranil group, phenanthryl group, pyrenyl group, biphenyl group, or terphenyl group.
R¹ to R¹⁰ are preferably a hydrogen atom, substituted or unsubstituted aromatic group having 6 to 50 carbon atoms, or alkyl group having 1 to 6 carbon atoms, more preferably a hydrogen atom, phenyl group, α-naphthyl group, β-naphthyl group, anthranil group, phenanthryl group, pyrenyl group, biphenyl group, terphenyl group, or alkyl group having 1 to 6 carbon atoms.

The anthracene derivative is preferably a compound in which the groups are not symmetrically bonded to 9 and 10 positions of the central anthracene with respect to the X-Y axis in the following formula.

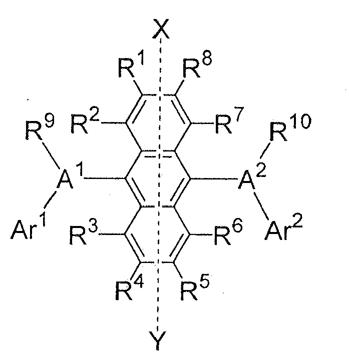

Following compounds are given as the anthracene compound.

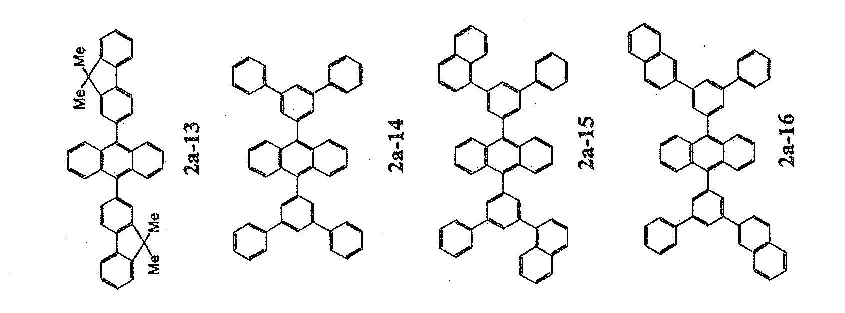

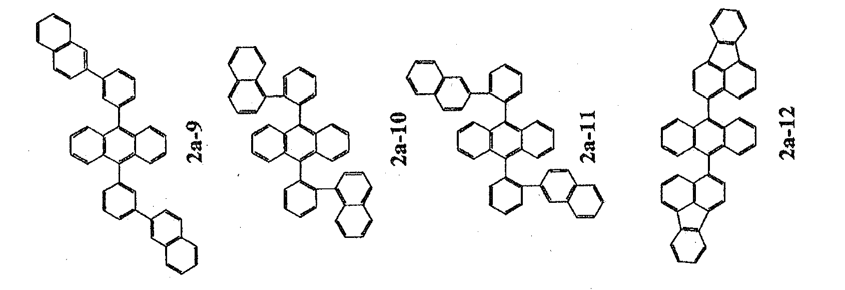

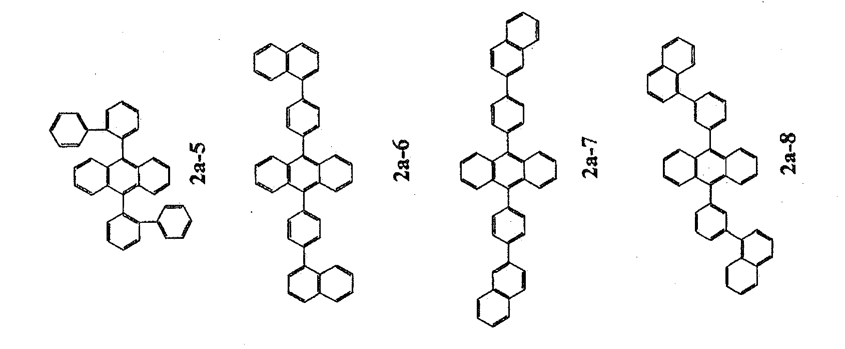

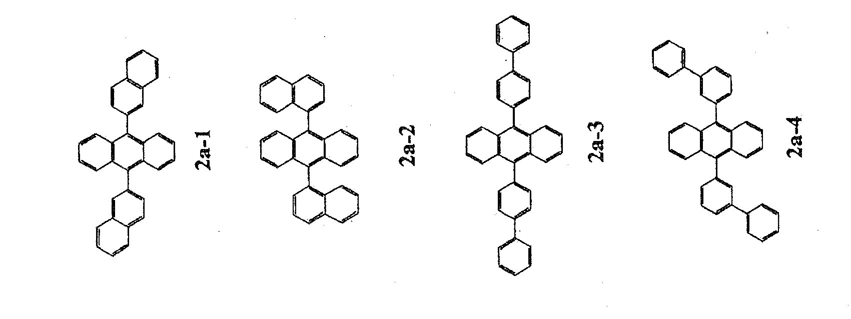

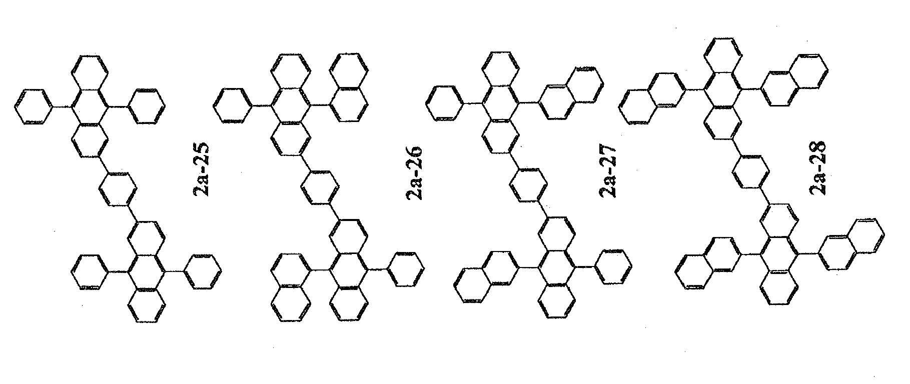

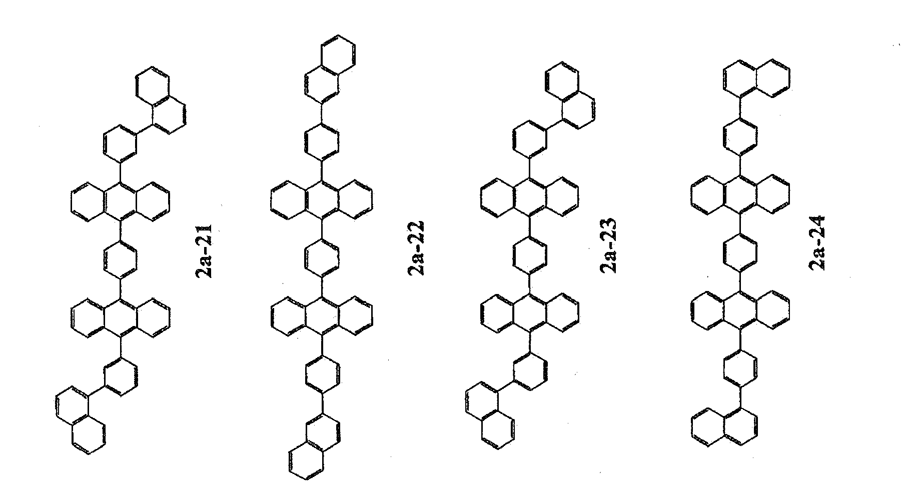

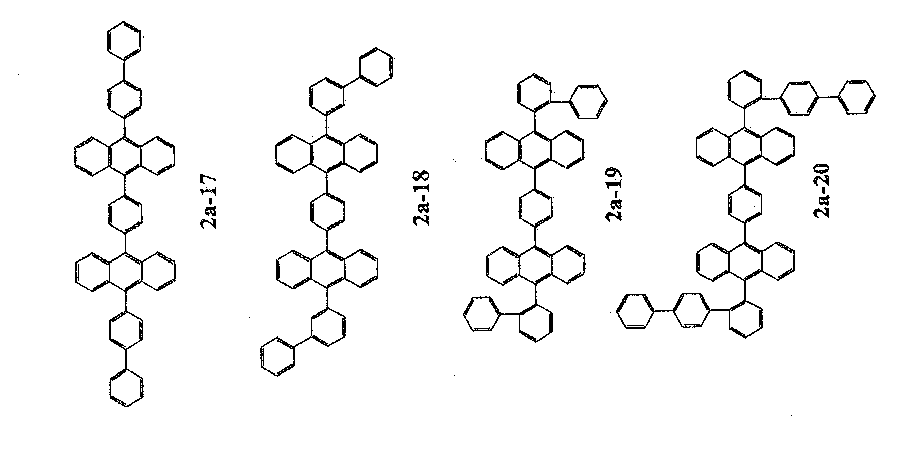

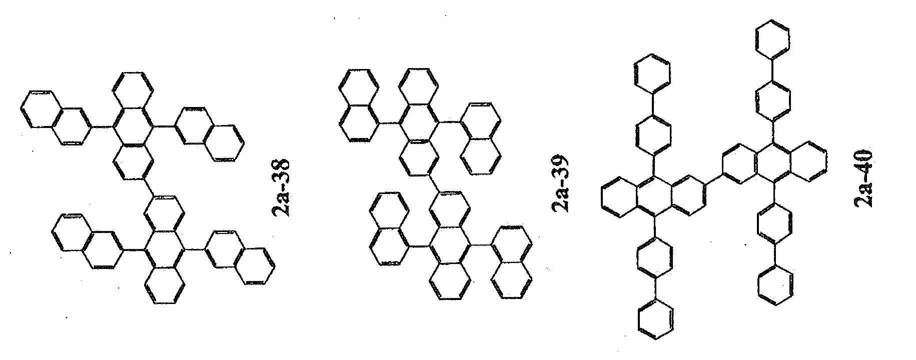

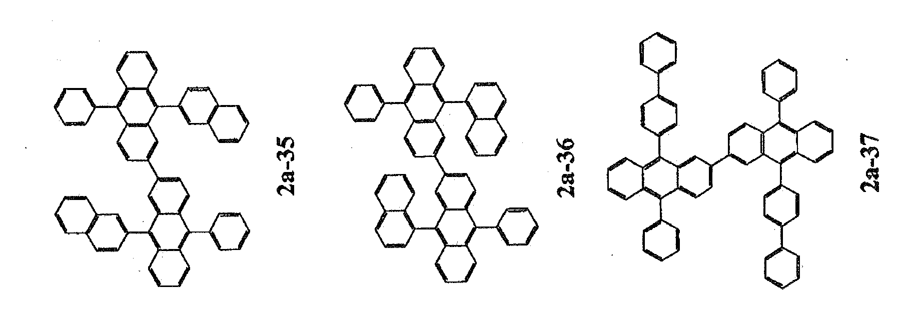

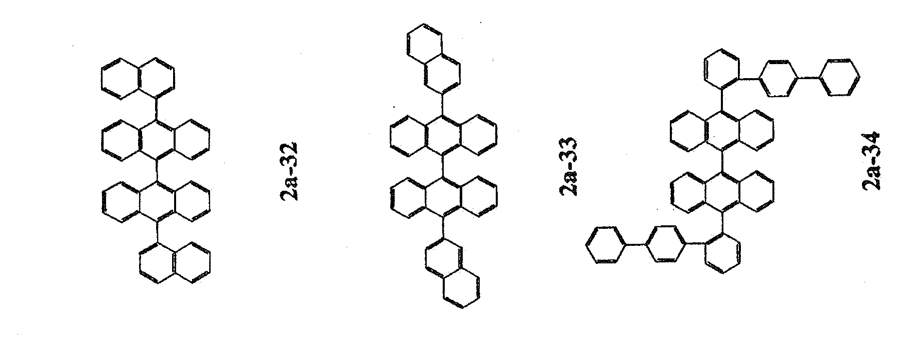

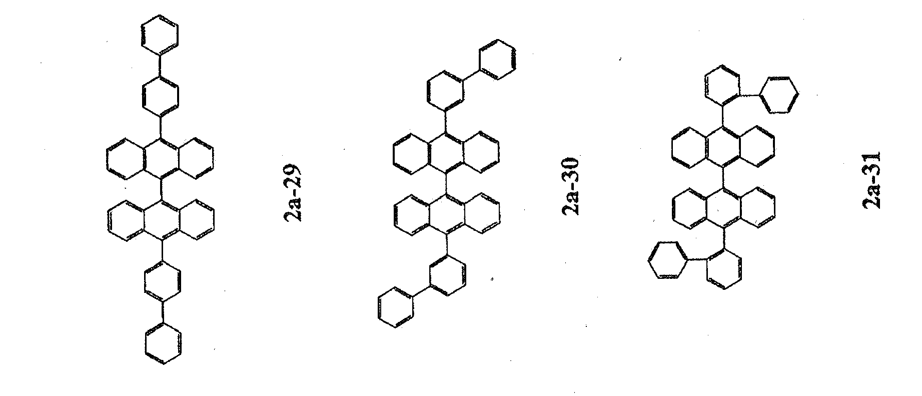

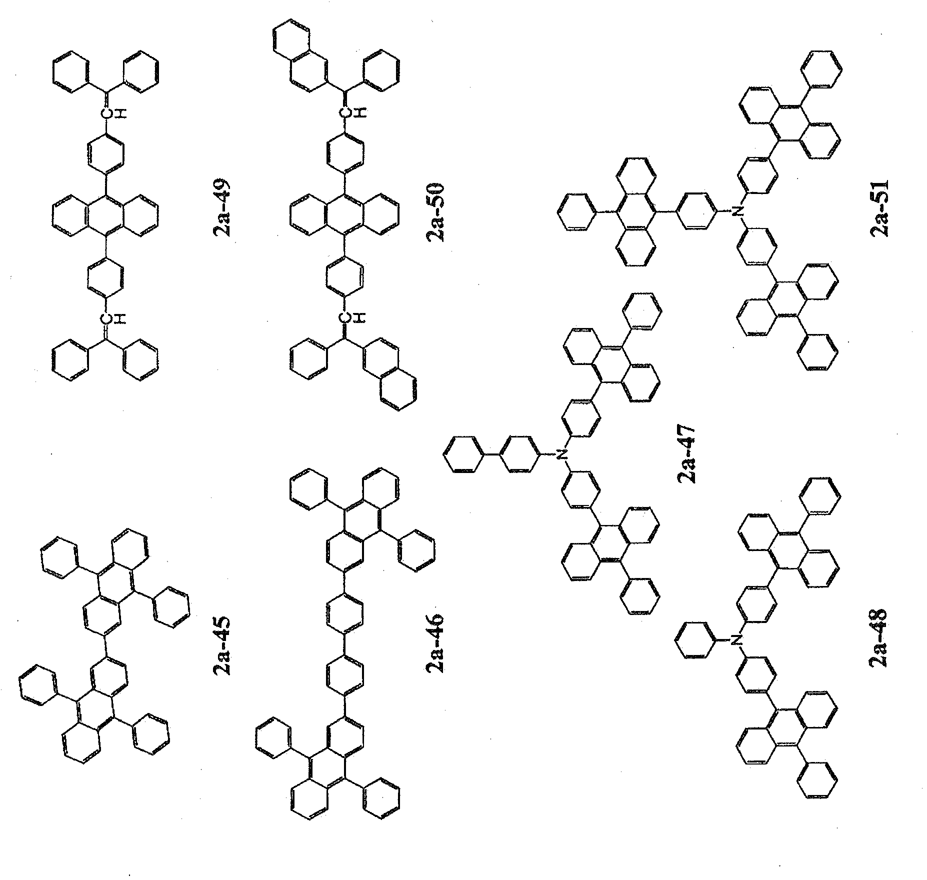

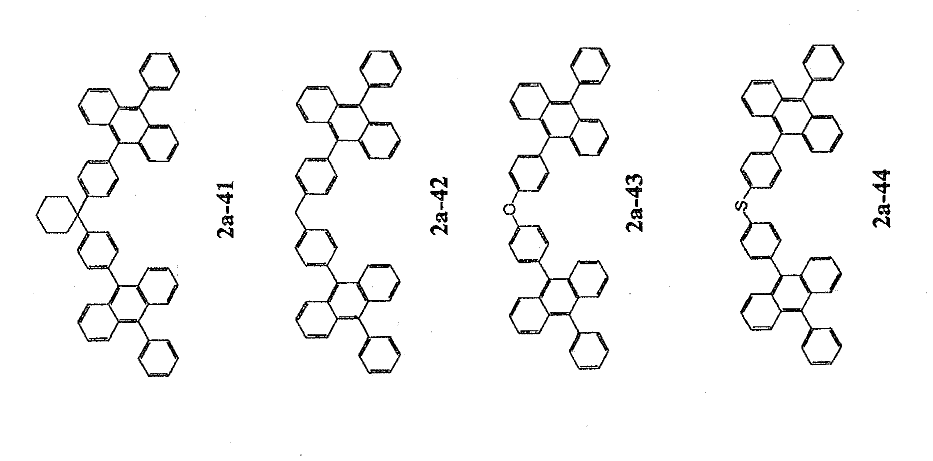

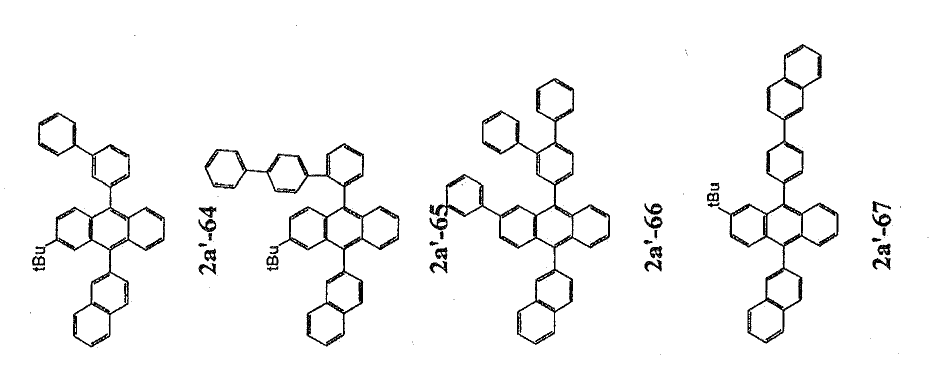

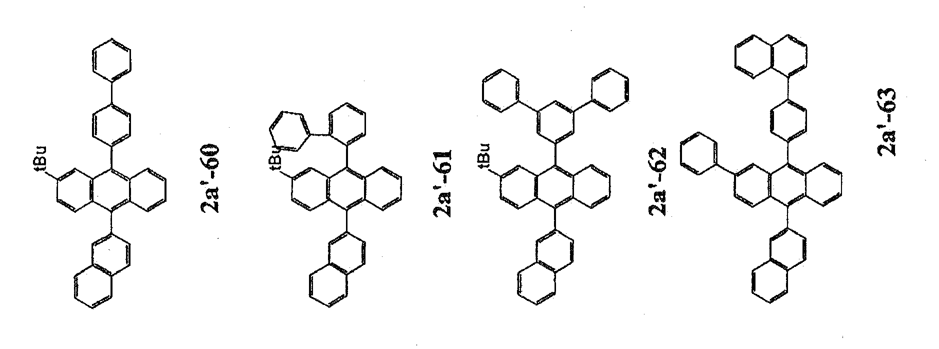

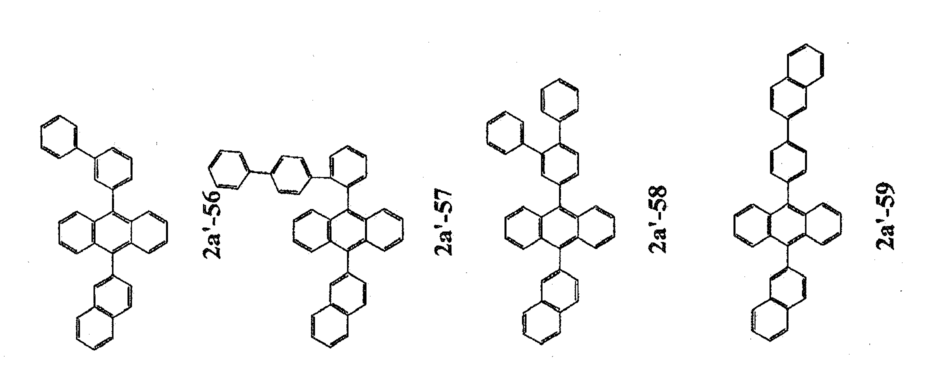

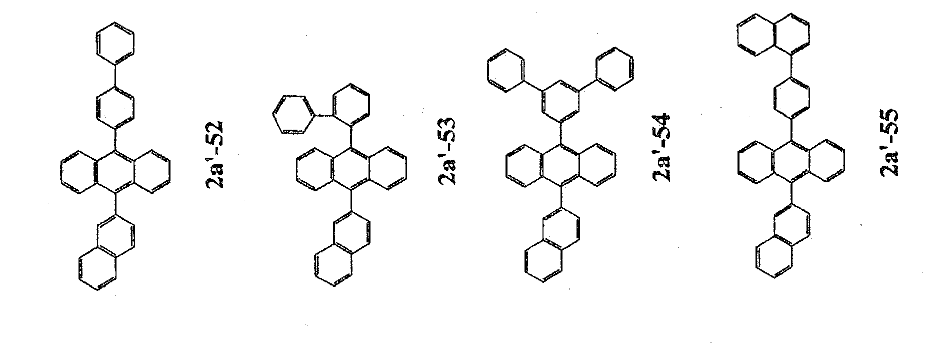

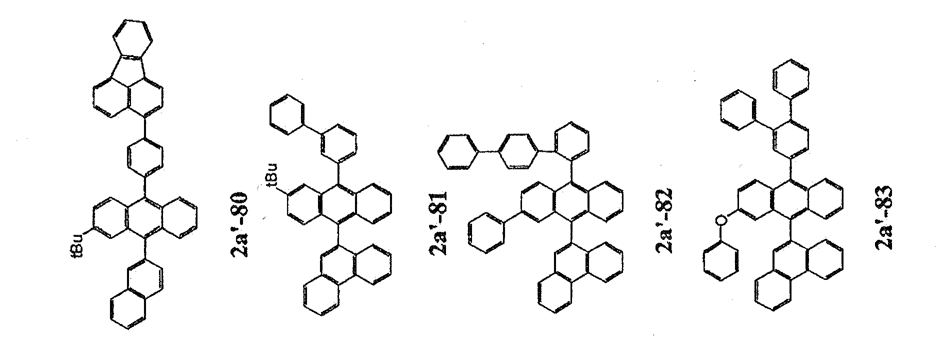

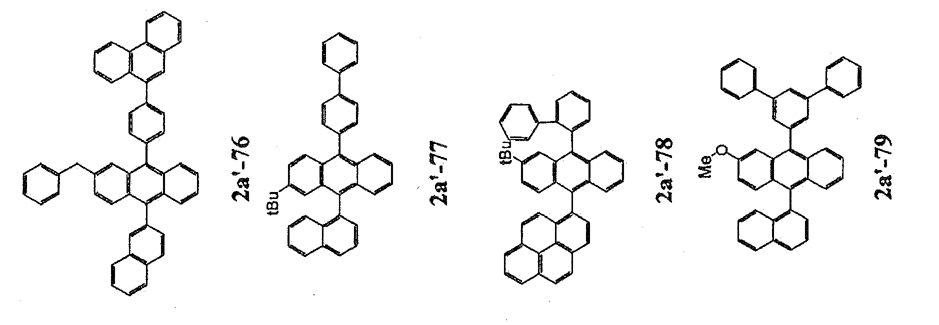

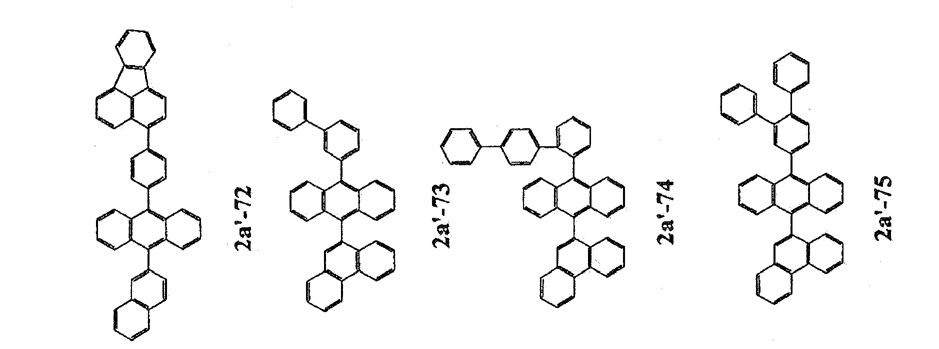

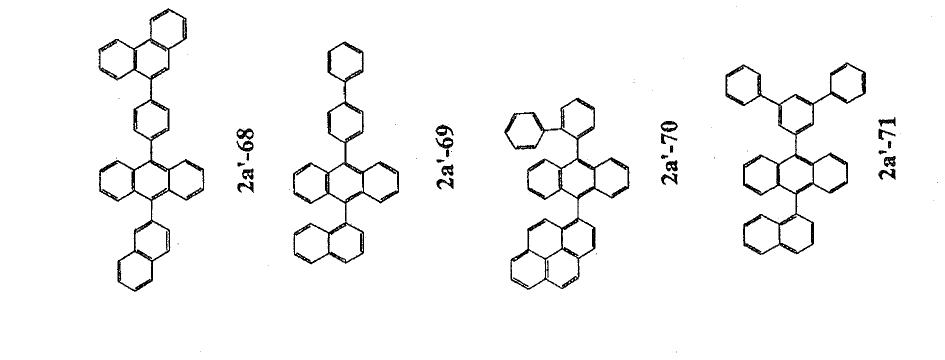

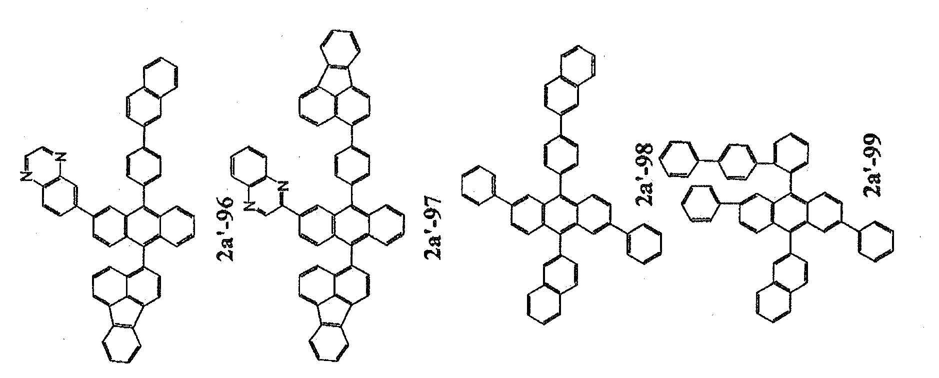

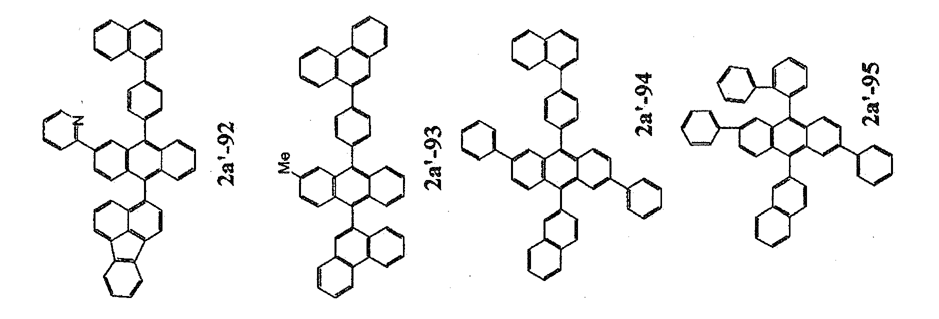

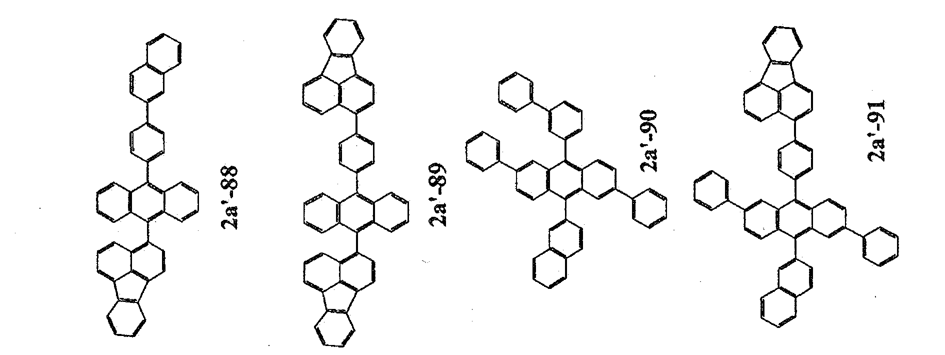

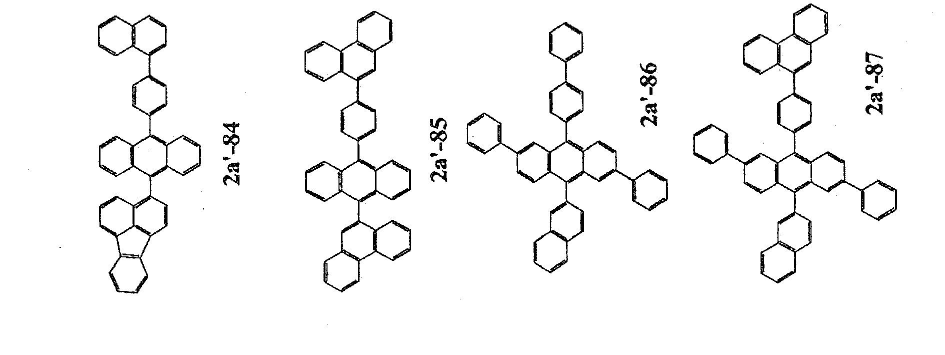

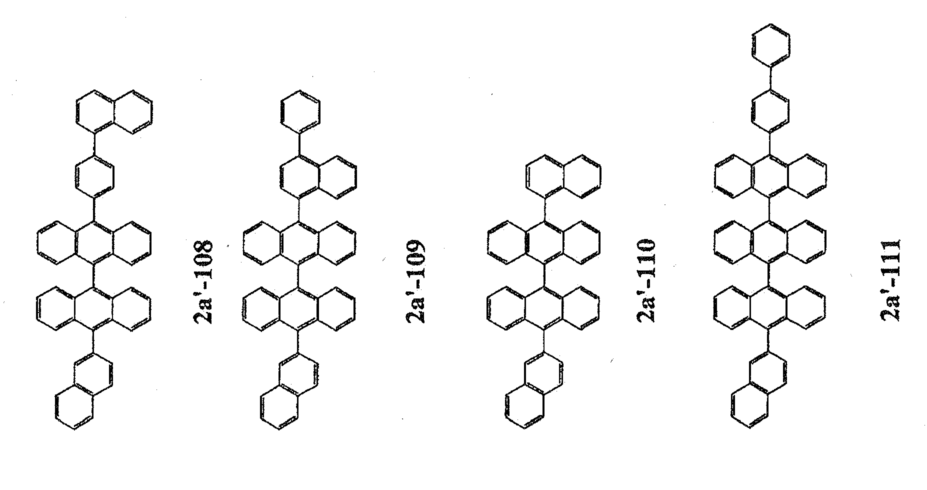

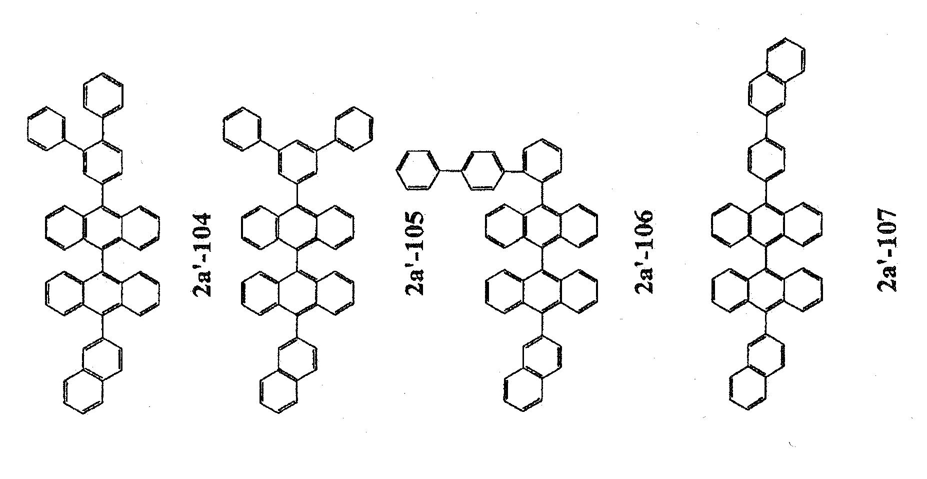

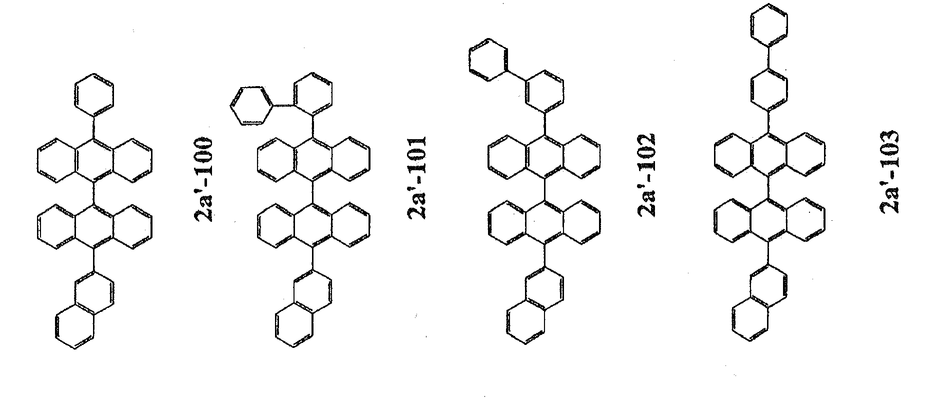

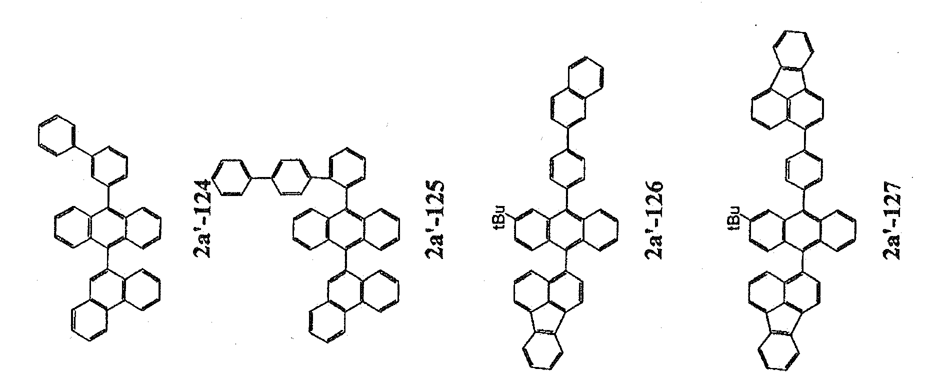

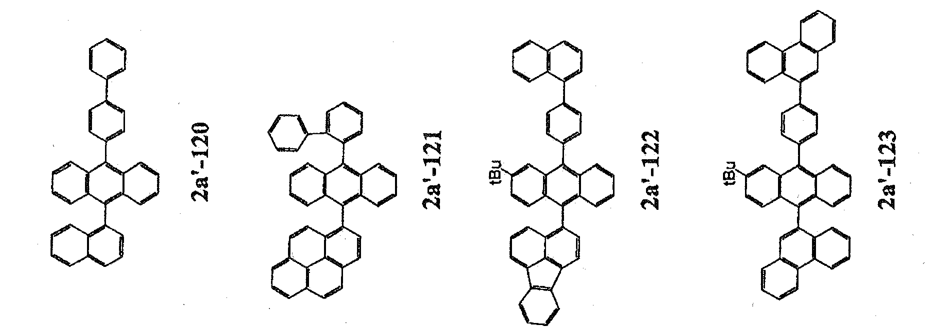

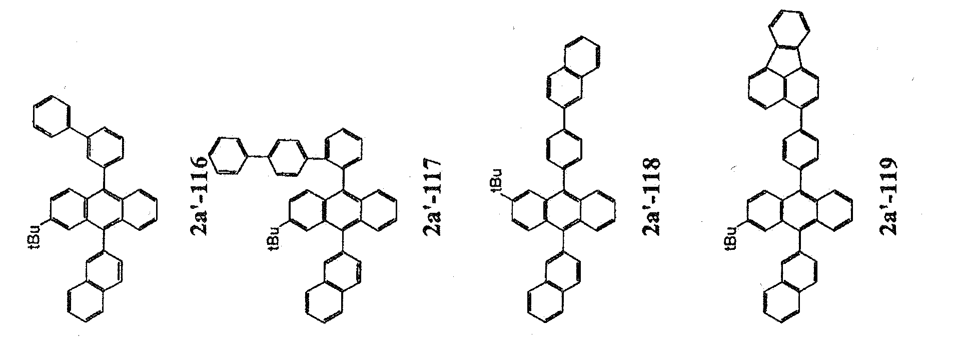

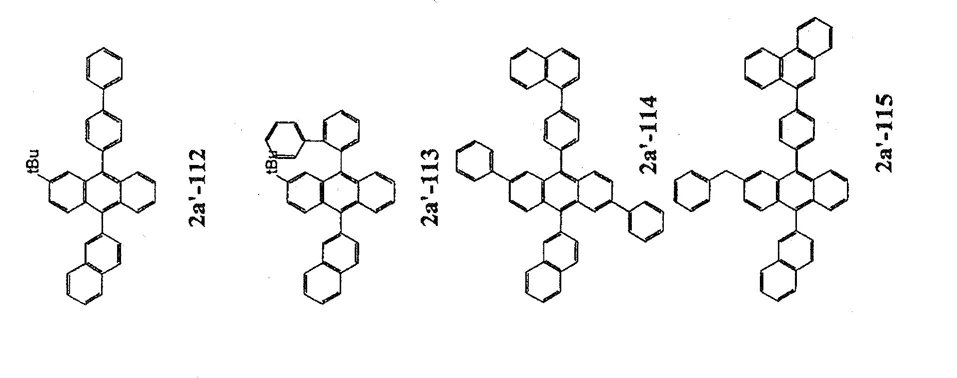

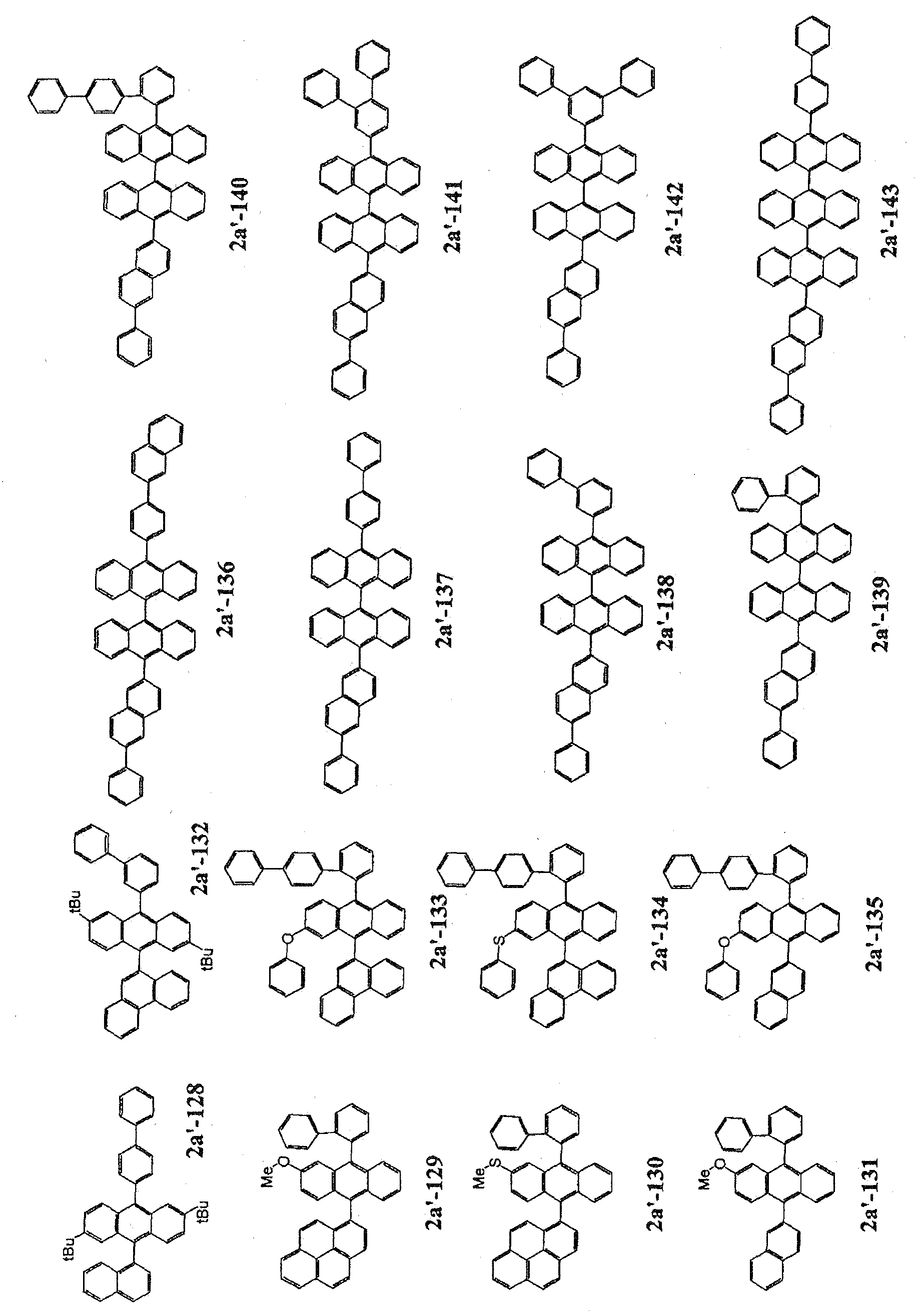

It is preferable that the electron transporting layer of the device of the invention contain a compound represented by the following formula (1).

(A)u-(B)v (1)

In the formula (1), A is an aromatic hydrocarbon group having 3 or more carbon rings, B is a heterocyclic group which may be substituted, and u and v are each an integer of 1 to 6.

The aromatic hydrocarbon group having 3 or more carbon rings, which is the group A, includes anthracene, phenanthrene, naphthacene, pyrene, chrysene, benzanthracene, pentacene, dibenzanthracene, benzopyrene, fluorene, benzofluorene, fluoranthene, benzofluoranthene, naphthofluoranthene, dibenzofluorene, dibenzopyrene and dibenzofluoranthene.

The substituted or unsubstituted heterocyclic group, which is the group B, includes pyridine, pyrimidine, pyrazine, pyridazine, triazine, quinoline, quinoxaline, acridine, imidazopyridine, imidazopyrimidine and phenenthroline.

It is preferred that the compound represented by the formula (1) be a compound having in its molecule one or more skeletons selected from anthracene, phenanthrene, naphthacene, pyrene, chrysene, benzanthracene, pentacene, dibenzanthracene, benzopyrene, fluorene, benzofluorene, fluoranthene, benzofluoranthene, naphthofluoranthene, dibenzofluorene, dibenzopyrene and dibenzofluoranthene.

The compound represented by the formula (1) is preferably a nitrogen-containing heterocyclic compound.
It is preferred that the nitrogen-containing heterocyclic compound be a compound having in its molecule one or more skeletons selected from pyridine, pyrimidine, pyrazine, pyridazine, triazine, quinoline, quinoxaline, acridine, imidazopyridine, imidazopyrimidine and phenenthroline.

The nitrogen-containing heterocyclic compound is more preferably a benzimidazole derivative represented by the following formula (7) or (8): 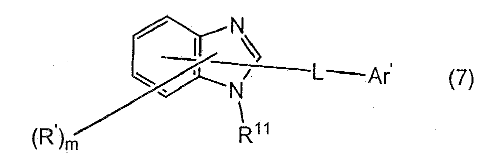 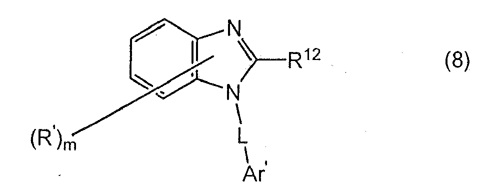 wherein R' is a hydrogen atom, a substituted or unsubstituted aryl group having 6 to 60 carbon atoms, a substituted or unsubstituted pyridyl group, a substituted or unsubstituted quinolyl group, a substituted or unsubstituted alkyl group having 1 to 20 carbon atoms, or a substituted or unsubstituted alkoxy group having 1 to 20 carbon atoms;
m is an integer of 0 to 4;
R¹¹ is a substituted or unsubstituted aryl group having 6 to 60 carbon atoms, a substituted or unsubstituted pyridyl group, a substituted or unsubstituted quinolyl group, a substituted or unsubstituted alkyl group having 1 to 20 carbon atoms, or an alkoxy group having 1 to 20 carbon atoms;
R¹² is a hydrogen atom, a substituted or unsubstituted aryl group having 6 to 60 carbon atoms, a substituted or unsubstituted pyridyl group, a substituted or unsubstituted quinolyl group, a substituted or unsubstituted alkyl group having 1 to 20 carbon atoms, or a substituted or unsubstituted alkoxy group having 1 to 20 carbon atoms;
L is a substituted or unsubstituted arylene group having 6 to 60 carbon atoms, a substituted or unsubstituted pyridinylene group, a substituted or unsubstituted quinolinylene group, or a substituted or unsubstituted fluorenylene group; and
Ar' is a substituted or unsubstituted aryl group having 6 to 60 carbon atoms, a substituted or unsubstituted pyridinyl group, or a substituted or unsubstituted quinolinyl group.
At least one of L and Ar' is a group containing an aromatic hydrocarbon group having 3 or more carbon rings.

In the formulas (7) and (8), R' is a hydrogen atom, substituted or unsubstituted aryl group having 6 to 60 carbon atoms, substituted or unsubstituted pyridyl group, substituted or unsubstituted quinolyl group, substituted or unsubstituted alkyl group having 1 to 50 carbon atoms or a substituted or unsubstituted alkoxy group having 1 to 50 carbon atoms.

The substituted or unsubstituted aryl group having 6 to 60 carbon atoms is preferably a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenyl-yl group, 4"-t-butyl-p-terphenyl-4-yl group, fluoranthenyl group, or fluorenyl group, more preferably a phenyl group, naphthyl group, biphenyl group, anthracenyl group, phenanthryl group, pyrenyl group, chrysenyl group, fluoranthenyl group, or fluorenyl group.

Preferable substituted or unsubstituted alkyl groups having 1 to 50 carbon atoms include a methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxyisopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group, 1,2,3-trinitropropyl group, cyclopropyl group, cyclobutyl group, cyclopentyl group, cyclohexyl group, 4-methylcyclohexyl group, 1-adamantyl group, 2-adamantyl group, 1-norbornyl group, and 2-norbornyl group.

The substituted or unsubstituted alkoxy group having 1 to 50 carbon atoms is represented by -OY. Examples of Y are the same as those of the alkyl group described above.

The substituents of the aryl group, pyridyl group, quinolyl group, alkyl group and an alkoxy group include a substituted or unsubstituted aryl group having 6 to 50 ring carbon atoms, substituted or unsubstituted aromatic heterocyclic group having 5 to 50 ring atoms, substituted or unsubstituted alkyl group having 1 to 50 carbon atoms, substituted or unsubstituted alkoxy group having 1 to 50 carbon atoms, substituted or unsubstituted aralkyl group having 1 to 50 carbon atoms, a substituted or unsubstituted aryloxy group having 5 to 50 ring atoms, substituted or unsubstituted arylthio group having 5 to 50 ring atoms, substituted or unsubstituted carboxyl group having 1 to 50 carbon atoms, halogen group, cyano group, nitro group, and hydroxyl group.

m is an integer of 0 to 4, preferably 0 to 3, and more preferably 0 to 2.

R¹¹ is a substituted or unsubstituted aryl group having 6 to 60 carbon atoms, substituted or unsubstituted pyridyl group, substituted or unsubstituted quinolyl group, substituted or unsubstituted alkyl group having 1 to 20 carbon atoms or a substituted or unsubstituted alkoxy group having 1 to 20 carbon atoms. Examples of each group and the substituent are the same as those of R'.

R¹² is a hydrogen atom, substituted or unsubstituted aryl group having 6 to 60 carbon atoms, substituted or unsubstituted pyridyl group, substituted or unsubstituted quinolyl group, substituted or unsubstituted alkyl group having 1 to 20 carbon atoms or a substituted or unsubstituted alkoxy group having 1 to 20 carbon atoms. Examples of each group and the substituent are the same as those of R'.

L is a substituted or unsubstituted arylene group having 6 to 60 carbon atoms, substituted or unsubstituted pyridinylene group, substituted or unsubstituted quinolinylene group or a substituted or unsubstituted fluorenylene group.

Preferred examples of the arylene group having 6 to 60 carbon atoms include a divalent substituent obtained by removing one hydrogen atom from the substituent described as examples of the aryl group having carbon atoms 6 to 60. More preferred examples of the arylene group having 6 to 60 carbon atoms are a phenylene group, naphthylene group, biphenylene group, anthracenylene group, phenanthrylene group, pyrenylene group, chrysenylene group, fluoranthenylene group, and fluorenylene group.

Examples of the substituent of the arylene group, pyridinylene group, quinolinylene group or fluorenylene group are the same as those of R'.

Ar' is a substituted or unsubstituted aryl group having 6 to 60 (preferably 6 to 30) carbon atoms, substituted or unsubstituted pyridinyl group or substituted or unsubstituted quinolyl group.
Examples of the aryl group having 6 to 60 carbon atoms, and the substituents of the aryl group, pyridinyl group and quinolyl group are the same as those of R'.

For the benzimidzole derivative of formula (7), preferably, m is O, R¹¹ is an aryl group, L is an arylene group having 6 to 30 carbon atoms (more preferably 6 to 20 carbon atoms) and Ar' is an aryl group having 6 to 30 carbon atoms.

In the benzimidazol derivative represented by the formula (8), it is preferable that m be 0, R¹² be an aryl group, L be an arylene group having 6 to 30 (more preferably 6 to 20) carbon atoms, and Ar' be an aryl group having 6 to 30 carbon atoms.

It is preferred that a chalcogenide layer, a metal halide layer or a metal oxide layer be provided on a surface of at least one of the cathode and the anode.
Such a layer can effectively suppress pixel defects caused by current leakage and short circuit.

Examples of the chalcogenide layer include Li₂O, LiO, Na₂S, Na₂Se, NaO, CaO, BaO, SrO, BeO, BaS and CaSe.
Examples of the metal halide layer include fluorides such as LiF, NaF, KF, LiCl, KCI, NaCl, CaF₂, BaF₂, SrF₂, MgF₂ and BeF₂, and halides other than fluorides.
Examples of the metal oxide layer include oxides containing one or two or more elements selected from Ba, Ca, Sr, Yb, Al, Ga, In, Li, Na, Cd, Cs, Mg, Ti, Si, Ge, Mo, Ru, Ta, Sb and Zn.

For the organic EL device of the invention, organic layers such as an emitting layer can be formed by dry film-formation methods such as vacuum deposition, molecular beam deposition (MBE), sputtering, plasma and ion plating, and applying methods such as spin coating, dipping, casting, bar coating, roll coating, flow coating and ink jetting using a solution wherein a raw material is dissolved in a solvent.

The thickness of each organic compound layer is not particularly limited but required to be appropriate. Generally, if the thickness is too thin, for example, pinholes may occur, so that sufficient luminance may not be obtained when an electric voltage is applied. If the thickness is too thick, a high electric voltage is required to obtain a certain light output, reducing fluoranthene. Thus, the thickness is generally 5 nm to 10 µm, preferably 10 nm to 0.2 µm.

In particular, if an emitting layer is formed by using a fluoranthene compound, the emitting layer can be formed by a wet process as well as deposition.

In a wet film forming method, a solution containing the above fluoranthene compound as a material for forming an emitting layer and a solvent can be used.
This solution preferably contains at least one of fluoranthene compounds represented by the formulas (2) to (6) and at least one selected from the anthracene compounds represented by the formula (9). Preferably, the fluoranthene compound is a dopant material, and the anthracene compound is a host material.

Preferable concentration of the fluoranthene compound is 0.01 to 20 wt%.

In this case, an organic El material for forming an emitting layer is dissolved or dispersed in an appropriate solvent to prepare a solution containing the organic EL material. A thin film is formed by using the solution. Any solvent may be used. The solvent includes, for example, halogen hydrocarbon solvents such as dichloromethane, dichloroethane, chloroform, carbon tetrachloride, tetrachloroethane, trichloroethane, chlorobenzene, dichlorobenzene, chlorotoluene and trifluorotoluene, ether solvents such as dibutyl ether, tetrahydrofuran, tetrahydropyrane, dioxane, anisole and dimethoxyethane, alcohol solvents such as methanol, ethanol, propanol, isopropanol, butanol, pentanol, hexanol, cyclohexanol, methylcellosolve, ethylcellosolve and ethyleneglycol, ketone solvents such as acetone, methylethylketone, diethylketone, 2-hexanone, methylisobutylketone, 2-heptanone, 4-heptanone, diisobutylketone, acetonylacetone, isophorone, cyclohexanone, methylhexanone and acetophenone, hydrocarbon solvents such as benzene, toluene, xylene, ethylbenzene, hexane, cyclohexane, octane, decane and tetralin, ester solvents such as ethyl acetate, butyl acetate and amyl acetate, chain carbonate solvents such as dimethylcarbonate, metylethyl carbonate and diethylcarbonate, and cyclic carbonate solvents such as ethylene carbonate and propylene carbonate. Of these, the hydrocarbon solvents and the ether solvents such as toluene and dioxane are preferable. These solvents can be used either singly or in combination of two or more. The usable solvents are not limited thereto.

In each organic compound layer, appropriate resins and additives may be used to improve the film-forming properties or prevent pinhole formation therein. Usable resins include insulating resins such as polystyrene, polycarbonate, polyarylate, polyester, polyamide, polyurethane, polysulfone, polymethylmethacrylate, polymethylacrylate and cellulose and copolymers thereof, photoconductive resins such as poly-N-vinylcarbazole and polysilane and conductive resins such as polyaniline, polythiophene and polypyrrole. Additives include an antioxidant, ultraviolet absorbing agent and plasticizing agent.

In order to improve the stability of the organic EL device of the invention for temperature, humidity, atmosphere and the like, the surface of the device may be provided with a protection layer or the whole device may be protected with a silicone oil, resin and the like.

### [Structure of organic EL device]

The typical examples of the structure of the organic EL device according to the invention are shown below. The invention is not limited to these.
(1) Anode/emitting layer/hole-transporting layer/cathode
(2) Anode/hole-transporting layer/emitting layer/electron-transporting layer/cathode
(3) Anode/hole-injecting layer/hole-transporting layer/emitting layer/electron-transporting layer/cathode
(4) Anode/hole-transporting layer/emitting layer/electron-transporting layer/electron-injecting layer/cathode
(5) Anode/hole-injecting layer/hole-transporting layer/emitting layer/electron-transporting layer/electron-injecting layer/cathode
(6) Anode/insulating layer/hole-transporting layer/emitting layer/electron-transporting layer/cathode
(7) Anode/hole-transporting layer/emitting layer/electron-transporting layer/insulating layer/cathode
(8) Anode/insulating layer/hole-transporting layer/emitting layer/electron-transporting layer/insulating layer/cathode
(9) Anode/hole-injecting layer/hole-transporting layer/emitting layer/electron-transporting layer/insulating layer/cathode
(10) Anode/insulating layer/hole-injecting layer/hole-transporting layer/emitting layer/electron-transporting layer/electron-injecting layer/cathode
(11) Anode/insulating layer/hole-injecting layer/hole-transporting layer/emitting layer/electron-transporting layer/electron-injecting layer/insulating layer/cathode
Of these, generally, the structures of (2), (3), (4), (5), (8), (9) and (11) are preferably used.

The functions and the like of each layer in the organic EL device will be explained below.

### [Substrate]

If an organic EL device is of under surface emission type or bottom emission type where light is outcoupled through a substrate, the organic EL device of the invention is preferably formed on a transparent substrate. The transparent substrate is preferably a flat and smooth substrate having a transmittance of 50% or more to light rays within visible ranges of 400 to 700 nm.
Specific examples thereof include glass plates and polymer plates. Examples of the glass plate include soda-lime glass, barium/strontium-containing glass, lead glass, aluminosilicate glass, borosilicate glass, barium borosilicate glass, and quartz. Examples of the polymer plate include polycarbonate, acrylic polymer, polyethylene terephthalate, polyethersulfide, and polysulfone. The substrate may be a TFT substrate in which TFT for driving is formed.

When the device is of an upper emitting type or top emission type in which light is emitted from the top of the device, it is not necessary for the substrate to be transparent. In this case, it is preferable that an appropriate light reflecting metal such as aluminum be formed on the substrate.

### [Anode]

The anode of the organic EL device plays a role for injecting holes into its hole-injecting/transporting layer or emitting layer. The anode effectively has a work function of 4.5 eV or more.
Specific examples of anode materials used in the invention include indium tin oxide alloy (ITO), tin oxide (NESA), indium zinc oxide alloy (IZO), gold, silver, platinum, and copper.
Although these materials may be used individually, alloys thereof or materials wherein another element is added to the materials can be selected for use.
The anode can be formed by forming these electrode materials into a thin film by vapor deposition, sputtering or the like.

In the case of under surface emission type or bottom emission type organic EL device, it is preferred that the anode preferably have a transmittance of emitted light of more than 10%. The sheet resistance of the anode is preferably several hundred Ω/□ or less. The film thickness of the anode, which varies depending upon the material thereof, is usually from 10 nm to 1 µm, preferably from 10 to 200 nm.

### [Emitting layer]

The emitting layer of the organic EL device has the following functions (1), (2) and (3) in combination.
(1) Injection function: function of allowing injection of holes from the anode or hole-injecting/transporting layer and injection of electrons from the cathode or electron-injecting/transporting layer upon application of an electric field
(2) Transporting function: function of moving injected carriers (electrons and holes) due to the force of an electric field
(3) Emitting function: function of allowing electrons and holes to recombine therein to emit light
   Note that electrons and holes may be injected into the emitting layer with different degrees, or the transportation capabilities indicated by the mobility of holes and electrons may differ. It is preferable that the emitting layer move either electrons or holes.

As the method of forming the emitting layer, a known method such as deposition, spin coating, or an LB method may be applied. It is preferable that the emitting layer be a molecular deposition film.
The molecular deposition film is usually a thin film formed by deposition of a gaseous material compound or solidification of a solution or liquid material compound. The molecular deposition film is generally distinguished from a thin film (molecular accumulation film) formed using the LB method by the difference in aggregation structure or higher order structure, or the difference in function due to the difference in structure.
The emitting layer may also be formed by dissolving a binder such as a resin and a material compound in a solvent to obtain a solution, and forming a thin film from the solution by spin coating or the like, as disclosed in JP-A-57-51781.

### [Hole-transporting layer and hole-injecting layer]

The hole-transporting layer is a layer for helping the injection of holes into the emitting layer so as to transport holes to an emitting region. The hole mobility thereof is large and the ionization energy thereof is usually as small as 5.5 eV or less. Such a hole-transporting layer is preferably made of a material which can transport holes to the emitting layer at a low electric field intensity, and more preferably have a hole mobility of at least 10⁻⁴ cm²/V · second, for example, upon application of an electric field of 10⁴ to 10⁶ V/cm.
Any materials which have the above preferable properties can be used as the material for forming the hole-transporting layer without particular limitation. The material can be arbitrarily selected from materials which have been widely used as a material transporting carriers of holes in photoconductive materials and known materials used in a hole-transporting layer of organic EL devices.

Specific examples include triazole derivatives (see USP No. 3,112,197 and others), oxadiazole derivatives (see USP No. 3,189,447 and others), imidazole derivatives (see JP-B-37-16096 and others), polyarylalkane derivatives (see USP Nos. 3,615,402, 3,820,989 and 3,542,544, JP-B-45-555 and 51-10983, JP-A-51-93224, 55-17105, 56-4148, 55-108667, 55-156953 and 56-36656, and others), pyrazoline derivatives and pyrazolone derivatives (see USP Nos. 3,180,729 and 4,278,746, JP-A-55-88064, 55-88065, 49-105537, 55-51086, 56-80051, 56-88141, 57-45545, 54-112637 and 55-74546, and others), phenylene diamine derivatives (see USP No. 3,615,404, JP-B-51-10105, 46-3712 and 47-25336, and 54-119925, and others), arylamine derivatives (see USP Nos. 3,567,450, 3,240,597, 3,658,520, 4,232,103, 4,175,961 and 4,012,376, JP-B-49-35702 and 39-27577, JP-A-55-144250, 56-119132 and 56-22437, DE1,110,518, and others), amino-substituted chalcone derivatives (see USP No. 3,526,501, and others), oxazole derivatives (ones disclosed in USP No. 3,257,203, and others), styrylanthracene derivatives (see JP-A-56-46234, and others), fluorenone derivatives (JP-A-54-110837, and others), hydrazone derivatives (see USP Nos. 3,717,462, JP-A-54-59143, 55-52063, 55-52064, 55-46760, 57-11350, 57-148749 and 2-311591, and others), stilbene derivatives (see JP-A-61-210363, 61-228451, 61-14642, 61-72255, 62-47646, 62-36674, 62-10652, 62-30255, 60-93455, 60-94462, 60-174749 and 60-175052, and others), silazane derivatives (USP No. 4,950,950), polysilanes (JP-A-2-204996), aniline copolymers (JP-A-2-282263), and electroconductive high molecular oligomers (in particular thiophene oligomers).

Preferably, materials represented by the following formula are used.

Q¹-G-Q²

Wherein, Q¹ and Q² are a part having at least one tertiary amine, and G is a linking group.

Further preferably, amine derivatives represented by the following formula are used. 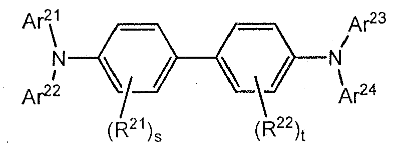 wherein Ar²¹ to Ar²⁴ are a substituted or unsubstituted aromatic ring having 6 to 50 ring carbon atoms or a substituted or unsubstituted heteroaromatic ring having 5 to 50 ring atoms.
R²¹ and R²² are a substituent, and s and t are each an integer of 0 to 4.
Ar²¹ and Ar²², or Ar²³ and Ar²⁴ may be bonded to each other to form a cyclic structure.
R²¹s or R²²s may be bonded to each other to form a cyclic structure.
Substituents of Ar²¹ to Ar²⁴, R²¹ and R²² are a substituted or unsubstituted aromatic ring having 6 to 50 ring carbon atoms, substituted or unsubstituted heteroaromatic ring having 5 to 50 ring atoms, alkyl group having 1 to 50 carbon atoms, alkoxy group having 1 to 50 carbon atoms, alkylaryl group having 1 to 50 carbon atoms, aralkyl group having 1 to 50 carbon atoms, styryl group, amino group substituted by an aromatic ring having 6 to 50 ring carbon atoms or a heteroaromatic ring having 5 to 50 ring atoms, aromatic ring having 6 to 50 ring carbon atoms substituted by an amino group substituted by an aromatic ring having 6 to 50 ring carbon atoms or a heteroaromatic ring having 5 to 50 ring atoms, or heteroaromatic ring having 5 to 50 ring atoms substituted by an amino group substituted by an aromatic ring having 6 to 50 ring carbon atoms or a heteroaromatic ring having 5 to 50 ring atoms.

A hole-injecting layer may further be formed to help injecting holes in addition to the hole-transporting layer. As the material for the hole-injecting layer, the same substances used for the hole-transporting layer can be used. The following can also be used: porphyrin compounds (disclosed in JP-A-63-295695 and others), aromatic tertiary amine compounds and styrylamine compounds (see USP No. 4,127,412, JP-A-53-27033, 54-58445, 55-79450, 55-144250, 56-119132, 61-295558, 61-98353 and 63-295695, and others). Aromatic tertiary amine compounds are particularly preferably used.

The following can also be given as examples: 4,4'-bis(N-(1-naphthyl)-N-phenylamino)biphenyl (abbreviated by NPD, hereinafter), which has in the molecule thereof two condensed aromatic rings, disclosed in USP No. 5,061,569, and 4,4',4"-tris(N-(3-methylphenyl)-N-phenylamino)triphenylamine (abbreviated by MTDATA, hereinafter) wherein three triphenylamine units are linked to each other in a star-burst form, disclosed in JP-A-4-308688.
Inorganic compounds such as p-type Si and p-type SiC as well as aromatic dimethylidene type compounds can also be used as the material of the hole-injecting layer.

The hole-injecting layer and hole-transporting layer can be formed from the above-mentioned compounds by a known method such as vacuum deposition, spin coating, casting or LB technique. The film thickness of the hole-injecting layer and hole-transporting layer is not particularly limited, and is usually from 5 nm to 5 µm. The hole-injecting layer or hole-transporting layer may be a single layer made of one, or two or more of the above-mentioned materials, or may be stacked hole-injecting layers or hole-transporting layers made of different compounds.

An organic semiconductor layer is one type of a hole- transporting layer for helping the injection of holes or electrons into an emitting layer, and is preferably a layer having an electric conductivity of 10⁻¹⁰ S/cm or more. As the material of such an organic semiconductor layer, electroconductive oligomers such as thiophene-containing oligomers or arylamine-containing oligomers disclosed in JP-A-8-193191, and electroconductive dendrimers such as arylamine-containing dendrimers may be used.

### [Electron-injecting/transporting layer (Electron-Transporting Zone)]

An electron-injecting/transporting layer may further be stacked on the cathode side of the organic luminescent medium layer. The electron-injecting/transporting layer is a layer for helping the injection of electrons into the emitting layer, and has a large electron mobility. The thickness of the electron-transporting layer is arbitrarily selected in the range of several nanometers to several micrometers. When the electron-transporting layer has a large thickness, it is preferable that the electron mobility be at least 10⁻⁵ cm²/Vs or more at an applied electric field of 10⁴ to 10⁶ V/cm in order to prevent an increase in voltage.

Nitrogen-containing heterocyclic derivatives of the following formulas 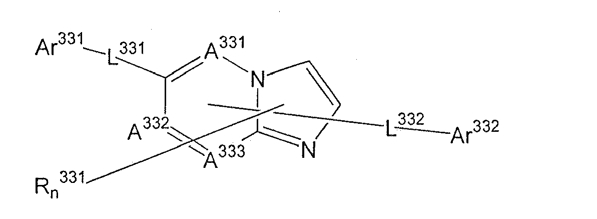 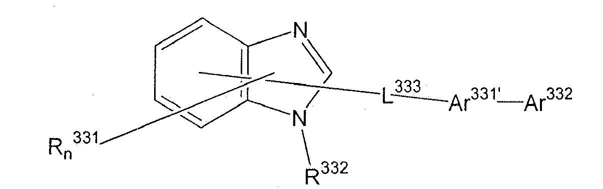 wherein A³³¹ to A³³³ are a nitrogen atom or a carbon atom;
R³³¹ and R³³² are a substituted or unsubstituted aryl group having 6 to 60 carbon atoms, a substituted or unsubstituted heteroaryl group having 3 to 60 carbon atoms, an alkyl group having 1 to 20 carbon atoms, a haloalkyl group having 1 to 20 carbon atoms, or an alkoxy group having 1 to 20 carbon atoms, and n is an integer of 0 to 5, provided that, when n is an integer of 2 or more, plural R³³¹s may be the same or different;
adjacent R³³¹s may be bonded to form a substituted or unsubstituted carbocyclic aliphatic ring or a substituted or unsubstituted carbocyclic aromatic ring;
Ar³³¹ is a substituted or unsubstituted aryl group having 6 to 60 carbon atoms or a substituted or unsubstituted heteroaryl group having 3 to 60 carbon atoms;
Ar^{331'} is a substituted or unsubstituted arylene group having 6 to 60 carbon atoms or a substituted or unsubstituted heteroarylene group having 3 to 60 carbon atoms;
Ar³³² is a hydrogen atom, an alkyl group having 1 to 20 carbon atoms, a haloalkyl group having 1 to 20 carbon atoms, an alkoxy group having 1 to 20 carbon atoms, a substituted or unsubstituted aryl group having 6 to 60 carbon atoms, or a substituted or unsubstituted heteroaryl group having 3 to 60 carbon atoms;
provided that one of Ar³³¹ and Ar³³² is a substituted or unsubstituted condensed ring group having 10 to 60 carbon atoms or a substituted or unsubstituted heterocondensed ring group having 3 to 60 carbon atoms; and
L³³¹, L³³², and L³³³ are independently a single bond, a substituted or unsubstituted condensed ring having 6 to 60 carbon atoms, a substituted or unsubstituted heterocondensed group having 3 to 60 carbon atoms or a substituted or unsubstituted fluorenylene group.

Nitrogen-containing heterocyclic derivatives of the following formula disclosed in Japanese Patent Application No. 2003-004193:

HAr-L³⁴¹-Ar³⁴¹-Ar³⁴²

wherein HAr is a substituted or unsubstituted nitrogen-containing heterocyclic group having 3 to 40 carbon atoms,
L³⁴¹ is a single bond, a substituted or unsubstituted arylene group having 6 to 60 carbon atoms, a substituted or unsubstituted heteroarylene group having 3 to 60 carbon atoms or a substituted or unsubstituted fluorenylene group,
Ar³⁴¹ is a substituted or unsubstituted divalent aromatic hydrocarbon group having 6 to 60 carbon atoms, and
Ar³⁴² is a substituted or unsubstituted aryl group having 6 to 60 carbon atoms or a substituted or unsubstituted heteroaryl group having 3 to 60 carbon atoms.

Silacyclopentadiene derivatives of the following formula disclosed in JP-A-09-087616 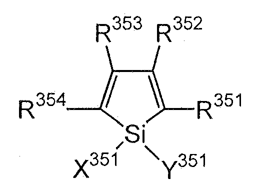 wherein X³⁵¹ and Y³⁵¹ are independently a saturated or unsaturated hydrocarbon group having 1 to 6 carbon atoms, alkoxy group, alkenyloxy group, alkynyloxy group, hydroxy group, substituted or unsubstituted aryl group, substituted or unsubstituted hetercycle, or saturated or unsaturated ring formed by the bonding of X³⁵¹ and Y³⁵¹, and R³⁵¹ to R³⁵⁴ are independently a hydrogen atom, halogen atom, substituted or unsubstituted alkyl group having 1 to 6 carbon atoms, alkoxy group, aryloxy group, perfluoroalkyl group, perfluoroalkoxy group, amino group, alkylcarbonyl group, arylcarbonyl group, alkoxycarbonyl group, aryloxycarbonyl group, azo group, alkylcarbonyloxy group, arylcarbonyloxy group, alkoxycarbonyloxy group, aryloxycarbonyloxy group, sulfinyl group, sulfonyl group, sulfanyl group, silyl group, carbamoyl group, aryl group, heterocyclic group, alkenyl group, alkynyl group, nitro group, formyl group, nitroso group, formyloxy group, isocyano group, cyanate group, isocyanate group, thiocyanate group, isothiocyanate group, cyano group, or a structure where an adjacent substituted or unsubstituted ring is condensed.

Silacyclopentadiene derivatives of the following formula disclosed in JP-A-09-194487 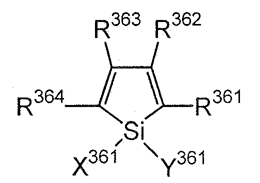 wherein X³⁶¹ and Y³⁶¹ are independently a saturated or unsaturated hydrocarbon group having 1 to 6 carbon atoms, alkoxy group, alkenyloxy group, alkynyloxy group, substituted or unsubstituted aryl group, substituted or unsubstituted heterocycle, or saturated or unsaturated ring formed by the bonding of X³⁶¹ and Y³⁶¹, and R³⁶¹ to R³⁶⁴ are independently a hydrogen atom, halogen atom, substituted or unsubstituted alkyl group having 1 to 6 carbon atoms, alkoxy group, aryloxy group, perfluoroalkyl group, perfluoroalkoxy group, amino group, alkylcarbonyl group, arylcarbonyl group, alkoxycarbonyl group, aryloxycarbonyl group, azo group, alkylcarbonyloxy group, arylcarbonyloxy group, alkoxycarbonyloxy group, aryloxycarbonyloxy group, sulfinyl group, sulfonyl group, sulfanyl group, silyl group, carbamoyl group, aryl group, heterocyclic group, alkenyl group, alkynyl group, nitro group, formyl group, nitroso group, formyloxy group, isocyano group, cyanate group, isocyanate group, thiocyanate group, isothiocyanate group, cyano group, or a structure where an adjacent substituted or unsubstituted ring is condensed.
However, when R³⁶¹ and R³⁶⁴ are a phenyl group, X³⁶¹ and Y³⁶¹ are not an alkyl group and phenyl group. When R³⁶¹ and R³⁶⁴ are a thienyl group, X³⁶¹ and Y³⁶¹ are not a monovelent group and R³⁶² and R³⁶³ are not an alkyl group, aryl group, alkenyl group or aliphatic group formed by bonding of R³⁶² and R³⁶³. When R³⁶¹ and R³⁶⁴ are a silyl group, R³⁶², R³⁶³, X³⁶¹ and Y³⁶¹ are not independently a monovalent hydrocarbon group having 1 to 6 carbon atoms or hydrogen atom. When R³⁶¹ and R³⁶² form a condensed benzene ring, X³⁶¹ and Y³⁶¹ are not an alkyl group or phenyl group.

Borane derivatives of the following formula disclosed in JP-T-2000-040586 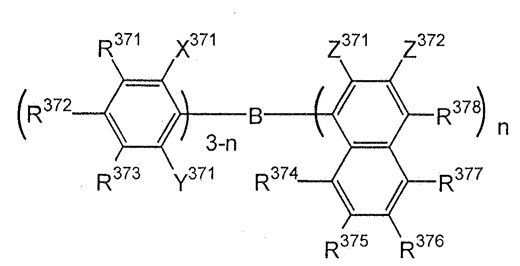 wherein R³⁷¹ to R³⁷⁸ and Z³⁷² are independently a hydrogen atom, a saturated or unsaturated hydrocarbon group, an aromatic group, a heterocyclic group, a substituted amino group, a substituted boryl group, an alkoxy group, or an aryloxy group; X³⁷¹, Y³⁷¹ and Z³⁷¹ are independently a saturated or unsaturated hydrocarbon group, an aromatic group, a heterocyclic group, a substituted amino group, an alkoxy group, or an aryloxy group; the substituents for Z³⁷¹ and Z³⁷² may be bonded to form a condensed ring; and n is an integer of 1 to 3, provided that Z³⁷¹s may differ when n is 2 or more, and a case in which n is 1, X³⁷¹, Y³⁷¹ and R³⁷² are methyl groups, and R³⁷⁸ is a hydrogen atom or a substituted boryl group, and a case in which n is 3 and Z³⁷¹ is a methyl group are excluded.

Compounds of the following formula disclosed in JP-A-10-088121 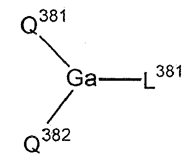 wherein Q³⁸¹ and Q³⁸² are independently a ligand of the following formula, L³⁸¹ is a halogen atom, a substituted or unsubstituted alkyl group, a substituted or unsubstituted cycloalkyl group, a substituted or unsubstituted aryl group, a substituted or unsubstituted heterocyclic group, -OR³⁹¹ (R³⁹¹ is a hydrogen atom, a substituted or unsubstituted alkyl group, a substituted or unsubstituted cycloalkyl group, a substituted or unsubstituted aryl group, or a substituted or unsubstituted heterocyclic group), or -O-Ga-O³⁹¹(Q³⁹²) (Q³⁹¹ and Q³⁹² have the same meanings as Q³⁸¹ and Q³⁸²).

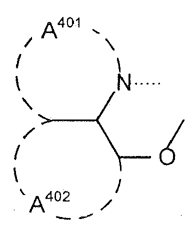

wherein the ring A⁴⁰¹ and the ring A⁴⁰² are substituted or unsubstituted aryl ring structures or heterocyclic structures which are bonded to each other.

Specific examples of the substituents for the rings A⁴⁰¹ and A⁴⁰² forming the ligands of the above formula include halogen atoms such as chlorine, bromine, iodine, and fluorine, substituted or unsubstituted alkyl groups such as a methyl group, ethyl group, propyl group, butyl group, sec-butyl group, tert-butyl group, pentyl group, hexyl group, heptyl group, octyl group, stearyl group, and trichloromethyl group, substituted or unsubstituted aryl groups such as a phenyl group, naphthyl group, 3-methylphenyl group, 3-methoxyphenyl group, 3-fluorophenyl group, 3-trichloromethylphenyl group, 3-trifluoromethylphenyl group, and 3-nitrophenyl group, substituted or unsubstituted alkoxy groups such as a methoxy group, n-butoxy group, tert-butoxy group, trichloromethoxy group, trifluoroethoxy group, pentafluoropropoxy group, 2,2,3,3-tetrafluoropropoxy group, 1,1,1,3,3,3-hexafluoro-2-propoxy group, and 6-(perfluoroethyl)hexyloxy group, substituted or unsubstituted aryloxy groups such as a phenoxy group p-nitrophenoxy group, p-tert-butylphenoxy group, 3-fluorophenoxy group, pentafluorophenyl group, and 3-trifluoromethylphenoxy group, substituted or unsubstituted alkylthio groups such as a methylthio group, ethylthio group, tert-butylthio group, hexylthio group, octylthio group, and trifluoromethylthio group, substituted or unsubstituted arylthio groups such as a phenylthio group, p-nitrophenylthio group, p-tert-butylphenylthio group, 3-fluorophenylthio group, pentafluorophenylthio group, and 3-trifluoromethylphenylthio group, cyano group, nitro group, amino group, mono- or di-substituted amino groups such as a methylamino group, diethylamino group, ethylamino group, diethylamino group, dipropylamino group, dibutylamino group, and diphenylamino group, acylamino groups such as a bis(acetoxymethyl)amino group, bis(acetoxyethyl)amino group, bis(acetoxypropyl)amino group, and bis(acetoxybutyl)amino group, hydroxyl group, siloxy group, acyl group, substituted or unsubstituted carbamoyl groups such as a carbamoyl group, methylcarbamoyl group, dimethylcarbamoyl group, ethylcarbamoyl group, diethylcarbamoyl group, propylcarbamoyl group, butylcarbamoyl group, and phenylcarbamoyl group, carboxylic acid group, sulfonic acid group, imide group, cycloalkyl groups such as a cyclopentane group and cyclohexyl group, aryl groups such as a phenyl group, naphthyl group, biphenyl group, anthranyl group, phenanthryl group, fluorenyl group, and pyrenyl group, heterocyclic groups such as a pyridinyl group, pyrazinyl group, pyrimidinyl group, pyridazinyl group, triazinyl group, indolinyl group, quinolinyl group, acridinyl group, pyrrolidinyl group, dioxanyl group, piperidinyl group, morpholidinyl group, piperazinyl group, carbazolyl group, furanyl group, thiophenyl group, oxazolyl group, oxadiazolyl group, benzoxazolyl group, thiazolyl group, thiadiazolyl group, benzothiazolyl group, triazolyl group, imidazolyl group, and benzimidazolyl group, and the like. The above substituents may be bonded to form a six-membered aryl ring or heterocyclic ring.

A preferred embodiment of the invention is a device containing a reducing dopant in an electron-transferring region or in an interfacial region between a cathode and an organic layer. The reducing dopant is defined as a substance which can reduce an electron-transporting compound. Various substances which have given reducing properties can be used. For example, at least one substance can be preferably used which is selected from the group consisting of alkali metals, alkaline earth metals, rare earth metals, alkali metal oxides, alkali metal halides, alkaline earth metal oxides, alkaline earth metal halides, rare earth metal oxides, rare earth metal halides, alkali metal organic complexes, alkaline earth metal organic complexes, and rare earth metal organic complexes.

More specific examples of the preferred reducing dopants include at least one alkali metal selected from the group consisting of Na (work function: 2.36 eV), K (work function: 2.28 eV), Rb (work function: 2.16 eV) and Cs (work function: 1.95 eV), and at least one alkaline earth metal selected from the group consisting of Ca (work function: 2.9 eV), Sr (work function: 2.0 to 2.5 eV), and Ba (work function: 2.52 eV). Metals having a work function of 2.9 eV or less are particularly preferred. Among these, a more preferable reducing dopant is at least one alkali metal selected from the group consisting of K, Rb and Cs. Even more preferable is Rb or Cs. Most preferable is Cs. These alkali metals are particularly high in reducing ability. Thus, the addition of a relatively small amount thereof to an electron-injecting zone improves the luminance of the organic EL device and make the lifetime thereof long. As a reducing agent having a work function of 2.9 eV or less, combinations of two or more alkali metals are preferable, particularly combinations including Cs, such as Cs and Na, Cs and K, Cs and Rb, or Cs, Na and K. The combination containing Cs makes it possible to exhibit the reducing ability efficiently. The luminance of the organic EL device can be improved and the lifetime thereof can be made long by the addition thereof to its electron-injecting zone.

In the invention, an electron-injecting layer made of an insulator or a semiconductor may further be provided between a cathode and an organic layer. By forming the electron-injecting layer, a current leakage can be effectively prevented and electron-injecting properties can be improved. As the insulator, at least one metal compound selected from the group consisting of alkali metal calcogenides, alkaline earth metal calcogenides, halides of alkali metals and halides of alkaline earth metals can be preferably used. When the electron-injecting layer is formed of the alkali metal calcogenide or the like, the injection of electrons can be preferably further improved. Specifically preferable alkali metal calcogenides include Li₂O, LiO, Na₂S, Na₂Se and NaO and preferable alkaline earth metal calcogenides include CaO, BaO, SrO, BeO, BaS and CaSe. Preferable halides of alkali metals include LiF, NaF, KF, LiCl, KCI and NaCl. Preferable halides of alkaline earth metals include fluorides such as CaF₂, BaF₂, SrF₂, MgF₂ and BeF₂ and halides other than fluorides.

Semiconductors forming an electron-injecting layer include one or combinations of two or more of oxides, nitrides, and oxidized nitrides containing at least one element of Ba, Ca, Sr, Yb, Al, Ga, In, Li, Na, Cd, Mg, Si, Ta, Sb and Zn. An inorganic compound forming an electron-injecting layer is preferably a microcrystalline or amorphous insulating thin film. When the electron-injecting layer is formed of the insulating thin films, more uniformed thin film is formed whereby pixel defects such as a dark spot are decreased. Examples of such an inorganic compound include the above-mentioned alkali metal calcogenides, alkaline earth metal calcogenides, halides of alkali metals, and halides of alkaline earth metals.

### [Cathode]

In order to inject electrons into the electron injecting/transporting layer or emitting layer, electrode materials for cathode material include metals, alloys, electric conductive compounds and mixtures thereof with a low work function (4 eV or less). Specific examples thereof include sodium, sodium-potassium alloy, magnesium, lithium, magnesium/silver alloy, aluminum/aluminum oxide, aluminum/lithium alloy, indium, and rare earth metals.

The cathode can be formed by making the electrode materials into a thin film by vapor deposition, sputtering or some other method.
In the case of upper surface emission type or bottom emission type organic EL device, the cathode preferably has a transmittance of emitted light of more than 10%.
The sheet resistance of the cathode is preferably several hundred Ω/□ or less, and the film thickness thereof is usually from 10 nm to 1 µm, preferably from 50 to 200 nm.

### [Insulating layer]

In the organic EL device, pixel defects due to leakage or a short circuit are easily generated since an electric field is applied to a super thin film. In order to prevent this, it is preferred to insert an insulative thin layer between the pair of electrodes.

Examples of the material used in the insulating layer include aluminum oxide, lithium fluoride, lithium oxide, cesium fluoride, cesium oxide, magnesium oxide, magnesium fluoride, calcium oxide, calcium fluoride, cesium fluoride, cesium carbonate, aluminum nitride, titanium oxide, silicon oxide, germanium oxide, silicon nitride, boron nitride, molybdenum oxide, ruthenium oxide, and vanadium oxide.
A mixture or laminate thereof may be used.

### EXAMPLES

The invention will be explained below in more detail with examples, but is not limited thereto.

### Example 1

A 120 nm thick transparent electrode (anode) composed of indium tin oxide was provided on a glass substrate with 25 mm by 75 mm by 0.7 mm. The glass substrate was cleaned by ultrasonic waves in isopropyl alcohol for five minutes and then by UV ozone for thirty minutes. This substrate was set in a vapor deposit apparatus.
On the substrate, a compound (A) was deposited in a thickness of 60 nm as a hole injecting layer and then N,N'-bis[4'-{N-(naphtyl-1-yl)-N-phenyl}aminobiphenyl-4-yl]-N-phenylamine was deposited in a thickness of 20 nm as a hole transporting layer. Thereafter, as an emitting layer, the anthracene derivative of 9-(2-naphtyl)-10-[4-(1-naphtyl)phenyl]anthracene and the fluoranthene compound represented by the following formula (2-22) were codeposited at a weight ratio of 40:3 in a thickness of 40 nm.
Next, a compound (B) was deposited as an electron transporting layer in a thickness of 20 nm thereon.

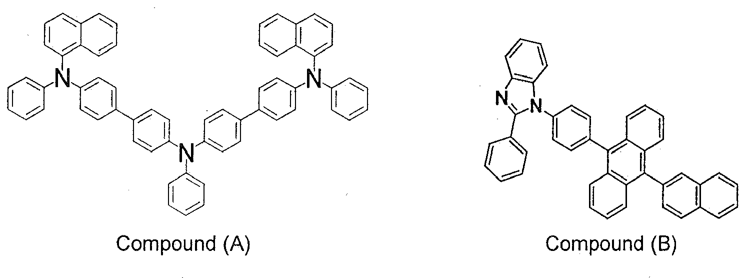

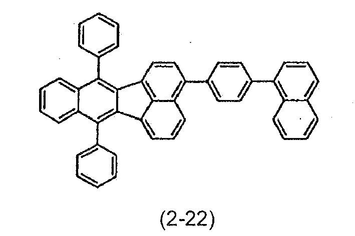

Lithium fluoride was deposited in a thickness of 0.5 nm, and aluminum was deposited in a thickness of 150 nm thereon. The aluminum/lithium fluoride served as a cathode. An organic EL device was thus prepared.

The device obtained was subjected to a current test. At a current density of 10 mA/cm², blue light emission with a driving voltage of 4.0V and a luminance of 793 cd/m² was obtained. The chromaticity coordinates were (0.150, 0.134), and the efficiency was 7.93 cd/A. In a continuous current test at an initial luminance of 5000 cd/m², the driving time was 840 hours until the luminance reached to 50% of the initial luminance.

### Example 2

A 120 nm thick transparent electrode (anode) composed of indium tin oxide was provided on a glass substrate with 25 mm by 75 mm by 0.7 mm. The glass substrate was cleaned by ultrasonic waves in isopropyl alcohol for five minutes and then by UV ozone for thirty minutes. This substrate was set in a vapor deposit apparatus.
On the substrate, a compound (A) was deposited in a thickness of 10 nm as a hole injecting layer and then N,N'-bis[4'-{N-(naphtyl-1-yl)-N-phenyl}aminobiphenyl-4-yl]-N-phenylamine was deposited in a thickness of 70 nm as a hole transporting layer. Thereafter, as an emitting layer, the anthracene derivative of 9-(2-naphtyl)-10-[4-(1-naphtyl)phehyl]anthracene and the fluoranthene compound represented by the following formula (2-29) were codeposited at a weight ratio of 20:1 in a thickness of 20 nm.
Next, a compound (C) was deposited as an electron transporting layer in a thickness of 35 nm thereon.

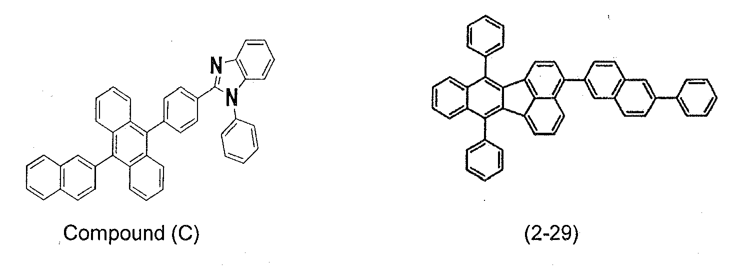

Lithium fluoride was deposited in a thickness of 0.5 nm, and aluminum was deposited in a thickness of 150 nm thereon. The aluminum/lithium fluoride served as a cathode. An organic EL device was thus prepared.

The device obtained was subjected to a current test. At a current density of 10 mA/cm², blue light emission with a driving voltage of 4.0V and a luminance of 710 cd/m² was obtained. The chromaticity coordinates were (0.150, 0.128), and the efficiency was 7.10 cd/A. In a continuous current test at an initial luminance of 5000 cd/m², the driving time was 860 hours until the luminance reached to 50% of the initial luminance.

### Comparative example 1

A 120 nm thick transparent electrode (anode) composed of indium tin oxide was provided on a glass substrate with 25 mm by 75 mm by 0.7 mm. The glass substrate was cleaned by ultrasonic waves in isopropyl alcohol for five minutes and then by UV ozone for thirty minutes. This substrate was set in a vapor deposit apparatus.
On the substrate, a compound (A) was deposited in a thickness of 60 nm as a hole injecting layer and then N,N'-bis[4'-{N-(naphtyl-1-yl)-N-phenyl}aminobiphenyl-4-yl]-N-phenylamine was deposited in a thickness of 20 nm as a hole transporting layer. Thereafter, as an emitting layer, the anthracene derivative of 9-(2-naphtyl)-10-[4-(1-naphtyl)phenyl]anthracene and the fluoranthene compound represented by the following formula (2-29) were codeposited at a weight ratio of 40:3 in a thickness of 40 nm.
Next, Alq was deposited as an electron transporting layer in a thickness of 20 nm thereon.
Lithium fluoride was deposited in a thickness of 0.5 nm, and aluminum was deposited in a thickness of 150 nm thereon. The aluminum/lithium fluoride served as a cathode. An organic EL device was thus prepared.

The device obtained was subjected to a current test. At a current density of 10 mA/cm², blue light emission with a driving voltage of 5.4V and a luminance of 332 cd/m² was obtained. The chromaticity coordinates were (0.153, 0.136), and the efficiency was 3.32 cd/A. In a continuous current test at an initial luminance of 5000 cd/m², the driving time was 170 hours until the luminance reached to 50% of the initial luminance.

### Example 3

An organic EL device was prepared in the same manner as Example 1 expect for using the following compound (3-1) instead of the compound (1-3).

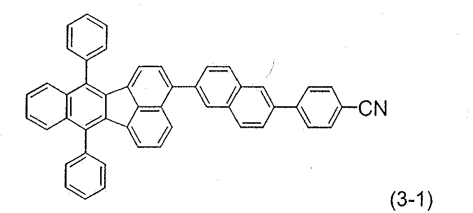

The device obtained was subjected to a current test. At a current density of 10mA/cm², blue light emission with a driving voltage of 4.0V and a luminance of 930 cd/m² was obtained. The chromaticity coordinates were (0.146, 0.132), and the efficiency was 9.3 cd/A. In a continuous current test at an initial luminance of 5000 cd/m², the driving time was 900 hours until the luminance reached to 50% of the initial luminance.

### Example 4

An organic EL device was prepared in the same manner as Example 1 expect for using the following compound (3-6) instead of the compound (1-3).

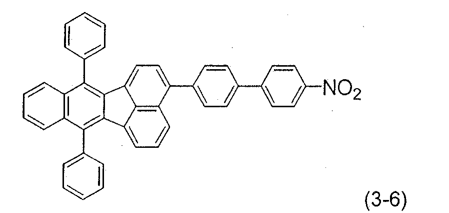

The device obtained was subjected to a current test. At a current density of 10 mA/cm², blue light emission with a driving voltage of 4.2V and a luminance of 798 cd/m² was obtained. The chromaticity coordinates were (0.146, 0.128), and the efficiency was 8.0 cd/A. In a continuous current test at an initial luminance of 5000 cd/m², the driving time was 810 hours until the luminance reached to 50% of the initial luminance.

### Example 5

An organic EL device was prepared in the same manner as Example 1 expect for using the following compound (3-12) instead of the compound (1-3).

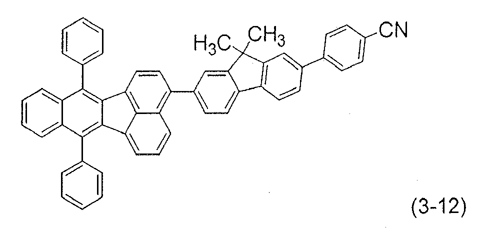

The device obtained was subjected to a current test. At a current density of 10 mA/cm², blue light emission with a driving voltage of 4.0V and a luminance of 850 cd/m² was obtained. The chromaticity coordinates were (0.146, 0.140), and the efficiency was 8.5 cd/A. In a continuous current test at an initial luminance of 5000 cd/m², the driving time was 950 hours until the luminance reached to 50% of the initial luminance.

### Example 6

An organic EL device was prepared in the same manner as Example 1 expect for using the following compound (3-32) instead of the compound (1-3).

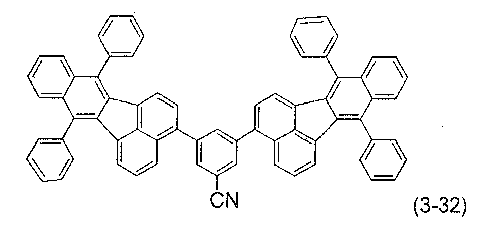

The device obtained was subjected to a current test. At a current density of 10 mA/cm², blue light emission with a driving voltage of 4.1V and a luminance of 780 cd/m² was obtained. The chromaticity coordinates were (0.146, 0.118), and the efficiency was 7.8 cd/A. In a continuous current test at an initial luminance of 5000 cd/m², the driving time was 795 hours until the luminance reached to 50% of the initial luminance.

Synthesis of the compounds used in Examples 3 to 6 are described below as a synthesis example of fluoranthene compounds.

### Synthesis example 1 [Synthesis of compound (3-1)]

### (1) Synthesis of 5-bromoacenaphthylene

29.2g (128.7 mmol) of 2,3-dichloro-5,6-dicyano-1,4-benzoquinone (DDQ) was added to 25.4g (107.3 mmol) of 5-bromoacenaphthene and 500 ml of dry benzene. The mixture was stirred for 6 hours while heated to reflux. 6.0g (26.4 mmol) of DDQ was added to the reactant mixture, and the resulting mixture was heated with stirring for 4 hours. After cooling, a precipitate was filtered and washed with chloroform. With the filtrate being added, the precipitate was washed with a 10% sodium hydroxide solution and water. After separation, an organic phase was dried using anhydrous sodium sulfate to distil the solvent away. The residue was dried under reduced pressure to obtain 13.0g (51.6% of yield) of 5-bromoacenaphtylene as a brown solid.

### (2) Synthesis of 3-bromo-7,12-dibenzo[k]fluoranthene

The mixture of 14.9g (55.2 mmol) of 1,3-diphenylisobenzofuran, 12.8g (55.2 mmol) of 5-bromo-acenaphtylene, and 50 ml of toluene was stirred for 16 hours while heated to reflux. After distilling the solvent away, 1200 ml of acetic acid was added thereto, and heated to 80°C. 150 ml of a 48 % HBr aqueous solution was added to the mixture, and stirred for 1 hour at 80°C. After cooling to room temperature, a precipitate was filtered and washed with methanol. The yellow solid obtained was recrystallized using 200 ml of toluene. The crystal was filtered to obtain 19.8 g (74% of yield) of 3-bromo-7,12-dibenzo[k]fluoranthene as a yellow solid.

### (3) Synthesis of 7,12-dibenzo[k]fluoranthene-3-yl-boronic acid

30.8g (64.0 mmol) of 3-bromo-7,12-dibenzo[k]fluoranthene was dissolved in 400 ml of dry THF and 300 ml of dry toluene. The solution was cooled to -70°C, and 44.6 ml (70.4 mmol) of n-butyllithium was dripped thereinto and stirred for 1 hour. 44.0 ml (192 mmol) of triisopropyl boronate was added thereto. The mixture was heated to room temperature for 2 hours. A precipitate was filtered, washed with toluene, dried under reduced pressure to obtain 25.14g (88% of yield) of 7,12-diphenylbenzo[k]fluoranthene-3-yl-boronic acid as a yellow solid.

### (4) Synthesis of 3-(6-bromonaphthalene-2-yl)-7,12-diphenylbenzo[k]fluoranthene

Under argon atmosphere, a 2M soldium carbonate aqueous solution (6.2 ml, 12.4 mmol) and 0.29g (0.25 mmol) of tetrakis(triphenylphosphino)palladium were added to the mixture of 2.3g (8.0 mmol) of 2,6-dibromonaphthalene, 4.3g (9.6 mmol) of 7,12-diphenylbenzo[k]fluoranthene-3-yl-boronic acid, 25 ml of toluene and 25 ml of dimethoxyethane. The mixture was stirred for 8 hours at 80°C. After cooling the mixture to room temperature, methanol was added thereto. A solid was filtered and washed with methanol. The solid obtained was recrystallized using dichloromethane and methanol to obtain 2.8g (58% of yield) of 3-(6-bromonaphthalene-2-yl)-7,12-diphenylbenzo[k]fluoranthene as a yellow solid.

### (5) Synthesis of 3-(6-(4-cyanophenyl)naphthalene-2-yl)-7,12-diphenylbenzo[k]fluoranthene (compound(3-1))

In (4) above, 3-(6-(4-cyanophenyl)naphthalene-2-yl)-7,12-diphenylbenzo[k]fluoranthene(compound 3-1) was obtained in the same manner as (4) except for using 3-(6-bromonaphthalene-2-yl)-7,12-diphenylbenzo[k]fluoranthene instead of 2,6-dibromonaphthalene, and 4-cyanophenylboronic acid instead of 7,12-diphenylbonzo[k]fluoranthene-3-yl-boronic acid. The FDMS, ultraviolet absorption maximum wavelength λmax in a toluene solution (molar absorption coefficient ε) and fluorescence emission maximum wavelength of the compound obtained are shown below.
FDMS, calcd for C₄₉H₂₉N=632, found m/z=632(M+)
UV(PhMe); λmax, 424(ε4.37), FL(PhMe, λex=420 nm); λmax, 444 nm

### Synthesis example 2 [synthesis of compound (3-6)]

### (1) Synthesis of 3-(4-bromophenyl)-7,12-diphenylbenzo[k]fluoranthene

3-(4-bromophenyl)-7,12-diphenylbenzo[k]fluoranthene was obtained in the same manner as Syntheses example 1 except for using 1-bromo-4-iodobenzen instead of 2,6-dibromonaphthalene in Synthesis example 1(4).

### (2) Synthesis of 3-(4-(4-nitrophenyl)phenyl)-7,12-diphenylbenzo[k]fluoranthene (compound(3-6))

3-(4-(4-nitrophenyl)phenyl)-7,12-diphenylbenzo[k]fluoranthene (compound(3-6)) was obtained in the same manner as Syntheses example 1 except for using 3-(4-bromophenyl)-7,12-diphenylbenzo[k]fluoranthene instead of 2,6-dibromonaphthalene, and 4-nitrophenyl-boronic acid instead of 7,12-diphenylbenzo[k]fluoranthene-3-ylboronic acid.
The FDMS of the compound obtained is shown below.
FDMS, calcd for C₄₄H₂₇NO₂=601, found m/z=601(M+)

### Synthesis example 3 [synthesis of compound (3-12)]

### (1) Synthesis of 3-(7-bromo-9,9-dimethylfluorene-2-yl)-7,12-diphenylbenzo[k]fluoranthene

3-(7-bromo-9,9-dimethylfluorene-2-yl)-7,12-diphenylbenzo[k]fluoranthene was obtained in the same manner as Syntheses example 1 except for using 2-bromo-7-iodo-9,9-dimethylfluorene instead of 2,6-dibromonaphthalene in synthesis example 1(4).

### (2) Synthesis of 3-(7-(4-cyanophenyl)9,9-dimethylfluorene-2-yl)-7,12-diphenylbenzo[k]fluoranthene (compound(3-12))

3-(7-(4-cyanophenyl)9,9-dimethylfluorene-2-yl)-7,12-diphenylbenzo[k]fluoranthene (compound(3-12)) was obtained in the same manner as Syntheses example 1 except for using 3-(7-bromo-9,9-dimethylfluorene-2-yl)-7,12-diphenylbenzo[k]fluoranthene instead of 2,6-dibromonaphthalene, and 4-cyanophenylboronic acid instead of 7,12-diphenylbenzo[k]fluoranthene-3-yl-boronic acid in synthesis example 1(4).
The FDMS of the compound obtained is shown below.
FDMS, calcd for C₅₄H₃₅N=697, found m/z=697(M+)

Synthesis example 4 [synthesis of compound (3-32)]
Compound (3-32) was obtained in the same manner as Syntheses example 1 except for using 1,3-dibromo-5-cyanoiodobenzen instead of 2,6-dibromonaphthalene in synthesis example 1(4).
The FDMS of the compound obtained is shown below.
FDMS, calcd for C₇₁H₄₁N=907, found m/z=907(M+)

### INDUSTRIAL APPLICABILITY

The organic EL device according to the invention can be applied to the area of various display devices, displays, backlights, illuminating sources, indicators, advertising displays, interiors and the like. The organic EL device is particularly suitable for display elements of color display.
The documents described in the specification are incorporated herein by reference in its entirety.

## Claims

1. An organic electroluminescent device comprising,
a cathode,
an anode, and
at least an emitting layer and an electron transporting layer between the cathode and the anode, the emitting layer comprising a host compound and a fluoranthene compound,
the electron transporting layer comprising a compound represented by the following formula (1),
(A)u-(B)v (1)
wherein A is a group containing an aromatic hydrocarbon group having 3 or more carbon rings, B is a heterocyclic group which may be substituted, and u and v are each an integer of 1 to 6.

2. The organic electroluminescent device according to claim 1, further comprising a hole injecting layer between the anode and the emitting layer.

3. The organic electroluminescent device according to claim 1 or 2, further comprising a hole transporting layer between the anode and the emitting layer.

4. The organic electroluminescent device according to any one of claims 1 to 3, wherein the fluoranthene compound is represented by the following formula (2),
FLₙ-G¹ (2)
wherein n is an integer of 2 to 4,
FL is a monovalent group having a fluoranthene structure, plural FLs may be the same or different, and
G¹ is a substituted or unsubstituted aromatic group having 6 to 40 carbon atoms, a substituted or unsubstituted monoamino aromatic group having 6 to 40 carbon atoms, a substituted or unsubstituted diamino aromatic group having 6 to 60 carbon atoms, a substituted or unsubstituted triamino aromatic group having 6 to 60 carbon atoms, a substituted or unsubstituted heterocyclic group having 3 to 40 carbon atoms, a substituted or unsubstituted ethenylene group, or a single bond.

5. The organic electroluminescent device according to claim 4, wherein FL of the formula (2) is a monovalent fluoranthene compound residue represented by any one of the following formulas (3) to (6), 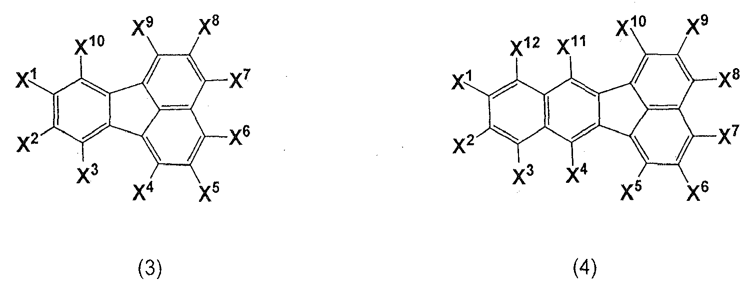 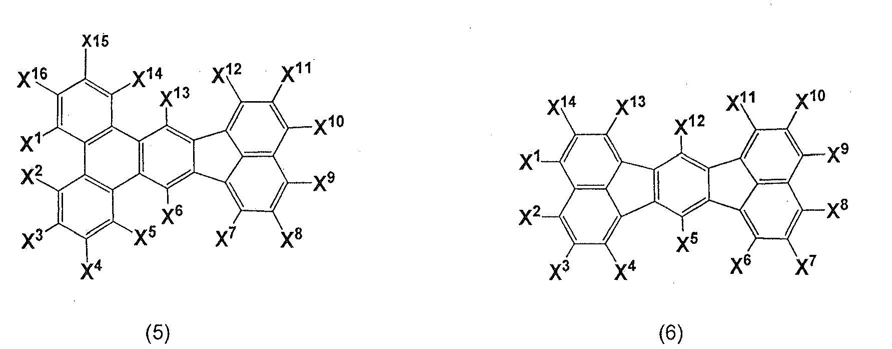 wherein X¹ to X¹⁶ are independently a hydrogen atom, a halogen atom, a nitro group, a cyano group, a substituted or unsubstituted straight, branched, or cyclic alkyl group having 1 to 30 carbon atoms, a substituted or unsubstituted straight , branched, or cyclic alkoxy group having 1 to 30 carbon atoms, a substituted or unsubstituted straight, branched, or cyclic alkylthio group having 1 to 30 carbon atoms, a substituted or unsubstituted straight, branched, or cyclic alkenyl group having 2 to 30 carbon atoms, a substituted or unsubstituted straight, branched, or cyclic alkenyloxy group having 2 to 30 carbon atoms, a substituted or unsubstituted straight, branched, or cyclic alkenylthio group having 2 to 30 carbon atoms, a substituted or unsubstituted aralkyl group having 7 to 30 carbon atoms, a substituted or unsubstituted aralkyloxy group having 7 to 30 carbon atoms, a substituted or unsubstituted aralkylthio group having 7 to 30 carbon atoms, a substituted or unsubstituted aryl group having 6 to 40 carbon atoms which is not shown by the formulas (3) to (6), a substituted or unsubstituted aryloxy group having 6 to 40 carbon atoms, a substituted or unsubstituted arylthio group having 6 to 40 carbon atoms, a substituted or unsubstituted heterocyclic group having 6 to 40 carbon atoms, a substituted or unsubstituted amino group having 2 to 30 carbon atoms, nitro group, cyano group, silyl group, hydroxy group, -COOR^{1e} group wherein R^{1e} is a hydrogen atom, a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms or a substituted or unsubstituted aryl group having 6 to 30 carbon atoms, -COR^{2e} group wherein R^{2e} is a hydrogen atom, a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms, a substituted or unsubstituted aryl group having 6 to 30 carbon atoms or an amino group, or -OCOR^{3e} group wherein R^{3e} is a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms or a substituted or unsubstituted aryl group having 6 to 30 carbon atoms; and adjacent groups and substituents of each group of X¹ to X¹⁶ may be bonded to each other to form a substituted or unsubstituted carbon ring.

6. The organic electroluminescent device according to claim 5, wherein the monovalent fluoranthene compound residue is a monovalent residue of a compound represented by the formula (3) or (4).

7. The organic electroluminescent device according to claim 4, wherein G¹ in the formula (2) is any one of groups represented by the following formulas.

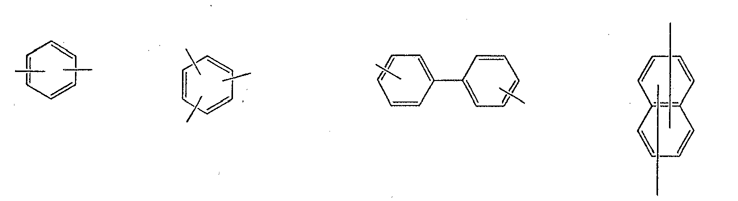

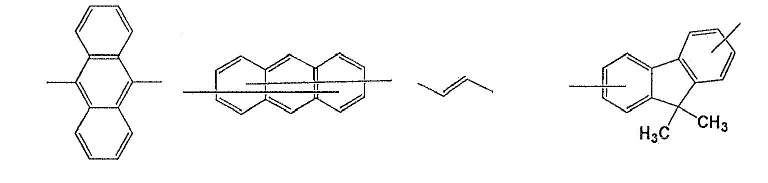

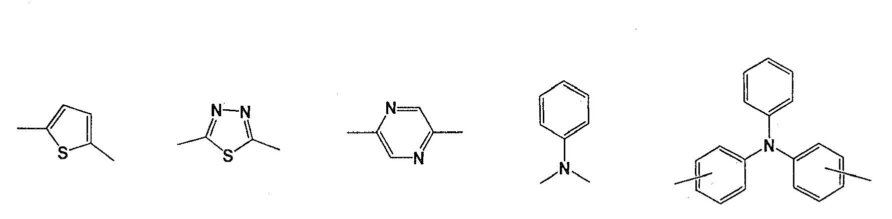

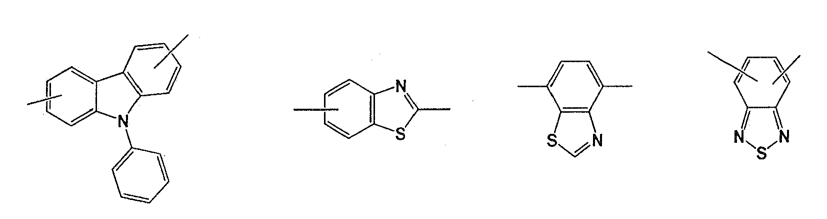

8. The organic electroluminescent device according to any one of claims 1 to 3, wherein the fluoranthene compound is a compound represented by any one of the following formulas (3) to (6): 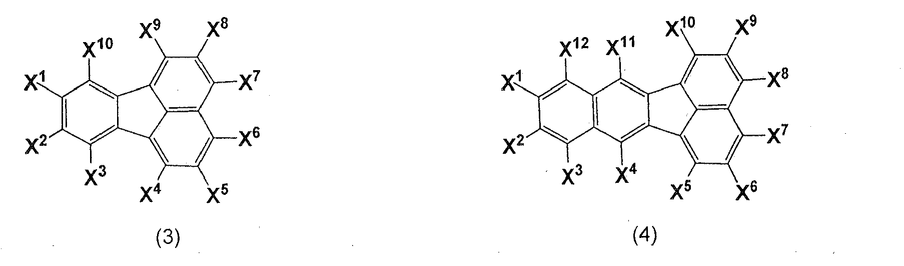 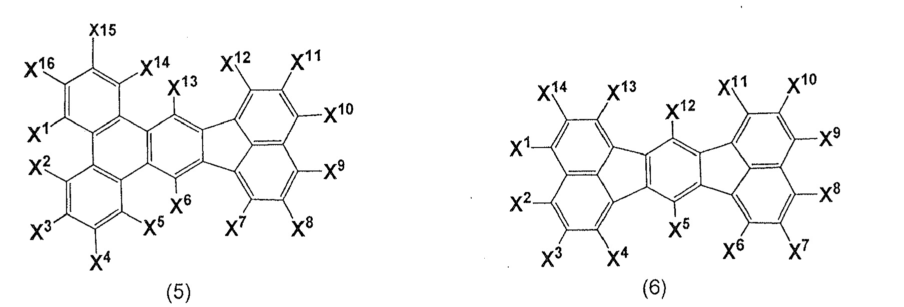 wherein X¹ to X¹⁶ are independently a hydrogen atom, a halogen atom, a nitro group, a cyano group, a substituted or unsubstituted straight, branched, or cyclic alkyl group having 1 to 30 carbon atoms, a substituted or unsubstituted straight , branched, or cyclic alkoxy group having 1 to 30 carbon atoms, a substituted or unsubstituted straight, branched, or cyclic alkylthio group having 1 to 30 carbon atoms, a substituted or unsubstituted straight, branched, or cyclic alkenyl group having 2 to 30 carbon atoms, a substituted or unsubstituted straight, branched, or cyclic alkenyloxy group having 2 to 30 carbon atoms, a substituted or unsubstituted straight, branched, or cyclic alkenylthio group having 2 to 30 carbon atoms, a substituted or unsubstituted aralkyl group having 7 to 30 carbon atoms, a substituted or unsubstituted aralkyloxy group having 7 to 30 carbon atoms, a substituted or unsubstituted aralkylthio group having 7 to 30 carbon atoms, a substituted or unsubstituted aryl group having 6 to 40 carbon atoms which is not shown by the formulas (3) to (6), a substituted or unsubstituted aryloxy group having 6 to 40 carbon atoms, a substituted or unsubstituted arylthio group having 6 to 40 carbon atoms, a substituted or unsubstituted heterocyclic group having 6 to 40 carbon atoms, a substituted or unsubstituted amino group having 2 to 30 carbon atoms, nitro group, cyano group, silyl group, hydroxy group, -COOR^{1e} group wherein R^{1e} is a hydrogen atom, a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms or a substituted or unsubstituted aryl group having 6 to 30 carbon atoms, -COR^{2e} group wherein R^{2e} is a hydrogen atom, a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms, a substituted or unsubstituted aryl group having 6 to 30 carbon atoms or an amino group, or -OCOR^{3e} group wherein R^{3e} is a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms or a substituted or unsubstituted aryl group having 6 to 30 carbon atoms; and adjacent groups and substituents of each group of X¹ to X¹⁶ may be bonded to each other to form a substituted or unsubstituted carbon ring.

9. The organic electroluminescent device according to claim 8, wherein the fluoranthene compound is a compound represented by the formula (3) or (4).

10. The organic electroluminescent device according to claim 8, wherein at least one of X¹ to X¹⁶ in the formulas (3) to (6) is an electron attracting group or a group having an electron attracting group selected from the group consisting of a nitro group, a cyano group, a halogen atom, a haloalkyl group, -COOR^{1e} group wherein R^{1e} is a hydrogen atom, a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms or a substituted or unsubstituted aryl group having 6 to 30 carbon atoms, and -COR^{2e} group wherein R^{2e} is a hydrogen atom, a substituted or unsubstituted straight, branched, or cyclic alkyl group having 1 to 30 carbon atoms, a substituted or unsubstituted straight, branched, or cyclic alkenyl group having 2 to 30 carbon atoms, a substituted or unsubstituted aralkyl group having 7 to 30 carbon atoms or a substituted or unsubstituted aryl group having 6 to 30 carbon atoms or an amino group.

11. The organic electroluminescent device according to claim 10, wherein the fluoranthene compound is a compound represented by the formula (3) or (4).

12. The organic electroluminescent device according to claim 8, wherein at least one of X¹ to X¹⁶ of the formulas (3) to (6) is a substituted aryl group having one or more electron attracting groups.

13. The organic electroluminescent device according to claim 8, wherein at least one of X¹ to X¹⁶ of the formulas (3) to (6) is a substituted aryl group, and a substituent of the aryl group is a substituted or unsubstituted aryl group having an electron attracting group.

14. The organic electroluminescent device according to any one of claims 10 to 13, wherein the electron attracting group is a group selected from the group consisting of a nitro group, a cyano group, a fluorine group and an haloalkyl group.

15. The organic electroluminescent device according to any one of claims 10 to 14, wherein X⁴ and X¹¹ of the compound (4) is a phenyl group.

16. The organic electroluminescent device according to any one of claims 10 to 15, wherein X⁷ of the compound (4) is a hydrogen atom, and X⁸ of the compound (4) is an aryl group having an electron attracting group.

17. The organic electroluminescent device according to any one of claims 10 to 15, wherein X⁷ of the compound (4) is a hydrogen atom, and X⁸ of the compound (4) is a substituted aryl group having an aryl group having an electron attracting group.

18. The organic electroluminescent device according to any one of claims 10 to 14, wherein X³ and X¹⁰ of the compound (3) is a phenyl group.

19. The organic electroluminescent device according to any one of claims 10 to 14 and 18, wherein X⁶ of the compound (3) is a hydrogen atom and X⁷ of the compound (3) is an aryl group having an electron attracting group.

20. The organic electroluminescent device according to any one of claims 10 to 14 and 18, wherein X⁶ of the compound (3) is a hydrogen atom and X⁷ of the compound (3) is a substituted aryl group having an aryl group having an electron attracting group.

21. The organic electroluminescent device according to any one of claims 1 to 20, wherein the compound represented by the formula (1) is a compound having in its molecule one or more skeletons selected from anthracene, phenanthrene, naphthacene, pyrene, chrysene, benzanthracene, pentacene, dibenzanthracene, benzopyrene, fluorene, benzofluorene, fluoranthene, benzofluoranthene, naphthofluoranthene, dibenzofluorene, dibenzopyrene and dibenzofluoranthene.

22. The organic electroluminescent device according to any one of claims 1 to 21, wherein the compound represented by the formula (1) is a nitrogen-containing heterocyclic compound.

23. The organic electroluminescent device according to claim 22, wherein the nitrogen-containing heterocyclic compound is a compound having in its molecule one or more skeletons selected from pyridine, pyrimidine, pyrazine, pyridazine, triazine, quinoline, quinoxaline, acridine, imidazopyridine, imidazopyrimidine and phenenthroline.

24. The organic electroluminescent device according to claim 22, wherein the nitrogen-containing heterocyclic compound is a benzimidazole derivative represented by the following formula (7) or (8): 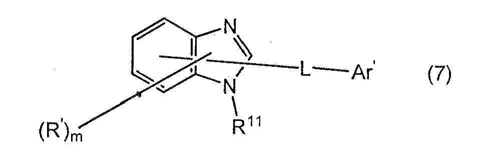 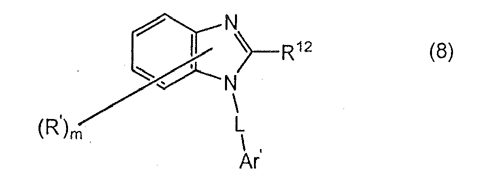 wherein R' is a hydrogen atom, a substituted or unsubstituted aryl group having 6 to 60 carbon atoms, a substituted or unsubstituted pyridyl group, a substituted or unsubstituted quinolyl group, a substituted or unsubstituted alkyl group having 1 to 20 carbon atoms, or a substituted or unsubstituted alkoxy group having 1 to 20 carbon atoms;
m is an integer of 0 to 4;
R¹¹ is a substituted or unsubstituted aryl group having 6 to 60 carbon atoms, a substituted or unsubstituted pyridyl group, a substituted or unsubstituted quinolyl group, a substituted or unsubstituted alkyl group having 1 to 20 carbon atoms, or an alkoxy group having 1 to 20 carbon atoms;
R¹² is a hydrogen atom, a substituted or unsubstituted aryl group having 6 to 60 carbon atoms, a substituted or unsubstituted pyridyl group, a substituted or unsubstituted quinolyl group, a substituted or unsubstituted alkyl group having 1 to 20 carbon atoms, or a substituted or unsubstituted alkoxy group having 1 to 20 carbon atoms;
L is a substituted or unsubstituted arylene group having 6 to 60 carbon atoms, a substituted or unsubstituted pyridinylene group, a substituted or unsubstituted quinolinylene group, or a substituted or unsubstituted fluorenylene group; and
Ar' is a substituted or unsubstituted aryl group having 6 to 60 carbon atoms, a substituted or unsubstituted pyridinyl group, or a substituted or unsubstituted quinolinyl group.

25. The organic electroluminescent device according to any one of claims 1 to 24, wherein the host compound is an anthracene derivative.

26. The organic electroluminescent device according to claim 25, wherein the anthracene derivative is a compound represented by the following formula (9): 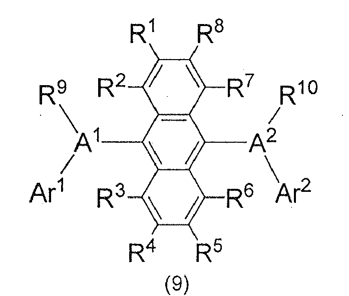 wherein A¹ and A² are independently a hydrogen atom or a substituted or unsubstituted aromatic group having 6 to 50 ring carbon atoms;
Ar¹ and Ar² are independently a hydrogen atom or a substituted or unsubstituted aromatic group having 6 to 50 ring carbon atoms;
R¹ to R¹⁰ are independently a hydrogen atom, a substituted or unsubstituted aromatic group having 6 to 50 ring carbon atoms, a substituted or unsubstituted aromatic hetrocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group or a hydroxyl group; and
Ar¹ and R⁹ and/or Ar² and R¹⁰ may be bonded to each other to form a saturated or unsaturated cyclic structure.

27. The organic electroluminescent device according to claim 26, wherein the anthracene derivative is a compound in which the groups are not symmetrically bonded to 9 and 10 positions of the central anthracene with respect to the X-Y axis in the following formula.

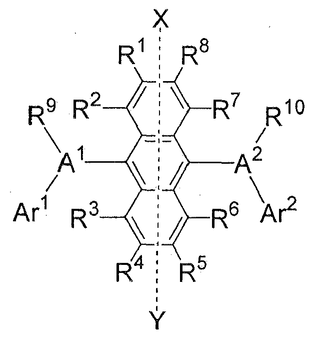

28. The organic electroluminescent device according to any one of claims 1 to 27, wherein the emitting layer contains the fluoranthene compound in an amount of 0.01 to 20 wt%.

29. The organic electroluminescent device according to any one of claims 1 to 28, wherein a chalcogenide layer, a metal halide layer or a metal oxide layer is provided on a surface of at least one of the cathode and the anode.

30. A solution for forming an emitting layer of an organic electroluminescent device comprising a fluoranthene compound represented by any one of the formulas (2) to (6) and a solvent.
